# EUROPEAN PATENT SPECIFICATION

(11) **EP 3 166 419 B1**
(45) Date of publication and mention of the grant of the patent: **18.09.2019**
(21) Application number: 15744426.6
(22) Date of filing: 10.07.2015
(51) Int. Cl.: A23K 50/00

(54) **PAENIBACILLUS AND BACILLUS SPP. MANNANASES**
PAENIBACILLUS UND BACILLUS SPP. MANNANASEN
MANNANASES DE PAENIBACILLUS ET BACILLUS SPP.

(30) Priority: 11.07.2014 WO PCT/CN2014/082034
(43) Date of publication of application: 17.05.2017
(73) Proprietor: Danisco US Inc., Palo Alto, California 94304 (US)
(72) Inventor: ADAMS, Christian D., Palo Alto, California 94304 (US); GHIRNIKAR, Roopa, Palo Alto, California 94304 (US); HUANG, Victoria, Palo Alto, California 94304 (US); JIN, Liling, Palo Alto, California 94304 (US); KOLKMAN, Marc, Palo Alto, California 94304 (US); QIAN, Zhen, Palo Alto, California 94304 (US)
(74) Representative: Mewburn Ellis LLP
(86) International application number: PCT/US2015/040057
(87) International publication number: WO 2016/007929

(56) References cited:
- EP-A1- 2 287 318
- WO-A1-2012/149317
- WO-A1-2012/149325
- WO-A1-2012/149333
- WO-A1-2014/100018
- WO-A2-2009/108941
- WO-A2-2009/108941
- WO-A2-2009/108941
- CN-A- 1 904 052
- CN-A- 1 904 052
- CN-A- 1 904 052
- DATABASE UniProt [Online] 2 November 2010 (2010-11-02), "SubName: Full=Endoglucanase {ECO:0000313|EMBL:ADM68451.1}; EC=3.2.1.78 {ECO:0000313|EMBL:ADM68451.1};", XP002779513, retrieved from EBI accession no. UNIPROT:E0RFB6 Database accession no. E0RFB6
- DATABASE UniProt [Online] 11 January 2011 (2011-01-11), "SubName: Full=Endoglucanase {ECO:0000313|EMBL:ADO54643.1};", XP002779514, retrieved from EBI accession no. UNIPROT:E3EGK4 Database accession no. E3EGK4
- DATABASE UniProt [Online] 22 September 2009 (2009-09-22), "SubName: Full=Beta-mannanase {ECO:0000313|EMBL:ACU30843.1}; EC=3.2.1.78 {ECO:0000313|EMBL:ACU30843.1};", XP002779515, retrieved from EBI accession no. UNIPROT:C7FFF2 Database accession no. C7FFF2
- LI YANAN ET AL: "Gene cloning, expression, and characterization of a novel beta-mannanase from Bacillus circulans CGMCC 1416", JOURNAL OF MICROBIOLOGY AND BIOTECHNOLOGY, KOREAN SOCIETY FOR APPLIED MICROBIOLOGY, SEOUL, KR, vol. 18, no. 1, 1 January 2008 (2008-01-01), pages 160-166, XP009186548, ISSN: 1017-7825
- PEILONG YANG ET AL: "A Novel [beta]-mannanase with High Specific Activity from Bacillus circulans CGMCC1554: Gene Cloning, Expression and Enzymatic Characterization", APPLIED BIOCHEMISTRY AND BIOTECHNOLOGY, vol. 159, no. 1, 24 September 2008 (2008-09-24), pages 85-94, XP055219229, United States ISSN: 0273-2289, DOI: 10.1007/s12010-008-8364-3
- ZHAO YUEJU ET AL: "Crystallization and preliminary X-ray study of alkaline .beta.-mannanase from the alkaliphilic Bacillus sp N16-5", ACTA CRYSTALLOGRAPHICA. SECTION F: STRUCTURAL BIOLOGY AND CRYSTALLIZATION COMMUNICATIONS, WILEY-BLACKWELL PUBLISHING, INC, US, vol. 64, no. Part 10, 1 October 2008 (2008-10-01), pages 957-959, XP008120224, ISSN: 1744-3091, DOI: 10.1107/S1744309108028571 -& DATABASE UniProt [Online] 23 November 2004 (2004-11-23), "SubName: Full=Beta-mannanase {ECO:0000313|EMBL:AAT06599.1}; EC=3.2.1.78 {ECO:0000313|EMBL:AAT06599.1};", XP002747724, retrieved from EBI accession no. UNIPROT:Q5YEX6 Database accession no. Q5YEX6
- YOSHIDA S ET AL: "CLONING, SEQUENCE ANALYSIS, AND EXPRESSION IN ESCHERICHIA COLI OF AGENE CODING FOR AN ENZYME FROM BACILLUS CIRCULANS K-1 THAT DEGRADES GUAR GUM", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 62, no. 3, 1 January 1998 (1998-01-01), pages 514-520, XP000881879, ISSN: 0916-8451, DOI: 10.1271/BBB.62.514 -& DATABASE UniProt [Online] 1 August 1998 (1998-08-01), "SubName: Full=Mannanase {ECO:0000313|EMBL:BAA25878.1};", XP002747725, retrieved from EBI accession no. UNIPROT:O66185 Database accession no. O66185
- PRAKRAM SINGH CHAUHAN ET AL: "Mannanases: microbial sources, production, properties and potential biotechnological applications", APPLIED MICROBIOLOGY AND BIOTECHNOLOGY, SPRINGER, BERLIN, DE, vol. 93, no. 5, 8 February 2012 (2012-02-08), pages 1817-1830, XP035019260, ISSN: 1432-0614, DOI: 10.1007/S00253-012-3887-5
- DHAWAN SAMRITIL ET AL: "Microbial mannanases: An overview of production and applications", CRC CRITICAL REVIEWS IN BIOTECHNOLOGY, CRC PRESS, BOCA RATON, FL, US, vol. 27, no. 4, 1 January 2007 (2007-01-01), pages 197-216, XP009161462, ISSN: 0738-8551
- DATABASE UniProt [Online] 2 November 2010 (2010-11-02), "SubName: Full=Endoglucanase {ECO:0000313|EMBL:ADM68451.1}; EC=3.2.1.78 {ECO:0000313|EMBL:ADM68451.1};", XP002779513, retrieved from EBI accession no. UNIPROT:E0RFB6 Database accession no. E0RFB6
- DATABASE UniProt [Online] 11 January 2011 (2011-01-11), "SubName: Full=Endoglucanase {ECO:0000313|EMBL:ADO54643.1};", XP002779514, retrieved from EBI accession no. UNIPROT:E3EGK4 Database accession no. E3EGK4
- DATABASE UniProt [Online] 22 September 2009 (2009-09-22), "SubName: Full=Beta-mannanase {ECO:0000313|EMBL:ACU30843.1}; EC=3.2.1.78 {ECO:0000313|EMBL:ACU30843.1};", XP002779515, retrieved from EBI accession no. UNIPROT:C7FFF2 Database accession no. C7FFF2

## Description

The present disclosure relates to endo-β-mannanases from *Paenibacillus or Bacillus spp,* polynucleotides encoding such endo-β-mannanases, compositions containing such mannanases, and methods of use thereof. Compositions containing such endo-β-mannanases are suitable for use as detergents and cleaning fabrics and hard surfaces, as well as a variety of other industrial applications.

Mannanase enzymes, including endo-β-mannanases, have been employed in detergent cleaning compositions for the removal of gum stains by hydrolyzing mannans. A variety of mannans are found in nature, such as, for example, linear mannan, glucomannan, galactomannan, and glucogalactomannan. Each such mannan is comprised of polysaccharides that containa β-1,4-linked backbone of mannose residues that may be substituted up to 33% with glucose residues (Yeoman et al., Adv Appl Microbiol, Elsivier). In galactomannans or glucogalactomannnans, galactose residues are linked in alpha-1,6-linkages to the mannan backbone (Moreira and Filho, Appl Microbiol Biotechnol, 79:165, 2008). Therefore, hydrolysis of mannan to its component sugars requires endo-1,4-β-mannanases that hydrolyze the backbone linkages to generate short chain manno-oligosaccharides that are further degraded to monosaccharides by 1,4-β-mannosidases. EP 2 287 318 A, WO 2014/100018 A1, WO 2012/149333 A1 and WO 2012/149325 disclose mannanases and their use in cleaning applications. CN 1 904 052 A discloses a β-mannanase designated MANB48, but suggests that SDS has an inhibitory effect on the enzyme.

Although endo-β-mannanases have been known in the art of industrial enzymes, there remains a need for further endo-β-mannanases that are suitable for particular conditions and uses.

The invention provides a cleaning composition comprising a surfactant and an endo-β-mannanase polypeptide comprising an amino acid sequence having at least 90% identity to an amino acid sequence selected from SEQ ID NO: 8, 10, 12, 42, 43, 44, 46, 47, 48, 50, 51 and 52, or an active fragment thereof, wherein said polypeptide or active fragment thereof has mannanase activity in the presence of a surfactant.

In some embodiments, the endo-β-mannanase polypeptide or active fragment thereof contains Asn33-Asp-34-Leu35 (NDL), wherein the amino acid positions of the polypeptide are numbered by correspondence with the amino sequence set forth in SEQ ID NO:32 and are based on the conserved linear sequence numbering.

The endo-β-mannanase polypeptide or active fragment thereof may further comprise a WXaKNDLXXAI motif at positions 30-38, wherein Xₐ is F or Y and X is any amino acid, wherein the amino acid positions of the polypeptide are numbered by correspondence with the amino sequence set forth in SEQ ID NO:32 and are based on the conserved linear sequence numbering.

For example, the endo-β-mannanase polypeptide or active fragment thereof may further comprise a WXₐKNDLX_{b}X_{c}AI motif at positions 30-38, wherein Xₐ is F or Y, X_{b} is N, Y or A, and X_{c} is A or T, wherein the amino acid positions of the polypeptide are numbered by correspondence with the amino sequence set forth in SEQ ID NO:32 and are based on the conserved linear sequence numbering.

The endo-β-mannanase polypeptide or active fragment thereof may further comprise a L₂₆₂D₂₆₃XXXGPXGXL₂₇₂T₂₇₃, motif at positions 262-273, where X is any amino acid and wherein the amino acid positions of the polypeptide are numbered by correspondence with the amino sequence set forth in SEQ ID NO:32 and are based on the conserved linear sequence numbering.

For example, the endo-β-mannanase polypeptide or active fragment thereof may further comprise a L₂₆₂D₂₆₃M/LV/AT/AGPX₁GX₂L₂₇₂T₂₇₃ motif at positions 262-273, where X₁ is N, A or S and X₂ is S, T or N, and wherein the amino acid positions of the polypeptide are numbered by correspondence with the amino sequence set forth in SEQ ID NO:32 and are based on the conserved linear sequence numbering.

The endo-β-mannanase polypeptide may be an NDL-Clade-1 polypeptide further comprising a LDM/LATGPA/NGS/TLT motif at positions 262-273, wherein the amino acid positions of the polypeptide are numbered by correspondence with the amino sequence set forth in SEQ ID NO:32 and are based on the conserved linear sequence numbering.

Alternatively, the endo-β-mannanase polypeptide may be an NDL-Clade 2 polypeptide further comprising a LDLA/VA/TGPS/NGNLT motif at positions 262-273, wherein the amino acid positions of the polypeptide are numbered by correspondence with the amino sequence set forth in SEQ ID NO:32 and are based on the conserved linear sequence numbering.

The endo-β-mannanase polypeptide or active fragment thereof may have mannanase activity on locust bean gum galactomannan or konjac glucomannan, or is capable of hydrolyzing a substrate selected from the group consisting of guar gum, locust bean gum, and combinations thereof.

In some embodiments, the endo-β-mannanase polypeptide or active fragment thereof retains at least 70% of its maximal mannanase activity at a pH range of 4.5-9.0; retains at least 70% of its maximal mannanase activity at a pH range of 5.5-8.5; retains at least 70% of its maximal mannanase activity at a pH range of 6.0-7.5; retains at least 70% of its maximal mannanase activity at a temperature range of 40°C to 70°C; retains at least 70% of its maximal mannanase activity at a temperature range of 45°C to 65°C; and/or retains at least 70% of its maximal mannanase activity at a temperature range of 50°C to 60°C.

In some embodiments, the endo-β-mannanase polypeptide or active fragment thereof does not comprise a carbohydrate-binding module.

The cleaning composition may further comprise at least one adjunct ingredient; and/or an enzyme selected from the group consisting of acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, oxidases, pectate lyases, pectin acetyl esterases, pectinases, pentosanases, perhydrolases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, and combinations thereof.

In some embodiments, the cleaning composition:
(i) is a detergent composition selected from the group consisting of a laundry detergent, a fabric softening detergent, a dishwashing detergent, and a hard-surface cleaning detergent;
(ii) is in a form selected from the group consisting of a liquid, a powder, a granulated solid, a tablet, a sheet, and a unit dose;
(iii) is phosphate-free; or
(iv) is boron-free.

In some preferred embodiments, the surfactant is an ionic surfactant. In some embodiments, the ionic surfactant is selected from the group consisting of an anionic surfactant, a cationic surfactant, a zwitterionic surfactant, and a combination thereof.

The present disclosure further provides methods for hydrolyzing a mannan substrate present in a soil or stain on a surface, comprising: contacting the surface with the detergent composition to produce a clean surface. Also provided are methods of textile cleaning comprising: contacting a soiled textile with the detergent composition to produce a clean textile.

These and other aspects of compositions and methods of the present invention will be apparent from the following description.

### DESCRIPTION OF THE DRAWINGS

Figure 1 provides a plasmid map of p2JM-PspMan4.
Figures 2A-B show the cleaning performance of *Paenibacillus* and *Bacillus spp.* mannanases on Locust bean gum (CS-73) at pH 8, 20 minutes.
Figures 3A-C show the CLUSTAL W (1.83) multiple sequence alignment of mannanases including BciMan1, BciMan3, BciMan4, PamMan2, PpaMan2, PpoMan1, PpoMan2, PspMan4, PspMan5, PspMan9, and PtuMan2.
Figure 4 shows a phylogenetic tree of mannanases including BciMan1, BciMan3, BciMan4, PamMan2, PpaMan2, PpoMan1, PpoMan2, PspMan4, PspMan5, PspMan9, and PtuMan2 showing the branching of the NDL-Clade mannanases from other mannanases and the differentiation of NDL-Clade 1 and NDL-Clade 2.
Figure 5 shows the motif of the NDL-Clade mannanases at positions 30-38, using the conserved linear sequence numbering.
Figure 6 shows the motif of the NDL-Clade mannanases, including the NDL-Clade 1 and NDL-Clade 2 mannanases, that is between the conserved Leu262-Asp263 (LD) and conserved Leu272-Thr273 (LT) residues, using the conserved linear sequence numbering.
Figure 7 shows the potential structural consequences of motif changes found in the NDL-Clade mannanases. The closest known mannanase structure from Bacillus sp. JAMB-602 (1WKY) is shown in black while modelled structures of PspMan4, PspMan9 and PpaMan2 are shown in gray. The location of the deletion motif is highlighted by an arrow. The deletion motif is postulated to impact the structure of the loop in which it is located.

Described herein are endo-β-mannanases from *Paenibacillus or Bacillus spp,* polynucleotides encoding such endo-β-mannanases, compositions containing such mannanases, and methods of use thereof. In one embodiment, the *Paenibacillus and Bacillus spp.* endo-β-mannanases described herein have glycosyl hydrolase activity in the presence of detergent compositions. This feature of the endo-β-mannanases described herein makes them well suited for use in a variety of cleaning and other industrial applications, for example, where the enzyme can hydrolyze mannans in the presence of surfactants and other components found in detergent compositions.

The following terms are defined for clarity. Terms and abbreviations not defined should be accorded their ordinary meaning as used in the art:

As used herein, a "mannan endo-1,4-β-mannosidase," "endo-1,4-β-mannanase," "endo-β-1,4-mannase," "β-mannanase B," "β-1, 4-mannan 4-mannanohydrolase," "endo-β-mannanase," "β-D-mannanase," "1,4-β-D-mannan mannanohydrolase," or "endo-β-mannanase" (EC 3.2.1.78) refers to an enzyme capable of the random hydrolysis of 1,4-β-D-mannosidic linkages in mannans, galactomannans and glucomannans. Endo-1,4-β-mannanases are members of several families of glycosyl hydrolases, including GH26 and GH5. In particular, endo-β-mannanases constitute a group of polysaccharases that degrade mannans and denote enzymes that are capable of cleaving polyose chains containing mannose units *(i.e.,* are capable of cleaving glycosidic bonds in mannans, glucomannans, galactomannans and galactoglucomannans). The "endo-β-mannanases" of the present disclosure may possess additional enzymatic activities (e.g., endo-1,4-β-glucanase, 1,4-β-mannosidase, cellodextrinase activities, etc.).

As used herein, a "mannanase," "mannosidic enzyme," "mannolytic enzyme," "mannanase enzyme," "mannanase polypeptides," or "mannanase proteins" refers to an enzyme, polypeptide, or protein exhibiting a mannan degrading capability. The mannanase enzyme may be, for example, an endo-β-mannanase, an exo-β-mannanase, or a glycosyl hydrolase. As used herein, mannanase activity may be determined according to any procedure known in the art *(See, e.g.,* Lever, Anal. Biochem, 47:248, 1972; U.S. Pat. No. 6, 602, 842; and International Publication No. WO 95/35362A1).

As used herein, "mannans" are polysaccharides having a backbone composed of β-1,4-linked mannose; "glucomannans" are polysaccharides having a backbone of more or less regularly alternating β-1,4 linked mannose and glucose; "galactomannans" and "galactoglucomannans" are mannans and glucomannans with alpha-1,6 linked galactose sidebranches. These compounds may be acetylated. The degradation of galactomannans and galactoglucomannans is facilitated by full or partial removal of the galactose sidebranches. Further the degradation of the acetylated mannans, glucomannans, galactomannans and galactoglucomannans is facilitated by full or partial deacetylation. Acetyl groups can be removed by alkali or by mannan acetylesterases. The oligomers that are released from the mannanases or by a combination of mannanases and alpha-galactosidase and/or mannan acetyl esterases can be further degraded to release free maltose by β-mannosidase and/or β-glucosidase

As used herein, "catalytic activity" or "activity" describes quantitatively the conversion of a given substrate under defined reaction conditions. The term "residual activity" is defined as the ratio of the catalytic activity of the enzyme under a certain set of conditions to the catalytic activity under a different set of conditions. The term "specific activity" describes quantitatively the catalytic activity per amount of enzyme under defined reaction conditions.

As used herein, "pH-stability" describes the property of a protein to withstand a limited exposure to pH-values significantly deviating from the pH where its stability is optimal *(e.g.,* more than one pH-unit above or below the pH-optimum, without losing its activity under conditions where its activity is measurable).

As used herein, the phrase "detergent stability" refers to the stability of a specified detergent composition component (such as a hydrolytic enzyme) in a detergent composition mixture.

As used herein, a "perhydrolase" is an enzyme capable of catalyzing a reaction that results in the formation of a peracid suitable for applications such as cleaning, bleaching, and disinfecting.

As used herein, the term "aqueous," as used in the phrases "aqueous composition" and "aqueous environment," refers to a composition that is made up of at least 50% water. An aqueous composition may contain at least 50% water, at least 60% water, at least 70% water, at least 80% water, at least 90% water, at least 95% water, at least 97% water, at least 99% water, or even at least 99% water.

As used herein, the term "surfactant" refers to any compound generally recognized in the art as having surface active qualities. Surfactants generally include anionic, cationic, nonionic, and zwitterionic compounds, which are further described, herein.

As used herein, "surface property" is used in reference to electrostatic charge, as well as properties such as the hydrophobicity and hydrophilicity exhibited by the surface of a protein.

The term "oxidation stability" refers to endo-β-mannanases of the present disclosure that retain a specified amount of enzymatic activity over a given period of time under conditions prevailing during the mannosidic, hydrolyzing, cleaning, or other process disclosed herein, for example while exposed to or contacted with bleaching agents or oxidizing agents. In some embodiments, the endo-β-mannanases retain at least about 50%, about 60%, about 70%, about 75%, about 80%, about 85%, about 90%, about 92%, about 95%, about 96%, about 97%, about 98%, or about 99% endo-β-mannanase activity after contact with a bleaching or oxidizing agent over a given time period, for example, at least about 1 minute, about 3 minutes, about 5 minutes, about 8 minutes, about 12 minutes, about 16 minutes, about 20 minutes, etc.

The term "chelator stability" refers to endo-β-mannanases of the present disclosure that retain a specified amount of enzymatic activity over a given period of time under conditions prevailing during the mannosidic, hydrolyzing, cleaning, or other process disclosed herein, for example while exposed to or contacted with chelating agents. In some embodiments, the endo-β-mannanases retain at least about 50%, about 60%, about 70%, about 75%, about 80%, about 85%, about 90%, about 92%, about 95%, about 96%, about 97%, about 98%, or about 99% endo-β-mannanase activity after contact with a chelating agent over a given time period, for example, at least about 10 minutes, about 20 minutes, about 40 minutes, about 60 minutes, about 100 minutes, etc.

The terms "thermal stability" and "thermostable" refer to endo-β-mannanases of the present disclosure that retain a specified amount of enzymatic activity after exposure to identified temperatures over a given period of time under conditions prevailing during the mannosidic, hydrolyzing, cleaning, or other process disclosed herein, for example, while exposed to altered temperatures. Altered temperatures include increased or decreased temperatures. In some embodiments, the endo-β-mannanases retain at least about 50%, about 60%, about 70%, about 75%, about 80%, about 85%, about 90%, about 92%, about 95%, about 96%, about 97%, about 98%, or about 99% endo-β-mannanase activity after exposure to altered temperatures over a given time period, for example, at least about 60 minutes, about 120 minutes, about 180 minutes, about 240 minutes, about 300 minutes, etc.

The term "cleaning activity" refers to the cleaning performance achieved by the endo-β-mannanase under conditions prevailing during the mannosidic, hydrolyzing, cleaning, or other process disclosed herein. In some embodiments, cleaning performance is determined by the application of various cleaning assays concerning enzyme sensitive stains arising from food products, household agents or personal care products. Some of these stains include, for example, ice cream, ketchup, BBQ sauce, mayonnaise, soups, chocolate milk, chocolate pudding, frozen desserts, shampoo, body lotion, sun protection products, toothpaste, locust bean gum, or guar gum as determined by various chromatographic, spectrophotometric or other quantitative methodologies after subjection of the stains to standard wash conditions. Exemplary assays include, but are not limited to those described in WO 99/34011, U.S. Pat. No. 6,605,458, and U.S. Pat. No. 6,566,114, as well as those methods included in the Examples.

As used herein, the terms "clean surface" and "clean textile" refer to a surface or textile respectively that has a percent stain removal of at least 10%, preferably at least 15%, 20%, 25%, 30%, 35%, or 40% of a soiled surface or textile.

The term "cleaning effective amount" of an endo-β-mannanase refers to the quantity of endo-β-mannanase described herein that achieves a desired level of enzymatic activity in a specific cleaning composition. Such effective amounts are readily ascertained by one of ordinary skill in the art and are based on many factors, such as the particular endo-β-mannanase used, the cleaning application, the specific composition of the cleaning composition, and whether a liquid or dry (e.g., granular, bar, powder, solid, liquid, tablet, gel, paste, foam, sheet, or unit dose) composition is required, etc.

The term "cleaning adjunct materials", as used herein, means any liquid, solid or gaseous material selected for the particular type of cleaning composition desired and the form of the product (e.g., liquid, granule, powder, bar, paste, spray, tablet, gel, unit dose, sheet, or foam composition), which materials are also preferably compatible with the endo-β-mannanase enzyme used in the composition. In some embodiments, granular compositions are in "compact" form, while in other embodiments, the liquid compositions are in a "concentrated" form.

As used herein, "cleaning compositions" and "cleaning formulations" refer to admixtures of chemical ingredients that find use in the removal of undesired compounds (e.g., soil or stains) from items to be cleaned, such as fabric, dishes, contact lenses, other solid surfaces, hair, skin, teeth, and the like. The compositions or formulations may be in the form of a liquid, gel, granule, powder, bar, paste, spray tablet, gel, unit dose, sheet, or foam, depending on the surface, item or fabric to be cleaned and the desired form of the composition or formulation.

As used herein, the terms "detergent composition" and "detergent formulation" refer to mixtures of chemical ingredients intended for use in a wash medium for the cleaning of soiled objects. Detergent compositions/formulations generally include at least one surfactant, and may optionally include hydrolytic enzymes, oxido-reductases, builders, bleaching agents, bleach activators, bluing agents and fluorescent dyes, caking inhibitors, masking agents, enzyme activators, antioxidants, and solubilizers.

As used herein, "dishwashing composition" refers to all forms of compositions for cleaning dishware, including cutlery, including but not limited to granular and liquid forms. In some embodiments, the dishwashing composition is an "automatic dishwashing" composition that finds use in automatic dish washing machines. It is not intended that the present disclosure be limited to any particular type or dishware composition. Indeed, the present disclosure finds use in cleaning dishware (e.g., dishes including, but not limited to plates, cups, glasses, bowls, etc.) and cutlery (e.g., utensils including, but not limited to spoons, knives, forks, serving utensils, etc.) of any material, including but not limited to ceramics, plastics, metals, china, glass, acrylics, etc. The term "dishware" is used herein in reference to both dishes and cutlery.

As used herein, the term "bleaching" refers to the treatment of a material (e.g., fabric, laundry, pulp, etc.) or surface for a sufficient length of time and under appropriate pH and temperature conditions to effect a brightening (*i.e.,* whitening) and/or cleaning of the material. Examples of chemicals suitable for bleaching include but are not limited to ClO₂, H₂O₂, peracids, NO₂, etc.

As used herein, "wash performance" of a variant endo-β-mannanase refers to the contribution of a variant endo-β-mannanase to washing that provides additional cleaning performance to the detergent composition. Wash performance is compared under relevant washing conditions.

The term "relevant washing conditions" is used herein to indicate the conditions, particularly washing temperature, time, washing mechanics, sud concentration, type of detergent, and water hardness, actually used in households in a dish or laundry detergent market segment.

As used herein, the term "disinfecting" refers to the removal of contaminants from the surfaces, as well as the inhibition or killing of microbes on the surfaces of items. It is not intended that the present disclosure be limited to any particular surface, item, or contaminant(s) or microbes to be removed.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, a preferred filler salt is sodium sulfate.

The terms "textile" or "textile material" refer to woven fabrics, as well as staple fibers and filaments suitable for conversion to or use as yarns, woven, knit, and non-woven fabrics. The term encompasses yarns made from natural, as well as synthetic (e.g., manufactured) fibers.

A nucleic acid or polynucleotide is "isolated" when it is at least partially or completely separated from other components, including but not limited to for example, other proteins, nucleic acids, cells, etc. Similarly, a polypeptide, protein or peptide is "isolated" when it is at least partially or completely separated from other components, including but not limited to for example, other proteins, nucleic acids, cells, etc. On a molar basis, an isolated species is more abundant than are other species in a composition. For example, an isolated species may comprise at least about 60%, about 65%, about 70%, about 75%, about 80%, about 85%, about 90%, about 91%, about 92%, about 93%, about 94%, about 95%, about 96%, about 97%, about 98%, about 99%, or about 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a nucleic acid or a protein sample, respectively, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 60%, about 65%, about 70%, about 75%, about 80%, about 85%, about 90%, about 91%, about 92%, about 93%, about 94%, about 95%, about 96%, about 97%, about 98%, about 99%, about 99.5%, about 99.6%, about 99.7%, about 99.8% or more pure (e.g., percent by weight on a molar basis). In a related sense, a composition is enriched for a molecule when there is a substantial increase in the concentration of the molecule after application of a purification or enrichment technique. The term "enriched" refers to a compound, polypeptide, cell, nucleic acid, amino acid, or other specified material or component that is present in a composition at a relative or absolute concentration that is higher than a starting composition.

As used herein, a "polypeptide" refers to a molecule comprising a plurality of amino acids linked through peptide bonds. The terms "polypeptide," "peptide," and "protein" are used interchangeably. Proteins may optionally be modified (e.g., glycosylated, phosphorylated, acylated, farnesylated, prenylated, sulfonated, and the like) to add functionality. Where such amino acid sequences exhibit activity, they may be referred to as an "enzyme." The conventional one-letter or three-letter codes for amino acid residues are used, with amino acid sequences being presented in the standard amino-to-carboxy terminal orientation (*i.e.,* N→C).

The terms "polynucleotide" encompasses DNA, RNA, heteroduplexes, and synthetic molecules capable of encoding a polypeptide. Nucleic acids may be single-stranded or doublestranded, and may have chemical modifications. The terms "nucleic acid" and "polynucleotide" are used interchangeably. Because the genetic code is degenerate, more than one codon may be used to encode a particular amino acid, and the present compositions and methods encompass nucleotide sequences which encode a particular amino acid sequence. Unless otherwise indicated, nucleic acid sequences are presented in a 5'-to-3' orientation.

As used herein, the terms "wild-type" and "native" refer to polypeptides or polynucleotides that are found in nature.

The terms, "wild-type," "parental," or "reference," with respect to a polypeptide, refer to a naturally-occurring polypeptide that does not include a man-made substitution, insertion, or deletion at one or more amino acid positions. Similarly, the terms "wild-type," "parental," or "reference," with respect to a polynucleotide, refer to a naturally-occurring polynucleotide that does not include a man-made nucleoside change. However, note that a polynucleotide encoding a wild-type, parental, or reference polypeptide is not limited to a naturally-occurring polynucleotide, and encompasses any polynucleotide encoding the wild-type, parental, or reference polypeptide.

As used herein, a "variant polypeptide" refers to a polypeptide that is derived from a parent (or reference) polypeptide by the substitution, addition, or deletion, of one or more amino acids, typically by recombinant DNA techniques. Variant polypeptides may differ from a parent polypeptide by a small number of amino acid residues and may be defined by their level of primary amino acid sequence homology/identity with a parent polypeptide. Preferably, variant polypeptides have at least 70%, at least 75%, at least 80%, at least 85%, at least 90%, at least 91%, at least 92%, at least 93%, at least 94%, at least 95%, at least 96%, at least 97%, at least 98%, or even at least 99% amino acid sequence identity with a parent polypeptide.

Sequence identity may be determined using known programs such as BLAST, ALIGN, and CLUSTAL using standard parameters. *(See, e.g.,* Altschul et al. [1990] J. Mol. Biol. 215:403-410; Henikoff et al. [1989] Proc. Natl. Acad. Sci. USA 89:10915; Karin et al. [1993] Proc. Natl. Acad. Sci USA 90:5873; and Higgins et al. [1988] Gene 73:237-244). Software for performing BLAST analyses is publicly available through the National Center for Biotechnology Information. Databases may also be searched using FASTA (Pearson et al. [1988] Proc. Natl. Acad. Sci. USA 85:2444-2448). One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative substitution..

As used herein, a "variant polynucleotide" encodes a variant polypeptide, has a specified degree of homo logy/identity with a parent polynucleotide, or hybridizes under stringent conditions to a parent polynucleotide or the complement, thereof. Preferably, a variant polynucleotide has at least 70%, at least 75%, at least 80%, at least 85%, at least 90%, at least 91%, at least 92%, at least 93%, at least 94%, at least 95%, at least 96%, at least 97%, at least 98%, or even at least 99% nucleotide sequence identity with a parent polynucleotide. Methods for determining percent identity are known in the art and described immediately above.

The term "derived from" encompasses the terms "originated from," "obtained from," "obtainable from," "isolated from," and "created from," and generally indicates that one specified material find its origin in another specified material or has features that can be described with reference to the another specified material.

As used herein, the term "hybridization" refers to the process by which a strand of nucleic acid joins with a complementary strand through base pairing, as known in the art.

As used herein, the phrase "hybridization conditions" refers to the conditions under which hybridization reactions are conducted. These conditions are typically classified by degree of "stringency" of the conditions under which hybridization is measured. The degree of stringency can be based, for example, on the melting temperature (Tm) of the nucleic acid binding complex or probe. For example, "maximum stringency" typically occurs at about Tm-5 °C (5° below the Tm of the probe); "high stringency" at about 5-10° below the Tm; "intermediate stringency" at about 10-20° below the Tm of the probe; and "low stringency" at about 20-25° below the Tm. Alternatively, or in addition, hybridization conditions can be based upon the salt or ionic strength conditions of hybridization and/or one or more stringency washes, *e.g*.,: 6X SSC = very low stringency; 3X SSC = low to medium stringency; 1X SSC = medium stringency; and 0.5X SSC = high stringency. Functionally, maximum stringency conditions may be used to identify nucleic acid sequences having strict identity or near-strict identity with the hybridization probe; while high stringency conditions are used to identify nucleic acid sequences having about 80% or more sequence identity with the probe. For applications requiring high selectivity, it is typically desirable to use relatively stringent conditions to form the hybrids (e.g., relatively low salt and/or high temperature conditions are used). As used herein, stringent conditions are defined as 50°C and 0.2X SSC (IX SSC = 0.15 M NaCl, 0.015 M sodium citrate, pH 7.0).

The phrases "substantially similar" and "substantially identical" in the context of at least two nucleic acids or polypeptides means that a polynucleotide or polypeptide comprises a sequence that has at least about 90%, at least about 91%, at least about 92%, at least about 93%, at least about 94%, at least about 95%, at least about 96%, at least about 97%, at least about 98%, or even at least about 99% identical to a parent or reference sequence, or does not include amino acid substitutions, insertions, deletions, or modifications made only to circumvent the present description without adding functionality.

As used herein, an "expression vector" refers to a DNA construct containing a DNA sequence that encodes a specified polypeptide and is operably linked to a suitable control sequence capable of effecting the expression of the polypeptides in a suitable host. Such control sequences include a promoter to effect transcription, an optional operator sequence to control such transcription, a sequence encoding suitable mRNA ribosome binding sites and sequences which control termination of transcription and translation. The vector may be a plasmid, a phage particle, or simply a potential genomic insert. Once transformed into a suitable host, the vector may replicate and function independently of the host genome, or may, in some instances, integrate into the genome itself.

The term "recombinant," refers to genetic material (*i.e.,* nucleic acids, the polypeptides they encode, and vectors and cells comprising such polynucleotides) that has been modified to alter its sequence or expression characteristics, such as by mutating the coding sequence to produce an altered polypeptide, fusing the coding sequence to that of another gene, placing a gene under the control of a different promoter, expressing a gene in a heterologous organism, expressing a gene at a decreased or elevated levels, expressing a gene conditionally or constitutively in manner different from its natural expression profile, and the like. Generally recombinant nucleic acids, polypeptides, and cells based thereon, have been manipulated by man such that they are not identical to related nucleic acids, polypeptides, and cells found in nature.

A "signal sequence" refers to a sequence of amino acids bound to the N-terminal portion of a polypeptide, and which facilitates the secretion of the mature form of the protein from the cell. The mature form of the extracellular protein lacks the signal sequence which is cleaved off during the secretion process.

The term "selective marker" or "selectable marker" refers to a gene capable of expression in a host cell that allows for ease of selection of those hosts containing an introduced nucleic acid or vector. Examples of selectable markers include but are not limited to antimicrobial substances (e.g., hygromycin, bleomycin, or chloramphenicol) and/or genes that confer a metabolic advantage, such as a nutritional advantage, on the host cell. The terms "selectable marker" or "selectable gene product" as used herein refer to the use of a gene, which encodes an enzymatic activity that confers resistance to an antibiotic or drug upon the cell in which the selectable marker is expressed.

The term "regulatory element" as used herein refers to a genetic element that controls some aspect of the expression of nucleic acid sequences. For example, a promoter is a regulatory element which facilitates the initiation of transcription of an operably linked coding region. Additional regulatory elements include splicing signals, polyadenylation signals and termination signals.

As used herein, "host cells" are generally prokaryotic or eukaryotic hosts which are transformed or transfected with vectors constructed using recombinant DNA techniques known in the art. Transformed host cells are capable of either replicating vectors encoding the protein variants or expressing the desired protein variant. In the case of vectors which encode the pre- or pro-form of the protein variant, such variants, when expressed, are typically secreted from the host cell into the host cell medium.

The term "introduced" in the context of inserting a nucleic acid sequence into a cell, means transformation, transduction or transfection. Means of transformation include protoplast transformation, calcium chloride precipitation, electroporation, naked DNA, and the like as known in the art. *(See,* Chang and Cohen [1979] Mol. Gen. Genet. 168:111-115; Smith et al. [1986] Appl. Env. Microbiol. 51:634; and the review article by Ferrari et al., in Harwood, Bacillus, Plenum Publishing Corporation, pp. 57-72, 1989).

Other technical and scientific terms have the same meaning as commonly understood by one of ordinary skill in the art to which this disclosure pertains *(See, e.g.,* Singleton and Sainsbury, Dictionary of Microbiology and Molecular Biology, 2d Ed., John Wiley and Sons, NY 1994; and Hale and Marham, The Harper Collins Dictionary of Biology, Harper Perennial, NY 1991).

The singular terms "a," "an," and "the" include the plural reference unless the context clearly indicates otherwise.

As used herein in connection with a numerical value, the term "about" refers to a range of -10% to +10% of the numerical value. For instance, the phrase a "pH value of about 6" refers to pH values of from 5.4 to 6.6.

Headings are provided for convenience and should not be construed as limitations. The description included under one heading may apply to the specification as a whole.

### Paenibacillus and Bacillus spp. Polypeptides

The compositions and methods of the invention employ an endo-β-mannanase polypeptide comprising an amino acid sequence having at least 90% identity to an amino acid sequence selected from SEQ ID NO: 8, 10, 12, 42, 43, 44, 46, 47, 48, 50, 51 and 52, or an active fragment thereof, wherein said polypeptide or active fragment thereof has mannanase activity in the presence of a surfactant. In some embodiments, the polypeptide or recombinant polypeptide or fragment, active fragment, or variant thereof, described herein contains Asn33-Asp-34-Leu35 (NDL), wherein the amino acid positions of the polypeptide are numbered by correspondence with the amino sequence set forth in SEQ ID NO:32 and are based on conserved linear sequence numbering.

In some embodiments, the endo-β-mannanase polypeptide or active fragment thereof comprises an Asn33-Asp-34-Leu35, wherein the amino acid positions of the polypeptide are numbered by correspondence with the amino sequence set forth in SEQ ID NO:32 and are based on the conserved linear sequence numbering. In another embodiment, the endo-β-mannanase polypeptide or active fragment thereof comprises a WXaKNDLXXAI motif at positions 30-38, wherein Xₐ is F or Y and X is any amino acid, wherein the amino acid positions of the polypeptide are numbered by correspondence with the amino sequence set forth in SEQ ID NO:32 and are based on the conserved linear sequence numbering. In some embodiments, the endo-β-mannanase polypeptide or active fragment thereof comprises a WXₐKNDLX_{b}X_{c}AI motif at positions 30-38, wherein Xₐ is F or Y, X_{b} is N, Y or A, and X_{c} is A or T, wherein the amino acid positions of the polypeptide are numbered by correspondence with the amino sequence set forth in SEQ ID NO:32 and are based on the conserved linear sequence numbering.

In a further embodiment, the endo-β-mannanase polypeptide or active fragment thereof comprises a L₂₆₂D₂₆₃XXXGPXGXL₂₇₂T₂₇₃, motif at positions 262-273, where X is any amino acid and wherein the amino acid positions of the polypeptide are numbered by correspondence with the amino sequence set forth in SEQ ID NO:32 and are based on the conserved linear sequence numbering. In yet a still further embodiment, the endo-β-mannanase polypeptide or active fragment thereof comprises a L₂₆₂D₂₆₃M/LV/AT/AGPX₁GX₂L₂₇₂T₂₇₃ motif at positions 262-273, where X₁ is N, A or S and X₂ is S, T or N, and wherein the amino acid positions of the polypeptide are numbered by correspondence with the amino sequence set forth in SEQ ID NO:32 and are based on the conserved linear sequence numbering. In some embodiments, the endo-β-mannanase polypeptide or active fragment thereof is an NDL-Clade 1 polypeptide which comprises a LDM/LATGPN/AGS/TLT motif at positions 262-273, wherein the amino acid positions of the polypeptide are numbered by correspondence with the amino sequence set forth in SEQ ID NO:32 and are based on the conserved linear sequence numbering. In some embodiments, the endo-β-mannanase polypeptide or active fragment thereof is an NDL-Clade 2 polypeptide which comprises an LDLA/VA/TGPS/NGNLT motif at positions 262-273, wherein the amino acid positions of the polypeptide are numbered by correspondence with the amino sequence set forth in SEQ ID NO:32 and are based on the conserved linear sequence numbering.

Exemplary *Paenibacillus or Bacillus spp.* polypeptides which may be used in the compositions and methods described include PpoMan1 (SEQ ID NO:8) isolated from *Paenibacillus polymyxa E681,* PpoMan2 (SEQ ID NO:10) isolated from *Paenibacillus polymyxa SC2,* and PspMan4 (SEQ ID NO:12) isolated from *Paenibacillus sp.* A1. These and other isolated endo-β-mannanase polypeptides polypeptides are described in more detail below.

In other embodiments, the endo-β-mannanase polypeptide or active fragment comprises an amino acid sequence which has at least 95% identity to the amino acid sequence selected from the group consisting of SEQ ID NO: 8, 10, 12, 42, 43, 44, 46, 47, 48, 50, 51 or 52.

In some embodiments, the endo-β-mannanase polypeptide polypeptide or active fragment comprises an amino acid sequence selected from the group consisting of SEQ ID NO: 8, 10, 12, 42, 43, 44, 46, 47, 48, 50, 51 or 52.

Sequence identity can be determined by amino acid sequence alignment, e.g., using a program such as BLAST, ALIGN, or CLUSTAL, as described herein. In some embodiments, the polypeptides of the present invention are isolated polypeptides.

The polypeptide or active fragment has mannanase activity in the presence of a surfactant. In some embodiments, the mannanase activity is activity on mannan gum. In some embodiments, the mannanase activity is activity on locust bean gum galactomannan. In some embodiments, the mannanase activity is activity on konjac glucomannan.

In some embodiments, the polypeptide or active fragment retains at least 70% of its maximal protease activity at a pH range of 4.5-9.0. In some embodiments, the polypeptide or active fragment retains at least 70% of its maximal protease activity at a pH range of 5.5-8.5. In some embodiments, the polypeptide or active fragment retains at least 70% of its maximal protease activity at a pH range of 6.0-7.5. In some embodiments, the polypeptide or active fragment retains at least 70% of its maximal protease activity at a pH above 3.0, 3.5, 4.0 or 4.5. In some embodiments, the polypeptide or active fragment retains at least 70% of its maximal protease activity at a pH below 10.0, 9.5, or 9.0.

In some embodiments, the polypeptide or active fragment retains at least 70% of its maximal protease activity at a temperature range of 40°C to 70°C. In some embodiments, the polypeptide or active fragment retains at least 70% of its maximal protease activity at a temperature range of 45°C to 65°C. In some embodiments, the polypeptide or active fragment retains at least 70% of its maximal protease activity at a temperature range of 50°C to 60°C. In some embodiments, the polypeptide or active fragment retains at least 70% of its maximal protease activity at a temperature above 20°C, 25°C, 30°C, 35°C, or 40°C. In some embodiments, the polypeptide or active fragment retains at least 70% of its maximal protease activity at a temperature below 90°C, 85°C, 80°C, 75°C, or 70°C.

In some embodiments, the polypeptide or active ragment has cleaning activity in a detergent composition.

In some embodiments, the polypeptide or active fragment has mannanase activity in the presence of a protease. In some embodiments, the polypeptide or active fragment is capable of hydrolyzing a substrate selected from the group consisting of guar gum, locust bean gum, and combinations thereof.

In some embodiments, the polypeptide or active fragment does not further comprise a carbohydrate-binding module.

The polypeptides and active fragments employed in the present invention may be produced recombinantly, produced synthetically, or may be purified from a native source.

In certain embodiments, the polypeptide or active fragment may includ substitutions that do not substantially affect the structure and/or function of the polypeptide. Exemplary substitutions are conservative mutations, as summarized in Table I.

**Table I. Amino Acid Substitutions**

| **Original Residue** | **Code** | **Acceptable Substitutions** |
|---|---|---|
| Alanine | A | D-Ala, Gly, beta-Ala, L-Cys, D-Cys |
| Arginine | R | D-Arg, Lys, D-Lys, homo-Arg, D-homo-Arg, Met, Ile, D-Met, D-Ile, Orn, D-Orn |
| Asparagine | N | D-Asn, Asp, D-Asp, Glu, D-Glu, Gln, D-Gln |
| Aspartic Acid | D | D-Asp, D-Asn, Asn, Glu, D-Glu, Gln, D-Gln |
| Cysteine | C | D-Cys, S-Me-Cys, Met, D-Met, Thr, D-Thr |
| Glutamine | Q | D-Gln, Asn, D-Asn, Glu, D-Glu, Asp, D-Asp |
| Glutamic Acid | E | D-Glu, D-Asp, Asp, Asn, D-Asn, Gln, D-Gln |
| Glycine | G | Ala, D-Ala, Pro, D-Pro, beta-Ala, Acp |
| Isoleucine | I | D-Ile, Val, D-Val, Leu, D-Leu, Met, D-Met |
| Leucine | L | D-Leu, Val, D-Val, Leu, D-Leu, Met, D-Met |
| Lysine | K | D-Lys, Arg, D-Arg, homo-Arg, D-homo-Arg, Met, D-Met, Ile, D-Ile, Orn, D-Orn |
| Methionine | M | D-Met, S-Me-Cys, Ile, D-Ile, Leu, D-Leu, Val, D-Val |
| Phenylalanine | F | D-Phe, Tyr, D-Thr, L-Dopa, His, D-His, Trp, D-Trp, Trans-3,4, or 5-phenylproline, cis-3,4, or 5-phenylproline |
| Proline | P | D-Pro, L-I-thioazolidine-4-carboxylic acid, D-or L-1-oxazolidine-4-carboxylic acid |
| Serine | S | D-Ser, Thr, D-Thr, allo-Thr, Met, D-Met, Met(O), D-Met(O), L-Cys, D-Cys |
| Threonine | T | D-Thr, Ser, D-Ser, allo-Thr, Met, D-Met, Met(O), D-Met(O), Val, D-Val |
| Tyrosine | Y | D-Tyr, Phe, D-Phe, L-Dopa, His, D-His |
| Valine | V | D-Val, Leu, D-Leu, Ile, D-Ile, Met, D-Met |

Substitutions involving naturally occurring amino acids are generally made by mutating a nucleic acid encoding a recombinant a polypeptide of the present invention, and then expressing the variant polypeptide in an organism. Substitutions involving non-naturally occurring amino acids or chemical modifications to amino acids are generally made by chemically modifying a recombinant a polypeptide of the present invention after it has been synthesized by an organism.

In some embodiments, the polypeptides are substantially identical to SEQ ID NO: 8, 10, 12, 42, 43, 44, 46, 47, 48, 50, 51 or 52, meaning that they do not include amino acid substitutions, insertions, or deletions that do not significantly affect the structure, function, or expression of the polypeptide.

In some embodiments, the polypeptide has 1,4-β-D-mannosidic hydrolase activity, which includes mannanase, endo-1,4-β-D-mannanase, exo-1,4-β-D-mannanasegalactomannanase, and/or glucomannanase activity. 1,4-β-D-mannosidic hydrolase activity can be determined and measured using the assays described herein, or by other assays known in the art. In some embodiments, the polypeptide has activity in the presence of a detergent composition.

It will be clear that fragments of "full-length" polypeptides that retain 1,4-β-D-mannosidic hydrolase activity may also be employed. Such fragments preferably retain the active site of the full-length polypeptides but may have deletions of non-critical amino acid residues. The activity of fragments can readily be determined using the assays described, herein, or by other assays known in the art. In some embodiments, the fragments retain 1,4-β-D-mannosidic hydrolase activity in the presence of a detergent composition.

In some embodiments, the polypeptide or active fragment is produced as a N- and/or C-terminal fusion protein, for example to aid in extraction, detection and/or purification and/or to add functional properties to a polypeptide of the present invention. Examples of fusion protein partners include, but are not limited to, glutathione-S-transferase (GST), 6XHis, GAL4 (DNA binding and/or transcriptional activation domains), FLAG, MYC, BCE103 (WO 2010/044786), or other tags well known to anyone skilled in the art. In some embodiments, a proteolytic cleavage site is provided between the fusion protein partner and the protein sequence of interest to allow removal of fusion protein sequences. Preferably, the fusion protein does not hinder the activity of the polypeptide.

In some embodiments, a polypeptide or active fragment is fused to a functional domain including a leader peptide, propeptide, one or more binding domain (modules) and/or catalytic domain. Suitable binding domains include, but are not limited to, carbohydrate-binding modules (e.g., CBM) of various specificities, providing increased affinity to carbohydrate components present during the application of a polypeptide. As described herein, the CBM and catalytic domain of a polypeptide are operably linked.

A carbohydrate-binding module (CBM) is defined as a contiguous amino acid sequence within a carbohydrate-active enzyme with a discreet fold having carbohydrate-binding activity. A few exceptions are CBMs in cellulosomal scaffold in proteins and rare instances of independent putative CBMs. The requirement of CBMs existing as modules within larger enzymes sets this class of carbohydrate-binding protein apart from other non-catalytic sugar binding proteins such as lectins and sugar transport proteins. CBMs were previously classified as cellulose-binding domains (CBDs) based on the initial discovery of several modules that bound cellulose (Tomme et al., Eur J Biochem, 170:575-581, 1988; and Gilkes et al., J Biol Chem, 263:10401-10407, 1988). However, additional modules in carbohydrate-active enzymes are continually being found that bind carbohydrates other than cellulose yet otherwise meet the CBM criteria, hence the need to reclassify these polypeptides using more inclusive terminology. Previous classification of cellulose-binding domains was based on amino acid similarity. Groupings of CBDs were called "Types" and numbered with roman numerals (e.g. Type I or Type II CBDs). In keeping with the glycoside hydrolase classification, these groupings are now called families and numbered with Arabic numerals. Families 1 to 13 are the same as Types I to XIII (Tomme et al., in Enzymatic Degradation of Insoluble Polysaccharides (Saddler, J.N. & Penner, M., eds.), Cellulose-binding domains: classification and properties. pp. 142-163, American Chemical Society, Washington, 1995). A detailed review on the structure and binding modes of CBMs can be found in (Boraston et al., Biochem J, 382:769-81, 2004). The family classification of CBMs is expected to: aid in the identification of CBMs, in some cases, predict binding specificity, aid in identifying functional residues, reveal evolutionary relationships and possibly be predictive of polypeptide folds. Because the fold of proteins is better conserved than their sequences, some of the CBM families can be grouped into superfamilies or clans. The current CBM families are 1-63. CBMs/CBDs have also been found in algae, *e.g.,* the red alga *Porphyra purpurea* as a non-hydrolytic polysaccharide-binding protein. However, most of the CBDs are from cellullases and xylanases. CBDs are found at the N-and C-termini of proteins or are internal. Enzyme hybrids are known in the art (See *e.g.,* WO 90/00609 and WO 95/16782) and may be prepared by transforming into a host cell a DNA construct comprising at least a fragment of DNA encoding the cellulose-binding domain ligated, with or without a linker, to a DNA sequence encoding a disclosed polypeptide and growing the host cell to express the fused gene. Enzyme hybrids may be described by the following formula: CBM-MR-X or X-MR-CBM

In the above formula, the CBM is the N-terminal or the C-terminal region of an amino acid sequence corresponding to at least the carbohydrate-binding module; MR is the middle region (the linker), and may be a bond, or a short linking group preferably of from about 2 to about 100 carbon atoms, more preferably of from 2 to 40 carbon atoms; or is preferably from about 2 to about 100 amino acids, more preferably from 2 to 40 amino acids; and X is an N-terminal or C-terminal region of a polypeptide or active fragment having mannanase catalytic activity. In addition, a mannanase may contain more than one CBM or other module(s)/domain(s) of non-glycolytic function. The terms "module" and "domain" are used interchangeably in the present disclosure.

Suitable enzymatically active domains possess an activity that supports the action of a polypeptide in producing a desired product. Non-limiting examples of catalytic domains include: cellulases, hemicellulases such as xylanase, exo-mannanases, glucanases, arabinases, galactosidases, pectinases, and/or other activities such as proteases, lipases, acid phosphatases and/or others or functional fragments thereof. Fusion proteins are optionally linked to a polypeptide through a linker sequence that simply joins a polypeptide and the fusion domain without significantly affecting the properties of either component, or the linker optionally has a functional importance for the intended application.

Alternatively, polypeptides described herein are used in conjunction with one or more additional proteins of interest. Non-limiting examples of proteins of interest include: acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, oxidases, pectate lyases, pectin acetyl esterases, pectinases, pentosanases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases and/or other enzymes.

In other embodiments, a polypeptide as described is fused to a signal peptide for directing the extracellular secretion of the polypeptide. For example, in certain embodiments, the signal peptide is the native signal peptide of the polypeptide. In other embodiments, the signal peptide is a non-native signal peptide such as the *B. subtilis* AprE signal peptide. In some embodiments, a polypeptide has an N-terminal extension of Ala-Gly-Lys between the mature form and the signal peptide.

In some embodiments, a polypeptide or active fragment as described is expressed in a heterologous organism, *i.e.,* an organism other than *Paenibacillus and Bacillus spp..* Exemplary heterologous organisms are Gram(+) bacteria such as *Bacillus subtilis, Bacillus licheniformis, Bacillus lentus, Bacillus brevis, Geobacillus* (formerly *Bacillus) stearothermophilus, Bacillus alkalophilus, Bacillus amyloliquefaciens, Bacillus coagulans, Bacillus circulans, Bacillus lautus, Bacillus megaterium, Bacillus thuringiensis, Streptomyces lividans,* or *Streptomyces murinus;* Gram(-) bacteria such as *Escherichia coli.;* yeast such as *Saccharomyces* spp. or *Schizosaccharomyces* spp., *e.g. Saccharomyces cerevisiae;* and filamentous fungi such as *Aspergillus* spp., *e.g., Aspergillus oryzae* or *Aspergillus niger,* and *Trichoderma reesei.* Methods from transforming nucleic acids into these organisms are well known in the art. A suitable procedure for transformation of *Aspergillus* host cells is described in EP 238 023.

In particular embodiments, a polypeptide or active fragment is expressed in a heterologous organism as a secreted polypeptide.

### Polynucleotides

Also described herein is a polynucleotide that encodes a polypeptide for use in the present invention (including active fragments thereof). In one aspect, the polynucleotide is provided in the context of an expression vector for directing the expression of a polypeptide in a heterologous organism, such as those identified, herein. The polynucleotide that encodes a polypeptide may be operably-linked to regulatory elements (e.g., a promoter, terminator, enhancer, and the like) to assist in expressing the encoded polypeptides.

Exemplary polynucleotide sequences encoding a polypeptide for use in the present invention has the nucleotide sequence of SEQ ID NO: 7, 9, 11, 41, 45 or 49. Similar, including substantially identical, polynucleotides encoding suitable polypeptides may occur in nature, *e.g*., in other strains or isolates of *B. agaradhaerens.* In view of the degeneracy of the genetic code, it will be appreciated that polynucleotides having different nucleotide sequences may encode the same polypeptide.

In some embodiments, polynucleotides can have a specified degree of nucleotide sequence identity to the polynucleotides described herein, *e.g.,* at least 60%, 65%, 70%, 75%, 80%, 85%, 90%, 91%, 92%, 93%, 94%, 95%, 96%, 97%, 98%, 99% or greater identity to the nucleotide sequence selected from the group consisting of SEQ ID NO: 7, 9, 11, 41, 45 or 49. Homology can be determined by amino acid sequence alignment, *e.g*., using a program such as BLAST, ALIGN, or CLUSTAL, as described herein.

In some embodiments, the polynucleotide that encodes a polypeptide for use in the present invention is fused in frame behind *(i.e.,* downstream of) a coding sequence for a signal peptide for directing the extracellular secretion of the polypeptide. Heterologous signal sequences include those from bacterial cellulase genes. Expression vectors may be provided in a heterologous host cell suitable for expressing a polypeptide, or suitable for propagating the expression vector prior to introducing it into a suitable host cell.

A polynucleotide may be naturally occurring or synthetic *(i.e.,* man-made), and may be codon-optimized for expression in a different host, mutated to introduce cloning sites, or otherwise altered to add functionality.

### Vectors and Host Cells

In order to produce a disclosed polypeptide, the DNA encoding the polypeptide can be chemically synthesized from published sequences or obtained directly from host cells harboring the gene *(e.g.,* by cDNA library screening or PCR amplification). In some embodiments, a polynucleotide is included in an expression cassette and/or cloned into a suitable expression vector by standard molecular cloning techniques. Such expression cassettes or vectors contain sequences that assist initiation and termination of transcription (e.g., promoters and terminators), and generally contain a selectable marker.

The expression cassette or vector is introduced in a suitable expression host cell, which then expresses the corresponding polynucleotide. Particularly suitable expression hosts are bacterial expression host genera including *Escherichia (e.g., Escherichia coli), Pseudomonas (e.g., P. fluorescens or P. stutzerei*)*, Proteus (e.g., Proteus mirabilis), Ralstonia (e.g., Ralstonia eutropha), Streptomyces, Staphylococcus (e.g., S. carnosus*)*, Lactococcus (e.g., L. lactis), or Bacillus (subtilis, megaterium, licheniformis, etc.).* Also particularly suitable are yeast expression hosts such as *Saccharomyces cerevisiae, Schizosaccharomyces pombe, Yarrowia lipolytica, Hansenula polymorpha, Kluyveromyces lactis or Pichia pastoris.* Especially suited are fungal expression hosts such as *Aspergillus niger, Chrysosporium lucknowense, Aspergillus (e.g., A. oryzae, A. niger, A. nidulans, etc.)* or *Trichoderma reesei.* Also suited are mammalian expression hosts such as mouse (*e.g*., NS0), Chinese Hamster Ovary (CHO) or Baby Hamster Kidney (BHK) cell lines. Other eukaryotic hosts such as insect cells or viral expression systems (e.g., bacteriophages such as M13, T7 phage or Lambda, or viruses such as Baculovirus) are also suitable for producing a polypeptide.

Promoters and/or signal sequences associated with secreted proteins in a particular host of interest are candidates for use in the heterologous production and secretion of endo-β-mannanases in that host or in other hosts. As an example, in filamentous fungal systems, the promoters that drive the genes for cellobiohydrolase I (cbh1), glucoamylase A (glaA), TAKA-amylase (amyA), xylanase (exlA), the gpd-promoter cbh1, cbhll, endoglucanase genes EGI-EGV, Cel61B, Cel74A, egl1-egl5, gpd promoter, Pgk1, pki1, EF-1alpha, tef1, cDNA1 and hex1 are particularly suitable and can be derived from a number of different organisms (*e.g., A. niger, T. reesei, A. oryzae, A. awamori* and A. *nidulans*). A polynucleotide encoding a polypeptide for use in the present invention may be recombinantly associated with a polynucleotide encoding a suitable homologous or heterologous signal sequence that leads to secretion of the polypeptide into the extracellular (or periplasmic) space, thereby allowing direct detection of enzyme activity in the cell supernatant (or periplasmic space or lysate). Particularly suitable signal sequences for *Escherichia coli,* other Gram negative bacteria and other organisms known in the art include those that drive expression of the HlyA, DsbA, Pbp, PhoA, PelB, OmpA, OmpT or M13 phage Gill genes. For *Bacillus subtilis,* Gram-positive organisms and other organisms known in the art, particularly suitable signal sequences further include those that drive expression of the AprE, NprB, Mpr, AmyA, AmyE, Blac, SacB, and for *S. cerevisiae* or other yeast, include the killer toxin, Bar1, Suc2, Mating factor alpha, InulA or Ggplp signal sequence. Signal sequences can be cleaved by a number of signal peptidases, thus removing them from the rest of the expressed protein. In some embodiments, the rest of the polypeptide is expressed alone or as a fusion with other peptides, tags or proteins located at the N- or C-terminus *(e.g.,* 6XHis, HA or FLAG tags). Suitable fusions include tags, peptides or proteins that facilitate affinity purification or detection *(e.g.,* BCE103, 6XHis, HA, chitin binding protein, thioredoxin or FLAG tags), as well as those that facilitate expression, secretion or processing of the target endo-β-mannanase. Suitable processing sites include enterokinase, STE13, Kex2 or other protease cleavage sites for cleavage in vivo or in vitro.

Polynucleotides can be introduced into expression host cells by a number of transformation methods including, but not limited to, electroporation, lipid-assisted transformation or transfection ("lipofection"), chemically mediated transfection *(e.g.,* CaCl and/or CaP), lithium acetate-mediated transformation *(e.g.,* of host-cell protoplasts), biolistic "gene gun" transformation, PEG-mediated transformation *(e.g.,* of host-cell protoplasts), protoplast fusion *(e.g.,* using bacterial or eukaryotic protoplasts), liposome-mediated transformation, *Agrobacterium tumefaciens,* adenovirus or other viral or phage transformation or transduction.

Alternatively, a polypeptide can be expressed intracellularly. Optionally, after intracellular expression of the enzyme variants, or secretion into the periplasmic space using signal sequences such as those mentioned above, a permeabilisation or lysis step can be used to release the polypeptide into the supernatant. The disruption of the membrane barrier is effected by the use of mechanical means such as ultrasonic waves, pressure treatment (French press), cavitation or the use of membrane-digesting enzymes such as lysozyme or enzyme mixtures. As a further alternative, the polynucleotides encoding the polypeptide can be expressed by use of a suitable cell-free expression system. In cell-free systems, the polynucleotide of interest is typically transcribed with the assistance of a promoter, but ligation to form a circular expression vector is optional. In other embodiments, RNA is exogenously added or generated without transcription and translated in cell free systems.

The polypeptides disclosed herein may have enzymatic activity over a broad range of pH conditions. In certain embodiments the disclosed polypeptides of the present invention have enzymatic activity from about pH 4.0 to about pH 11.0, or from about pH 4.5 to about pH 11.0. In preferred embodiments, the polypeptides have substantial enzymatic activity, for example, at least 50%, 60%, 70%, 80%, 90%, 95%, or 100% activity from about pH 4.0 to 11.0, pH 4.5 to 11.0, pH 4.5 to 9.0, pH 5.5 to 8.5, or pH 6.0 to 7.5. It should be noted that the pH values described herein may vary by ± 0.2. For example a pH value of about 8.0 could vary from pH 7.8 to pH 8.2.

The polypeptides disclosed herein may have enzymatic activity over a wide range of temperatures, *e.g*., from about 20°C or lower to 90°C, 30°C to 80°C, 40°C to 70°C, 45°C to 65°C, or 50°C to 60°C. In certain embodiments, the polypeptides have substantial enzymatic activity, for example, at least 50%, 60%, 70%, 80%, 90%, 95%, or 100% activity at a temperature range of about 20°C or lower to 90°C, 30°C to 80°C, 40°C to 70°C, 45°C to 65°C, or 50°C to 60°C. It should be noted that the temperature values described herein may vary by ± 0.2°C. For example a temperature of about 50°C could vary from 49.8°C to 50.2°C.

### Detergent Compositions

An aspect of the compositions and methods disclosed herein is a detergent composition comprising an isolated polypeptide as described (including active fragments thereof) and methods for using such compositions in cleaning applications. Cleaning applications include, but are not limited to, laundry or textile cleaning, laundry or textile softening, dishwashing (manual and automatic), stain pre-treatment, and the like. Particular applications are those where mannans (e.g., locust bean gum, guar gum, etc.) are a component of the soils or stains to be removed. Detergent compositions typically include an effective amount of any of the polypeptides described herein, *e.g.,* at least 0.0001 weight percent, from about 0.0001 to about 1, from about 0.001 to about 0.5, from about 0.01 to about 0.1 weight percent, or even from about 0.1 to about 1 weight percent, or more. An effective amount of a polypeptide in the detergent composition results in the polypeptide having enzymatic activity sufficient to hydrolyze a mannan-containing substrate, such as locust bean gum, guar gum, or combinations thereof.

Additionally, detergent compositions having a concentration from about 0.4 g/L to about 2.2 g/L, from about 0.4 g/L to about 2.0 g/L, from about 0.4 g/L to about 1.7 g/L, from about 0.4 g/L to about 1.5 g/L, from about 0.4 g/L to about 1 g/L, from about 0.4 g/L to about 0.8 g/L, or from about 0.4 g/L to about 0.5 g/L may be mixed with an effective amount of an isolated polypeptide as described. The detergent composition may also be present at a concentration of about 0.4 ml/L to about 2.6 ml/L, from about 0.4 ml/L to about 2.0 ml/L, from about 0.4 ml/L to about 1.5 m/L, from about 0.4 ml/L to about 1 ml/L, from about 0.4 ml/L to about 0.8 ml/L, or from about 0.4 ml/L to about 0.5 ml/L.

Unless otherwise noted, all component or composition levels provided herein are made in reference to the active level of that component or composition, and are exclusive of impurities, for example, residual solvents or by-products, which may be present in commercially available sources. Enzyme components weights are based on total active protein. All percentages and ratios are calculated by weight unless otherwise indicated. All percentages and ratios are calculated based on the total composition unless otherwise indicated. In the exemplified detergent compositions, the enzymes levels are expressed by pure enzyme by weight of the total composition and unless otherwise specified, the detergent ingredients are expressed by weight of the total compositions.

In some embodiments, the detergent composition comprises one or more surfactants, which may be non-ionic, semi-polar, anionic, cationic, zwitterionic, or combinations and mixtures thereof. The surfactants are typically present at a level of from about 0.1% to 60% by weight. Exemplary surfactants include but are not limited to sodium dodecylbenzene sulfonate, C12-14 pareth-7, C12-15 pareth-7, sodium C12-15 pareth sulfate, C14-15 pareth-4, sodium laureth sulfate (*e.g.,* Steol CS-370), sodium hydrogenated cocoate, C12 ethoxylates (Alfonic 1012-6, Hetoxol LA7, Hetoxol LA4), sodium alkyl benzene sulfonates *(e.g.,* Nacconol 90G), and combinations and mixtures thereof.

Anionic surfactants that may be used with the detergent compositions described herein include but are not limited to linear alkylbenzenesulfonate (LAS), alpha-olefinsulfonate (AOS), alkyl sulfate (fatty alcohol sulfate) (AS), alcohol ethoxysulfate (AEOS or AES), secondary alkanesulfonates (SAS), alpha-sulfo fatty acid methyl esters, alkyl- or alkenylsuccinic acid, or soap. It may also contain 0-40% of nonionic surfactant such as alcohol ethoxylate (AEO or AE), carboxylated alcohol ethoxylates, nonylphenol ethoxylate, alkylpolyglycoside, alkyldimethylamine oxide, ethoxylated fatty acid monoethanolamide, fatty acid monoethanolamide, polyhydroxy alkyl fatty acid amide *(e.g.,* as described in WO 92/06154), and combinations and mixtures thereof.

Nonionic surfactants that may be used with the detergent compositions described herein include but are not limited to polyoxyethylene esters of fatty acids, polyoxyethylene sorbitan esters *(e.g.,* TWEENs), polyoxyethylene alcohols, polyoxyethylene isoalcohols, polyoxyethylene ethers *(e.g.,* TRITONs and BRIJ), polyoxyethylene esters, polyoxyethylene-*p-*tert-octylphenols or octylphenyl-ethylene oxide condensates (e.g., NONIDET P40), ethylene oxide condensates with fatty alcohols *(e.g.,* LUBROL), polyoxyethylene nonylphenols, polyalkylene glycols (SYNPERONIC F108), sugar-based surfactants *(e.g.,* glycopyranosides, thioglycopyranosides), and combinations and mixtures thereof.

The detergent compositions disclosed herein may have mixtures that include, but are not limited to 5-15% anionic surfactants, < 5% nonionic surfactants, cationic surfactants, phosphonates, soap, enzymes, perfume, butylphenyl methylptopionate, geraniol, zeolite, polycarboxylates, hexyl cinnamal, limonene, cationic surfactants, citronellol, and benzisothiazolinone.

Detergent compositions may additionally include one or more detergent builders or builder systems, a complexing agent, a polymer, a bleaching system, a stabilizer, a foam booster, a suds suppressor, an anti-corrosion agent, a soil-suspending agent, an anti-soil redeposition agent, a dye, a bactericide, a hydrotope, a tarnish inhibitor, an optical brightener, a fabric conditioner, and a perfume. The detergent compositions may also include enzymes, including but not limited to proteases, amylases, cellulases, lipases, pectin degrading enzymes, xyloglucanases, or additional carboxylic ester hydrolases. The pH of the detergent compositions should be neutral to basic, as described herein.

In some embodiments incorporating at least one builder, the detergent compositions comprise at least about 1%, from about 3% to about 60% or even from about 5% to about 40% builder by weight of the cleaning composition. Builders may include, but are not limited to, the alkali metals, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1,3,5-trihydroxy benzene-2, 4, 6-trisulphonic acid, and carboxymethyloxysuccinic acid, the various alkali metals, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof. Indeed, it is contemplated that any suitable builder will find use in various embodiments of the present disclosure.

In some embodiments, the builders form water-soluble hardness ion complexes *(e.g.,* sequestering builders), such as citrates and polyphosphates *(e.g.,* sodium tripolyphosphate and sodium tripolyphospate hexahydrate, potassium tripolyphosphate, and mixed sodium and potassium tripolyphosphate, etc.). It is contemplated that any suitable builder will find use in the present disclosure, including those known in the art *(See, e.g.,* EP 2 100 949).

As indicated herein, in some embodiments, the cleaning compositions described herein further comprise adjunct materials including, but not limited to surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents *(See, e.g.,* U.S. Pat. Nos. 6,610,642; 6,605,458; 5,705,464; 5,710,115; 5,698,504; 5,695,679; 5,686,014; and 5,646,101). Embodiments of specific cleaning composition materials are exemplified in detail below. In embodiments in which the cleaning adjunct materials are not compatible with the polypeptides of the present invention in the cleaning compositions, suitable methods of keeping the cleaning adjunct materials and the endo-β-mannanase(s) separated (*i.e.,* not in contact with each other), until combination of the two components is appropriate, are used. Such separation methods include any suitable method known in the art *(e.g.,* gelcaps, encapsulation, tablets, physical separation, etc.).

The cleaning compositions described herein are advantageously employed for example, in laundry applications, hard surface cleaning, dishwashing applications, as well as cosmetic applications such as dentures, teeth, hair, and skin. In addition, due to the unique advantages of increased effectiveness in lower temperature solutions, the polypeptides described herein are ideally suited for laundry and fabric softening applications. Furthermore, the polypeptides of the present invention may find use in granular and liquid compositions.

A polypeptide or isolated polypeptide described herein may also find use cleaning in additive products. In some embodiments, low temperature solution cleaning applications find use. In some embodiments, the present disclosure provides cleaning additive products including at least one disclosed a polypeptide of the present invention is ideally suited for inclusion in a wash process when additional bleaching effectiveness is desired. Such instances include, but are not limited to low temperature solution cleaning applications. In some embodiments, the additive product is in its simplest form, one or more endo-β-mannanases. In some embodiments, the additive is packaged in dosage form for addition to a cleaning process. In some embodiments, the additive is packaged in dosage form for addition to a cleaning process where a source of peroxygen is employed and increased bleaching effectiveness is desired. Any suitable single dosage unit form finds use with the present disclosure, including but not limited to pills, tablets, gelcaps, or other single dosage units such as pre-measured powders or liquids. In some embodiments, filler(s) or carrier material(s) are included to increase the volume of such compositions. Suitable filler or carrier materials include, but are not limited to various salts of sulfate, carbonate, and silicate as well as talc, clay, and the like. Suitable filler or carrier materials for liquid compositions include, but are not limited to water or low molecular weight primary and secondary alcohols including polyols and diols. Examples of such alcohols include, but are not limited to methanol, ethanol, propanol, and isopropanol. In some embodiments, the compositions contain from about 5% to about 90% of such materials. Acidic fillers find use to reduce pH. Alternatively, in some embodiments, the cleaning additive includes adjunct ingredients, as described more fully below.

In one embodiment, the present cleaning compositions or cleaning additives contain an effective amount of at least one polypeptide described herein, optionally in combination with other endo-β-mannanases and/or additional enzymes. In certain embodiments, the additional enzymes include, but are not limited to, at least one enzyme selected from acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, metalloproteases, oxidases, pectate lyases, pectin acetyl esterases, pectinases, pentosanases, perhydrolases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, and mixtures thereof.

The required level of enzyme is achieved by the addition of one or more polypeptide or active fragment as described. Typically the present cleaning compositions will comprise at least about 0.0001 weight percent, from about 0.0001 to about 10, from about 0.001 to about 1, or even from about 0.01 to about 0.1 weight percent of at least one of the disclosed polypeptides.

The cleaning compositions herein are typically formulated such that, during use in aqueous cleaning operations, the wash water will have a pH of from about 3.0 to about 11. Liquid product formulations are typically formulated to have a neat pH from about 5.0 to about 9.0. Granular laundry products are typically formulated to have a pH from about 8.0 to about 11.0. Techniques for controlling pH at recommended usage levels include the use of buffers, alkalis, acids, etc., and are well known to those skilled in the art.

Suitable low pH cleaning compositions typically have a neat pH of from about 3.0 to about 5.0 or even from about 3.5 to about 4.5. Low pH cleaning compositions are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine, or hydrochloric acid, to provide such cleaning composition with a neat pH of from about 3.0 to about 5.0. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of the composition wherein the solvent is distilled water. In these embodiments, all pH measurements are taken at 20°C, unless otherwise indicated.

Suitable high pH cleaning compositions typically have a neat pH of from about 9.0 to about 11.0, or even a net pH of from 9.5 to 10.5. Such cleaning compositions typically comprise a sufficient amount of a pH modifier, such as sodium hydroxide, monoethanolamine, or hydrochloric acid, to provide such cleaning composition with a neat pH of from about 9.0 to about 11.0. Such compositions typically comprise at least one base-stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, when the polypeptide is employed in a granular composition or liquid, it is desirable for the polypeptide to be in the form of an encapsulated particle to protect the polypeptide from other components of the granular composition during storage. In addition, encapsulation is also a means of controlling the availability of the polypeptide during the cleaning process. In some embodiments, encapsulation enhances the performance of the polypeptide and/or additional enzymes. In this regard, the polypeptide is encapsulated with any suitable encapsulating material known in the art. In some embodiments, the encapsulating material typically encapsulates at least part of the catalyst for the polypeptide described herein. Typically, the encapsulating material is water-soluble and/or water-dispersible. In some embodiments, the encapsulating material has a glass transition temperature (Tg) of 0°C or higher. Glass transition temperature is described in more detail in the PCT application WO 97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch *(See, e.g.,* EP 0 922 499; U.S. 4,977,252; U.S. 5,354,559; and U.S. 5,935,826). In some embodiments, the encapsulating material is a microsphere made from plastic such as thermoplastics, acrylonitrile, methacrylonitrile, polyacrylonitrile, polymethacrylonitrile, and mixtures thereof; commercially available microspheres that find use include, but are not limited to those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

The term "granular composition" refers to a conglomeration of discrete solid, macroscopic particles. Powders are a special class of granular material due to their small particle size, which makes them more cohesive and more easily suspended.

In using detergent compositions that include a polypeptide of the present invention in cleaning applications, the fabrics, textiles, dishes, or other surfaces to be cleaned are incubated in the presence of a detergent composition for a time sufficient to allow the active polypeptide to hydrolyze mannan substrates including, but not limited to, locust bean gum, guar gum, and combinations thereof present in soil or stains, and then typically rinsed with water or another aqueous solvent to remove the detergent composition along with hydrolyzed mannans.

As described herein, the compositions of the present invention find particular use in the cleaning industry, including, but not limited to laundry and dish detergents. These applications place enzymes under various environmental stresses. A polypeptide as described may provide advantages over many currently used enzymes, due to their stability under various conditions.

Indeed, there are a variety of wash conditions including varying detergent formulations, wash water volumes, wash water temperatures, and lengths of wash time, to which endo-β-mannanases involved in washing are exposed. In addition, detergent formulations used in different geographical areas have different concentrations of their relevant components present in the wash water. For example, European detergents typically have about 4500-5000 ppm of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000 ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example about 667 ppm in Japan, to between about 800 ppm to about 2000 ppm ("medium detergent concentration geographies"), for example about 975 ppm in U.S. and about 1500 ppm in Brazil, to greater than about 2000 ppm ("high detergent concentration geographies"), for example about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C *(e.g.,* about 20°C), whereas the temperature of wash water in Europe is typically between about 30 and about 60°C *(e.g.,* about 40°C). Accordingly, in certain embodiments, the detergent compositions described herein may be utilized at temperature from about 10°C to about 60°C, or from about 20°C to about 60°C, or from about 30°C to about 60°C, or from about 40°C to about 60°C, as well as all other combinations within the range of about 40°C to about 55°C, and all ranges within 10°C to 60°C. However, in the interest of saving energy, many consumers are switching to using cold water washing. In addition, in some further regions, cold water is typically used for laundry, as well as dish washing applications. In some embodiments, the "cold water washing" of the present disclosure utilizes washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed Ca²⁺/Mg²⁺. Hardness is a measure of the amount of calcium (Ca²⁺) and magnesium (Mg²⁺) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

**Table II. Water Hardness Levels**

| **Water** | **Grains per gallon** | **Parts per million** |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed Ca²⁺/Mg²⁺ *(e.g.,* about 15 grains per gallon mixed Ca²⁺/Mg²⁺). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed Ca²⁺/Mg²⁺.

Accordingly, polypeptides and fragments as described may show surprising wash performance in at least one set of wash conditions *(e.g.,* water temperature, water hardness, and/or detergent concentration). In some embodiments, the polypeptide is comparable in wash performance to other endo-β-mannanases. In some embodiments, the polypeptide exhibits enhanced wash performance as compared to endo-β-mannanases currently commercially available. Thus, in some preferred embodiments, the polypeptide exhibits enhanced oxidative stability, enhanced thermal stability, enhanced cleaning capabilities under various conditions, and/or enhanced chelator stability. In addition, the polypeptide may find use in cleaning compositions that do not include detergents, again either alone or in combination with builders and stabilizers.

In some embodiments of the present disclosure, the cleaning compositions comprise at least one a polypeptide of the present invention of the present disclosure at a level from about 0.00001 % to about 10% by weight of the composition and the balance (e.g., about 99.999% to about 90.0%) comprising cleaning adjunct materials by weight of composition. In other aspects of the present disclosure, the cleaning compositions comprises at least one a polypeptide of the present invention at a level of about 0.0001% to about 10%, about 0.001% to about 5%, about 0.001% to about 2%, about 0.005% to about 0.5% by weight of the composition and the balance of the cleaning composition (e.g., about 99.9999% to about 90.0%, about 99.999 % to about 98%, about 99.995% to about 99.5% by weight) comprising cleaning adjunct materials.

In addition to the polypeptides provided herein, any other suitable endo-β-mannanases find use in the compositions described herein. Suitable endo-β-mannanases include, but are not limited to, endo-β-mannanases of the GH26 family of glycosyl hydrolases, endo-β-mannanases of the GH5 family of glycosyl hydrolases, acidic endo-β-mannanases, neutral endo-β-mannanases, and alkaline endo-β-mannanases. Examples of alkaline endo-β-mannanases include those described in U.S. Pat. Nos. 6,060,299, 6,566,114, and 6,602,842; WO 9535362A1, WO 9964573A1, WO9964619A1, and WO2015022428. Additionally, suitable endo-β-mannanases include, but are not limited to those of animal, plant, fungal, or bacterial origin. Chemically or genetically modified mutants are encompassed by the present disclosure.

Examples of useful endo-β-mannanases include *Bacillus* endo-β-mannanases such as *B. subtilis* endo-β-mannanase *(See, e.g.,* U.S. Pat. No. 6, 060,299, and WO 9964573A1), *B.* sp. 1633 endo-β-mannanase *(See, e.g.,* U.S. Pat. No. 6,566,114 and WO9964619A1), *Bacillus* sp. AAI12 endo-β-mannanase *(See, e.g.,* U.S. Pat. No. 6,566,114 and WO9964619A1), *B.* sp. AA349 endo-β-mannanase *(See, e.g.,* U.S. Pat. No. 6,566,114 and WO9964619A1), *B. agaradhaerens* NCIMB 40482 endo-β-mannanase *(See, e.g.,* U.S. Pat. No. 6,566,114 and WO9964619A1), *B. halodurans* endo-β-mannanase, *B. clausii* endo-β-mannanase (*See, e.g.,* U.S. Pat. No. 6,566,114 and WO9964619A1), *B. licheniformis* endo-β-mannanase (*See, e.g.,* U.S. Pat. No. 6,566,114 and WO9964619A1), *Humicola* endo-β-mannanases such as *H. insolens* endo-β-mannanase *(See, e.g.,* U.S. Pat. No. 6,566,114 and WO9964619A1), and *Caldocellulosiruptor* endo-β-mannanases such as C. sp. endo-β-mannanase *(See, e.g.,* U.S. Pat. No. 6,566,114 and WO9964619A1).

Furthermore, a number of identified mannanases (*i.e*., endo-β-mannanases and exo-β-mannanases) find use in some embodiments of the present disclosure, including but not limited to *Agaricus bisporus* mannanase *(See,* Tang et al., [2001] Appl. Environ. Microbiol. 67: 2298-2303), *Aspergillu tamarii* mannanase *(See,* Civas et al., [1984] Biochem. J. 219: 857-863), *Aspergillus aculeatus* mannanase *(See,* Christgau et al., [1994] Biochem. Mol. Biol. Int. 33: 917-925), *Aspergillus awamori* mannanase *(See,* Setati et al., [2001] Protein Express Purif. 21: 105-114), *Aspergillus fumigatus* mannanase *(See,* Puchart et al., [2004] Biochimica et biophysica Acta. 1674: 239-250), *Aspergillus niger* mannanase *(See,* Ademark et al., [1998] J. Biotechnol. 63: 199-210), *Aspergillus oryzae* NRRL mannanase *(See,* Regalado et al., [2000] J. Sci. Food Agric. 80: 1343-1350), *Aspergillus sulphureus* mannanase *(See,* Chen et al., [2007] J. Biotechnol. 128(3): 452-461), *Aspergillus terrus* mannanase *(See,* Huang et al., [2007] Wei Sheng Wu Xue Bao. 47(2): 280-284), *Paenibacillus and Bacillus spp.* mannanase *(See,* U.S. Pat No. 6,376,445.), *Bacillus* AM001 mannanase *(See,* Akino et al., [1989] Arch. Microbiol. 152: 10-15), *Bacillus brevis* mannanase *(See,* Araujo and Ward, [1990] J. Appl. Bacteriol. 68: 253-261), *Bacillus circulans* K-1 mannanase *(See,* Yoshida et al., [1998] Biosci. Biotechnol. Biochem. 62(3): 514-520), *Bacillus polymyxa* mannanase *(See,* Araujo and Ward, [1990] J. Appl. Bacteriol. 68: 253-261), *Bacillus sp* JAMB-750 mannanase *(See,* Hatada et al., [2005] Extremophiles. 9: 497-500), *Bacillus sp.* M50 mannanase *(See,* Chen et al., [2000] Wei Sheng Wu Xue Bao. 40: 62-68), *Bacillus sp.* N 16-5 mannanase *(See,* Yanhe et al., [2004] Extremophiles 8: 447-454), *Bacillus stearothermophilu* mannanase *(See,* Talbot and Sygusch, [1990] Appl. Environ. Microbiol. 56: 3505-3510), *Bacillus subtilis* mannanase *(See,* Mendoza et al., [1994] World J. Microbiol. Biotechnol. 10: 51-54), *Bacillus subtilis* B36 mannanase (Li et al., [2006] Z. Naturforsch (C). 61: 840-846), *Bacillus subtilis* BM9602 mannanase *(See,* Cui et al., [1999] Wei Sheng Wu Xue Bao. 39(1): 60-63), *Bacillus subtilis SA-22* mannanase *(See,* Sun et al., [2003] Sheng Wu Gong Cheng Xue Bao. 19(3): 327-330), *Bacillus subtilis* 168 mannanase *(See,* Helow and Khattab, [1996] Acta Microbiol. Immunol. Hung. 43: 289-299), *Bacteroides ovatus* mannanase *(See,* Gherardini et al., [1987] J. Bacteriol. 169: 2038-2043), *Bacteroides ruminicola* mannanase *(See,* Matsushita et al., [1991] J. Bacteriol. 173: 6919-6926), *Caldibacillus cellulovorans* mannanase *(See,* Sunna et al., [2000] Appl. Environ. Microbiol. 66: 664-670), *Caldocellulosiruptor saccharolyticus* mannanase *(See,* Morris et al., [1995] Appl. Environ. Microbiol. 61: 2262-2269), *Caldocellum saccharolyticum* mannanase *(See,* Bicho et al., [1991] Appl. Microbiol. Biotechnol. 36: 337-343), *Cellulomonas fimi* mannanase *(See,* Stoll et al., [1999] Appl. Environ. Microbiol. 65(6):2598-2605), *Clostridium butyricum*/ *beijerinckii* mannanase *(See,* Nakajima and Matsuura, [1997] Biosci. Biotechnol. Biochem. 61: 1739-1742), *Clostridium cellulolyticum* mannanase *(See,* Perret et al., [2004] Biotechnol. Appl. Biochem. 40: 255-259), *Clostridium tertium* mannanase *(See,* Kataoka and Tokiwa, [1998] J. Appl. Microbiol. 84: 357-367), *Clostridium thermocellum* mannanase *(See,* Halstead et al., [1999] Microbiol. 145: 3101-3108), *Dictyoglomus thermophilum* mannanase *(See,* Gibbs et al., [1999] Curr. Microbiol. 39(6): 351-357), *Flavobacterium sp* mannanase *(See,* Zakaria et al., [1998] Biosci. Biotechnol. Biochem. 62: 655-660), *Gastropoda pulmonata* mannanase *(See,* Charrier and Rouland, [2001] J. Expt. Zool. 290: 125-135), *Littorina brevicula* mannanase *(See,* Yamamura et al., [1996] Biosci. Biotechnol. Biochem. 60: 674-676), *Lycopersicon esculentum* mannanase *(See,* Filichkin et al., [2000] Plant Physiol. 134:1080-1087), *Paenibacillus curdlanolyticus* mannanase *(See,* Pason and Ratanakhanokchai, [2006] Appl. Environ. Microbiol. 72: 2483-2490), *Paenibacillus polymyxa* mannanase *(See,* Han et al., [2006] Appl. Microbiol Biotechnol. 73(3): 618-630), *Phanerochaete chrysosporium* mannanase *(See,* Wymelenberg et al., [2005] J. Biotechnol. 118: 17-34), *Piromyces sp.* mannanase *(See,* Fanutti et al., [1995] J. Biol. Chem. 270(49): 29314-29322), *Pomacea insulars* mannanase *(See,* Yamamura et al., [1993] Biosci. Biotechnol. Biochem. 7: 1316-1319), *Pseudomonas fluorescens* subsp. Cellulose mannanase *(See,* Braithwaite et al., [1995] Biochem J. 305: 1005-1010), *Rhodothermus marinus* mannanase *(See,* Politz et al., [2000] Appl. Microbiol. Biotechnol. 53 (6): 715-721), *Sclerotium rolfsii* mannanase *(See,* Sachslehner et al., [2000] J. Biotechnol. 80:127-134), *Streptomyces galbus* mannanase *(See,* Kansoh and Nagieb, [2004] Anton. van. Leeuwonhoek. 85: 103-114), *Streptomyces lividans* mannanase (*See,* Arcand et al., [1993] J.Biochem. 290: 857-863), *Thermoanaerobacterium Polysaccharolyticum* mannanase *(See,* Cann et al., [1999] J. Bacteriol. 181: 1643-1651), *Thermomonospora fusca* mannanase *(See,* Hilge et al., [1998] Structure 6: 1433-1444), *Thermotoga maritima* mannanase *(See,* Parker et al., [2001] Biotechnol. Bioeng. 75(3): 322-333), *Thermotoga neapolitana* mannanase *(See,* Duffaud et al., [1997] Appl. Environ. Microbiol. 63: 169-177), *Trichoderma harzanium* strain T4 mannanase *(See,* Franco et al., [2004] Biotechnol Appl. Biochem. 40: 255-259), *Trichoderma reesei* mannanase *(See,* Stalbrand et al., [1993] J. Biotechnol. 29: 229-242), and *Vibrio sp.* mannanase *(See,* Tamaru et al., [1997] J. Ferment. Bioeng. 83: 201-205).

Additional suitable endo-β-mannanases include commercially available endo-β-mannanases such as HEMICELL® (Chemgen); GAMANASE® and MANNAWAY®, (Novozymes A/S, Denmark); PURABRITE™ and MANNASTAR™ (Genencor, A Danisco Division, Palo Alto, CA); and PYROLASE® 160 and PYROLASE® 200 (Diversa).

In some embodiments of the present disclosure, the cleaning compositions of the present disclosure further comprise endo-β-mannanases at a level from about 0.00001% to about 10% of additional endo-β-mannanase by weight of the composition and the balance of cleaning adjunct materials by weight of composition. In other aspects of the present disclosure, the cleaning compositions of the present disclosure also comprise endo-β-mannanases at a level of about 0.0001% to about 10%, about 0.001% to about 5%, about 0.001% to about 2%, about 0.005% to about 0.5% endo-β-mannanase by weight of the composition.

In some embodiments of the present disclosure, any suitable protease may be used. Suitable proteases include those of animal, vegetable or microbial origin. In some embodiments, chemically or genetically modified mutants are included. In some embodiments, the protease is a serine protease, preferably an alkaline microbial protease or a trypsin-like protease. Various proteases are described in PCT applications WO 95/23221 and WO 92/21760; U.S. Pat. Publication No. 2008/0090747; and U.S. Pat. Nos. 5,801,039; 5,340,735; 5,500,364; 5,855,625; U.S. RE 34,606; 5,955,340; 5,700,676; 6,312,936; 6,482,628; and various other patents. In some further embodiments, metalloproteases find use in the present disclosure, including but not limited to the neutral metalloprotease described in PCT application WO 07/044993. Commercially available protease enzymes that find use in the present invention include, but are not limited to MAXATASE®, MAXACAL™, MAXAPEM™, OPTICLEAN®, OPTIMASE®, PROPERASE®, PURAFECT®, PURAFECT® OXP, PURAMAX™, EXCELLASE™, PREFERENZ™ proteases (e.g. P100, P110, P280), EFFECTENZ™ proteases (e.g. P1000, P1050, P2000), EXCELLENZ™ proteases (e.g. P1000), ULTIMASE®, and PURAFAST™ (DuPont); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); BLAP™ and BLAP™ variants (Henkel Kommanditgesellschaft auf Aktien, Duesseldorf, Germany), and KAP *(B. alkalophilus* subtilisin; Kao Corp., Tokyo, Japan).

In some embodiments of the present disclosure, any suitable amylase may be used. In some embodiments, any amylase (e.g., alpha and/or beta) suitable for use in alkaline solutions also find use. Suitable amylases include, but are not limited to those of bacterial or fungal origin. Chemically or genetically modified mutants are included in some embodiments. Amylases that find use in the present disclosure include, but are not limited to α-amylases obtained from *B. licheniformis* (*See, e.g.,* GB 1,296,839). Commercially available amylases that find use in the present disclosure include, but are not limited to DURAMYL®, TERMAMYL®, FUNGAMYL®, STAINZYME®, STAINZYME PLUS®, STAINZYME ULTRA®, and BAN™ (Novozymes A/S, Denmark), as well as PURASTAR®, POWERASE™, RAPIDASE®, and MAXAMYL® P (Genencor, A Danisco Division, Palo Alto, CA).

In some embodiments of the present disclosure, the disclosed cleaning compositions further comprise amylases at a level from about 0.00001% to about 10% of additional amylase by weight of the composition and the balance of cleaning adjunct materials by weight of composition. In other aspects of the present disclosure, the cleaning compositions also comprise amylases at a level of about 0.0001% to about 10%, about 0.001% to about 5%, about 0.001% to about 2%, about 0.005% to about 0.5% amylase by weight of the composition.

In some embodiments of the present disclosure, any suitable pectin degrading enzyme may be used. As used herein, "pectin degrading enzyme(s)" encompass arabinanase (EC 3.2.1.99), galactanases (EC 3.2.1.89), polygalacturonase (EC 3.2.1.15) exo-polygalacturonase (EC 3.2.1.67), exo-poly-alpha-galacturonidase (EC 3.2.1.82), pectin lyase (EC 4.2.2.10), pectin esterase (EC 3.2.1.11), pectate lyase (EC 4.2.2.2), exo-polygalacturonate lyase (EC 4.2.2.9) and hemicellulases such as endo-1,3-β-xylosidase (EC 3.2.1.32), xylan-1,4-β-xylosidase (EC 3.2.1.37) and α-L-arabinofuranosidase (EC 3.2.1.55). Pectin degrading enzymes are natural mixtures of the above mentioned enzymatic activities. Pectin enzymes therefore include the pectin methylesterases which hdyrolyse the pectin methyl ester linkages, polygalacturonases which cleave the glycosidic bonds between galacturonic acid molecules, and the pectin transeliminases or lyases which act on the pectic acids to bring about non-hydrolytic cleavage of α-1,4 glycosidic linkages to form unsaturated derivatives of galacturonic acid.

Suitable pectin degrading enzymes include those of plant, fungal, or microbial origin. In some embodiments, chemically or genetically modified mutants are included. In some embodiments, the pectin degrading enzymes are alkaline pectin degrading enzymes, *i.e.,* enzymes having an enzymatic activity of at least 10%, preferably at least 25%, more preferably at least 40% of their maximum activity at a pH of from about 7.0 to about 12. In certain other embodiments, the pectin degrading enzymes are enzymes having their maximum activity at a pH of from about 7.0 to about 12. Alkaline pectin degrading enzymes are produced by alkalophilic microorganisms *e.g*., bacterial, fungal, and yeast microorganisms such as Bacillus species. In some embodiments, the microorganisms are *Bacillus firmus, Bacillus circulans,* and Bacillus *subtilis* as described in JP 56131376 and JP 56068393. Alkaline pectin decomposing enzymes may include but are not limited to galacturn-1,4-α-galacturonase (EC 3.2.1.67), polygalacturonase activities (EC 3.2.1.15, pectin esterase (EC 3.1.1.11), pectate lyase (EC 4.2.2.2) and their iso enzymes. Alkaline pectin decomposing enzymes can be produced by the Erwinia species. In some embodiments, the alkaline pectin decomposing enzymes are produced by *E*. *chrysanthemi, E. carotovora, E. amylovora, E. herbicola,* and *E. dissolvens* as described in JP 59066588, JP 63042988, and in World J. Microbiol. Microbiotechnol. (8, 2, 115-120) 1992. In certain other embodiments, the alkaline pectin enzymes are produced by Bacillus species as disclosed in JP 73006557 *and* Agr. Biol. Chem. (1972), 36 (2) 285-93.

In some embodiments of the present disclosure, the disclosed cleaning compositions further comprise pectin degrading enzymes at a level from about 0.00001% to about 10% of additional pectin degrading enzyme by weight of the composition and the balance of cleaning adjunct materials by weight of composition. In other aspects of the present disclosure, the cleaning compositions also comprise pectin degrading enzymes at a level of about 0.0001% to about 10%, about 0.001% to about 5%, about 0.001% to about 2%, about 0.005% to about 0.5% pectin degrading enzyme by weight of the composition.

In some other embodiments, any suitable xyloglucanase finds used in the cleaning compositions of the present disclosure. Suitable xyloglucanases include, but are not limited to those of plant, fungal, or bacterial origin. Chemically or genetically modified mutants are included in some embodiments. As used herein, "xyloglucanase(s)" encompass the family of enzymes described by Vincken and Voragen at Wageningen University [Vincken et al (1994) Plant Physiol., 104, 99-107] and are able to degrade xyloglucans as described in Hayashi et al (1989) Plant. Physiol. Plant Mol. Biol., 40, 139-168. Vincken et al demonstrated the removal of xyloglucan coating from cellulose of the isolated apple cell wall by a xyloglucanase purified from *Trichoderma viride* (endo-IV-glucanase). This enzyme enhances the enzymatic degradation of cell wall-embedded cellulose and work in synergy with pectic enzymes. Rapidase LIQ+ from Gist-Brocades contains a xyloglucanase activity.

In some embodiments of the present disclosure, the disclosed cleaning compositions further comprise xyloglucanases at a level from about 0.00001% to about 10% of additional xyloglucanase by weight of the composition and the balance of cleaning adjunct materials by weight of composition. In other aspects of the present disclosure, the cleaning compositions also comprise xyloglucanases at a level of about 0.0001% to about 10%, about 0.001% to about 5%, about 0.001% to about 2%, about 0.005% to about 0.5% xyloglucanase by weight of the composition. In certain other embodiments, xyloglucanases for specific applications are alkaline xyloglucanases, *i.e.,* enzymes having an enzymatic activity of at least 10%, preferably at lest 25%, more preferably at least 40% of their maximum activity at a pH ranging from 7 to 12. In certain other embodiments, the xyloglucanases are enzymes having their maximum activity at a pH of from about 7.0 to about 12.

In some further embodiments, any suitable cellulase finds used in the cleaning compositions of the present disclosure. Suitable cellulases include, but are not limited to those of bacterial or fungal origin. Chemically or genetically modified mutants are included in some embodiments. Suitable cellulases include, but are not limited to *Humicola insolens* cellulases *(See, e.g.,* U.S. Pat. No. 4,435,307). Especially suitable cellulases are the cellulases having color care benefits *(See, e.g.,* EP 0 495 257). Commercially available cellulases that find use in the present disclosure include, but are not limited to ENDOLASE®, CELLUCLEAN®, CELLUZYME®, CAREZYME® (Novozymes A/S, Denmark). Additional commercially available cellulases include PURADEX® (Genencor, A Danisco Division, Palo Alto, CA) and KAC-500(B)™ (Kao Corporation). In some embodiments, cellulases are incorporated as portions or fragments of mature wild-type or variant cellulases, wherein a portion of the N-terminus is deleted *(See, e.g.,* U.S. Pat. No. 5,874,276). In some embodiments, the cleaning compositions of the present disclosure further comprise cellulases at a level from about 0.00001% to about 10% of additional cellulase by weight of the composition and the balance of cleaning adjunct materials by weight of composition. In other aspects of the present disclosure, the cleaning compositions also comprise cellulases at a level of about 0.0001% to about 10%, about 0.001% to about 5%, about 0.001% to about 2%, about 0.005% to about 0.5% cellulase by weight of the composition.

In still further embodiments, any lipase suitable for use in detergent compositions also finds use in the present disclosure. Suitable lipases include, but are not limited to those of bacterial or fungal origin. Chemically or genetically modified mutants are included in some embodiments. Examples of useful lipases include *Humicola lanuginosa* lipase *(See, e.g.,* EP 258 068, and EP 305 216), *Rhizomucor miehei* lipase *(See, e.g.,* EP 238 023), *Candida* lipase, such as *C. antarctica* lipase *(e.g.,* the *C. antarctica* lipase A or B; *see, e.g.,* EP 214 761), *Pseudomonas* lipases such as *P. alcaligenes* lipase and *P. pseudoalcaligenes* lipase *(See, e.g.,* EP 218 272), *P. cepacia* lipase *(See, e.g.,* EP 331 376), *P. stutzeri* lipase *(See, e.g.,* GB 1,372,034), *P. fluorescens* lipase, *Bacillus* lipase (*e.g., B. subtilis* lipase [Dartois et al., (1993) Biochem. Biophys. Acta 1131:253-260]; *B. stearothermophilus* lipase [*See, e.g.,* JP 64/744992]; and *B*. *pumilus* lipase [*See, e.g.,* WO 91/16422]). Furthermore, a number of cloned lipases find use in some embodiments of the present disclosure, including but not limited to *Penicillium camembertii* lipase *(See,* Yamaguchi et al., [1991] Gene 103:61-67), *Geotricum candidum* lipase *(See,* Schimada et al., [1989] J. Biochem. 106:383-388), and various *Rhizopus* lipases such as *R. delemar* lipase *(See,* Hass et al., [1991] Gene 109:117-113), *R. niveus* lipase (Kugimiya et al., [1992] Biosci. Biotech. Biochem. 56:716-719), and *R*. *oryzae* lipase. Other types of lipolytic enzymes such as cutinases also find use in some embodiments of the present disclosure, including but not limited to the cutinase derived from *Pseudomonas mendocina* (*See,* WO 88/09367), and the cutinase derived from *Fusarium solani pisi* (*See,* WO 90/09446). Additional suitable lipases include commercially available lipases such as M1 LIPASE™, LUMA FAST™, and LIPOMAX™ (Genencor, A Danisco Division, Palo Alto, CA); LIPEX®, LIPOCLEAN®, LIPOLASE® and LIPOLASE® ULTRA (Novozymes A/S, Denmark); and LIPASE P™ "Amano" (Amano Pharmaceutical Co. Ltd., Japan).

In some embodiments, the disclosed cleaning compositions further comprise lipases at a level from about 0.00001 % to about 10% of additional lipase by weight of the composition and the balance of cleaning adjunct materials by weight of composition. In other aspects of the present disclosure, the cleaning compositions also comprise lipases at a level of about 0.0001% to about 10%, about 0.001% to about 5%, about 0.001% to about 2%, about 0.005% to about 0.5% lipase by weight of the composition.

In some embodiments, peroxidases are used in combination with hydrogen peroxide or a source thereof *(e.g.,* a percarbonate, perborate or persulfate) in the compositions of the present disclosure. In some alternative embodiments, oxidases are used in combination with oxygen. Both types of enzymes are used for "solution bleaching" (*i.e.,* to prevent transfer of a textile dye from a dyed fabric to another fabric when the fabrics are washed together in a wash liquor), preferably together with an enhancing agent *(See, e.g.,* WO 94/12621 and WO 95/01426). Suitable peroxidases/oxidases include, but are not limited to those of plant, bacterial or fungal origin. Chemically or genetically modified mutants are included in some embodiments. In some embodiments, the cleaning compositions of the present disclosure further comprise peroxidase and/or oxidase enzymes at a level from about 0.00001% to about 10% of additional peroxidase and/or oxidase by weight of the composition and the balance of cleaning adjunct materials by weight of composition. In other aspects of the present disclosure, the cleaning compositions also comprise peroxidase and/or oxidase enzymes at a level of about 0.0001% to about 10%, about 0.001% to about 5%, about 0.001% to about 2%, about 0.005% to about 0.5% peroxidase and/or oxidase enzymes by weight of the composition.

In some embodiments, additional enzymes find use, including but not limited to perhydrolases *(See, e.g.,* WO 05/056782). In addition, in some particularly preferred embodiments, mixtures of the above mentioned enzymes are encompassed herein, in particular one or more additional protease, amylase, lipase, mannanase, and/or at least one cellulase. Indeed, it is contemplated that various mixtures of these enzymes will find use in the present disclosure. It is also contemplated that the varying levels of a polypeptide of the present invention(s) and one or more additional enzymes may both independently range to about 10%, the balance of the cleaning composition being cleaning adjunct materials. The specific selection of cleaning adjunct materials are readily made by considering the surface, item, or fabric to be cleaned, and the desired form of the composition for the cleaning conditions during use (*e.g.,* through the wash detergent use).

Examples of suitable cleaning adjunct materials include, but are not limited to, surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracis, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents *(See, e.g.,* U.S. Pat. Nos. 6,610,642; 6,605,458; 5,705,464; 5,710,115; 5,698,504; 5,695,679; 5,686,014; and 5,646,101). Embodiments of specific cleaning composition materials are exemplified in detail below. In embodiments in which the cleaning adjunct materials are not compatible with the disclosed a polypeptide of the present inventions in the cleaning compositions, then suitable methods of keeping the cleaning adjunct materials and the endo-β-mannanase(s) separated *(i.e.,* not in contact with each other) until combination of the two components is appropriate are used. Such separation methods include any suitable method known in the art (e.g., gelcaps, encapsulation, tablets, physical separation, etc.).

In some preferred embodiments, an effective amount of one or more polypeptide of the present invention(s) provided herein are included in compositions useful for cleaning a variety of surfaces in need of stain removal. Such cleaning compositions include cleaning compositions for such applications as cleaning hard surfaces, fabrics, and dishes. Indeed, in some embodiments, the present disclosure provides fabric cleaning compositions, while in other embodiments, the present disclosure provides non-fabric cleaning compositions. Notably, the present disclosure also provides cleaning compositions suitable for personal care, including oral care (including dentrifices, toothpastes, mouthwashes, etc., as well as denture cleaning compositions), skin, and hair cleaning compositions. Additionally, in still other embodiments, the present disclosure provides fabric softening compositions. It is intended that the present disclosure encompass detergent compositions in any form (*i.e.,* liquid, granular, bar, solid, semisolid, gel, paste, emulsion, tablet, capsule, unit dose, sheet, foam etc.).

By way of example, several cleaning compositions wherein the disclosed a polypeptide of the present inventions find use are described in greater detail below. In some embodiments in which the disclosed cleaning compositions are formulated as compositions suitable for use in laundry machine washing method(s), the compositions of the present disclosure contain at least one surfactant and at least one builder compound, as well as one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. In some embodiments, laundry compositions also contain softening agents (*i.e.,* as additional cleaning adjunct materials). The compositions of the present disclosure also find use detergent additive products in solid or liquid form. Such additive products are intended to supplement and/or boost the performance of conventional detergent compositions and can be added at any stage of the cleaning process. In some embodiments, the density of the laundry detergent compositions herein ranges from about 400 to about 1200 g/liter, while in other embodiments, it ranges from about 500 to about 950 g/liter of composition measured at 20°C.

In embodiments formulated as compositions for use in manual dishwashing methods, the compositions of the disclosure contain at least one surfactant and preferably at least one additional cleaning adjunct material selected from organic polymeric compounds, suds enhancing agents, group II metal ions, solvents, hydrotropes, and additional enzymes.

In some embodiments, various cleaning compositions such as those provided in U.S. Pat. No. 6,605,458 find use with a polypeptide or active fragment as described. Thus, in some embodiments, the composition is a compact granular fabric cleaning composition, while in other embodiments, the composition is a granular fabric cleaning composition useful in the laundering of colored fabrics, in further embodiments, the composition is a granular fabric cleaning composition which provides softening through the wash capacity, in additional embodiments, the composition is a heavy duty liquid fabric cleaning composition. In some embodiments, the compositions are fabric cleaning compositions such as those described in U.S. Pat. Nos. 6,610,642 and 6,376,450. In addition, a polypeptide as described may find use in granular laundry detergent compositions of particular utility under European or Japanese washing conditions *(See, e.g.,* U.S. Pat. No. 6,610,642).

In some alternative embodiments, the present disclosure provides hard surface cleaning compositions. Thus, in some embodiments, the composition is a hard surface cleaning composition such as those described in U.S. Pat. Nos. 6,610,642; 6,376,450; and 6,376,450.

In yet further embodiments, the present disclosure provides dishwashing compositions. Thus, in some embodiments, the composition is a hard surface cleaning composition such as those in U.S. Pat. Nos. 6,610,642 and 6,376,450. In some still further embodiments, the present disclosure provides dishwashing compositions. In some further embodiments, the compositions comprise oral care compositions such as those in U.S. Pat. Nos. 6,376,450 and 6,605,458. The formulations and descriptions of the compounds and cleaning adjunct materials contained in the aforementioned U.S. Pat. Nos. 6,376,450; 6,605,458; and 6,610,642 may be of use.

In still further embodiments, the compositions comprise fabric softening compositions such as those in GB-A1 400898, GB-A1 514 276, EP 0 011 340, EP 0 026 528, EP 0 242 919, EP 0 299 575, EP 0 313 146, and U.S. Pat. No. 5,019,292. The formulations and descriptions of the compounds and softening agents contained in the aforementioned GB-A1 400898, GB-A1 514 276, EP 0 011 340, EP 0 026 528, EP 0 242 919, EP 0 299 575, EP 0 313 146, and U.S. Pat. No. 5,019,292 may be of use.

The cleaning compositions of the present disclosure are formulated into any suitable form and prepared by any process chosen by the formulator, non-limiting examples of which are described in U.S. Pat. Nos. 5,879,584; 5,691,297; 5,574,005; 5,569,645; 5,565,422; 5,516,448; 5,489,392; and 5,486,303. When a low pH cleaning composition is desired, the pH of such composition is adjusted via the addition of a material such as monoethanolamine or an acidic material such as HCl.

In some embodiments, the cleaning compositions of the present invention are provided in unit dose form, including tablets, capsules, sachets, pouches, sheets, and multi-compartment pouches. In some embodiments, the unit dose format is designed to provide controlled release of the ingredients within a multi-compartment pouch (or other unit dose format). Suitable unit dose and controlled release formats are known in the art (*See e.g.,* EP 2 100 949, WO 02/102955, US Pat. Nos. 4,765,916 and 4,972,017, and WO 04/111178 for materials suitable for use in unit dose and controlled release formats). In some embodiments, the unit dose form is provided by tablets wrapped with a water-soluble film or water-soluble pouches. Various unit dose formats are provided in EP 2 100 947 and WO2013/165725, and are known in the art.

While not essential for the purposes of the present disclosure, the non-limiting list of adjuncts illustrated hereinafter are suitable for use in the instant cleaning compositions. In some embodiments, these adjuncts are incorporated for example, to assist or enhance cleaning performance, for treatment of the substrate to be cleaned, or to modify the aesthetics of the cleaning composition as is the case with perfumes, colorants, dyes or the like. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Pat. Nos. 5,576,282; 6,306,812; and 6,326,348. The aforementioned adjunct ingredients may constitute the balance of the cleaning compositions of the present disclosure.

In some embodiments, the cleaning compositions according to the present disclosure comprise at least one surfactant and/or a surfactant system wherein the surfactant is selected from nonionic surfactants, anionic surfactants, cationic surfactants, ampholytic surfactants, zwitterionic surfactants, semi-polar nonionic surfactants, and mixtures thereof. In some low pH cleaning composition embodiments (e.g., compositions having a neat pH of from about 3 to about 5), the composition typically does not contain alkyl ethoxylated sulfate, as it is believed that such surfactant may be hydrolyzed by such compositions' acidic contents. In some embodiments, the surfactant is present at a level of from about 0.1% to about 60%, while in alternative embodiments the level is from about 1% to about 50%, while in still further embodiments the level is from about 5% to about 40%, by weight of the cleaning composition.

In some embodiments, the cleaning compositions of the present disclosure contain at least one chelating agent. Suitable chelating agents may include, but are not limited to copper, iron, and/or manganese chelating agents, and mixtures thereof. In embodiments in which at least one chelating agent is used, the cleaning compositions of the present disclosure comprise from about 0.1% to about 15% or even from about 3.0% to about 10% chelating agent by weight of the subject cleaning composition.

In some still further embodiments, the cleaning compositions provided herein contain at least one deposition aid. Suitable deposition aids include, but are not limited to, polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present disclosure. In some preferred embodiments, non-ionic surfactants find use. For example, in automatic dishwashing embodiments, non-ionic surfactants find use for surface modification purposes, in particular for sheeting, to avoid filming and spotting and to improve shine. These non-ionic surfactants also find use in preventing the re-deposition of soils. In some preferred embodiments, the anti-redeposition agent is a non-ionic surfactant as known in the art *(See, e.g.,* EP 2 100 949).

In some embodiments, the cleaning compositions of the present disclosure include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones, and polyvinylimidazoles, or mixtures thereof. In embodiments in which at least one dye transfer inhibiting agent is used, the cleaning compositions of the present disclosure comprise from about 0.0001% to about 10%, from about 0.01% to about 5%, or even from about 0.1% to about 3% by weight of the cleaning composition.

In some embodiments, silicates are included within the compositions of the present disclosure. In some such embodiments, sodium silicates *(e.g.,* sodium disilicate, sodium metasilicate, and crystalline phyllosilicates) find use. In some embodiments, silicates are present at a level of from about 1% to about 20%. In some preferred embodiments, silicates are present at a level of from about 5% to about 15% by weight of the composition.

In some still additional embodiments, the cleaning compositions of the present disclosure also contain dispersants. Suitable water-soluble organic materials include, but are not limited to the homo- or co-polymeric acids or their salts, in which the polycarboxylic acid comprises at least two carboxyl radicals separated from each other by not more than two carbon atoms.

In some further embodiments, the enzymes used in the cleaning compositions are stabilized by any suitable technique. In some embodiments, the enzymes employed herein are stabilized by the presence of water-soluble sources of calcium and/or magnesium ions in the finished compositions that provide such ions to the enzymes. In some embodiments, the enzyme stabilizers include oligosaccharides, polysaccharides, and inorganic divalent metal salts, including alkaline earth metals, such as calcium salts. It is contemplated that various techniques for enzyme stabilization will find use in the present disclosure. For example, in some embodiments, the enzymes employed herein are stabilized by the presence of water-soluble sources of zinc (II), calcium (II), and/or magnesium (II) ions in the finished compositions that provide such ions to the enzymes, as well as other metal ions (*e.g*., barium (II), scandium (II), iron (II), manganese (II), aluminum (III), tin (II), cobalt (II), copper (II), nickel (II), and oxovanadium (IV). Chlorides and sulfates also find use in some embodiments of the present disclosure. Examples of suitable oligosaccharides and polysaccharides *(e.g.,* dextrins) are known in the art *(See, e.g.,* WO 07/145964). In some embodiments, reversible protease inhibitors also find use, such as boron-containing compounds *(e.g.,* borate, 4-formyl phenyl boronic acid) and/or a tripeptide aldehyde find use to further improve stability, as desired.

In some embodiments, bleaches, bleach activators, and/or bleach catalysts are present in the compositions of the present disclosure. In some embodiments, the cleaning compositions of the present disclosure comprise inorganic and/or organic bleaching compound(s). Inorganic bleaches may include, but are not limited to perhydrate salts *(e.g.,* perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the present disclosure *(See, e.g.,* EP 2 100 949).

In some embodiments, bleach activators are used in the compositions of the present disclosure. Bleach activators are typically organic peracid precursors that enhance the bleaching action in the course of cleaning at temperatures of 60°C and below. Bleach activators suitable for use herein include compounds which, under perhydrolysis conditions, give aliphatic peroxycarboxylic acids having preferably from about 1 to about 10 carbon atoms, in particular from about 2 to about 4 carbon atoms, and/or optionally substituted perbenzoic acid. Additional bleach activators are known in the art and find use in the present disclosure *(See, e.g.,* EP 2 100 949).

In addition, in some embodiments and as further described herein, the cleaning compositions of the present disclosure further comprise at least one bleach catalyst. In some embodiments, the manganese triazacyclononane and related complexes find use, as well as cobalt, copper, manganese, and iron complexes. Additional bleach catalysts find use in the present disclosure *(See, e.g.,* U.S. Pat. No. 4,246,612; U.S. Pat. No. 5,227,084; U.S. Pat. No. 4,810,410; WO 99/06521; and EP 2 100 949).

In some embodiments, the cleaning compositions of the present disclosure contain one or more catalytic metal complexes. In some embodiments, a metal-containing bleach catalyst finds use. In some preferred embodiments, the metal bleach catalyst comprises a catalyst system comprising a transition metal cation of defined bleach catalytic activity, *(e.g.,* copper, iron, titanium, ruthenium, tungsten, molybdenum, or manganese cations), an auxiliary metal cation having little or no bleach catalytic activity *(e.g.,* zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used *(See, e.g.,* U.S. Pat. No. 4,430,243). In some embodiments, the cleaning compositions of the present disclosure are catalyzed by means of a manganese compound. Such compounds and levels of use are well known in the art (*See, e.g.,* U.S. Pat. No. 5,576,282). In additional embodiments, cobalt bleach catalysts find use in the cleaning compositions of the present disclosure. Various cobalt bleach catalysts are known in the art *(See, e.g.,* U.S. Pat. Nos. 5,597,936 and 5,595,967) and are readily prepared by known procedures.

In some additional embodiments, the cleaning compositions of the present disclosure include a transition metal complex of a macropolycyclic rigid ligand (MRL). As a practical matter, and not by way of limitation, in some embodiments, the compositions and cleaning processes provided by the present disclosure are adjusted to provide on the order of at least one part per hundred million of the active MRL species in the aqueous washing medium, and in some preferred embodiments, provide from about 0.005 ppm to about 25 ppm, more preferably from about 0.05 ppm to about 10 ppm, and most preferably from about 0.1 ppm to about 5 ppm, of the MRL in the wash liquor.

In some embodiments, preferred transition-metals in the instant transition-metal bleach catalyst include, but are not limited to manganese, iron, and chromium. Preferred MRLs also include, but are not limited to special ultra-rigid ligands that are cross-bridged *(e.g.,* 5,12-diethyl-1,5,8,12-tetraazabicyclo[6.6.2] hexadecane). Suitable transition metal MRLs are readily prepared by known procedures *(See, e.g.,* WO 2000/32601 and U.S. Pat. No. 6,225,464).

In some embodiments, the cleaning compositions of the present disclosure comprise metal care agents. Metal care agents find use in preventing and/or reducing the tarnishing, corrosion, and/or oxidation of metals, including aluminum, stainless steel, and non-ferrous metals *(e.g.,* silver and copper). Suitable metal care agents include those described in EP 2 100 949, WO 94/26860, and WO 94/26859). In some embodiments, the metal care agent is a zinc salt. In some further embodiments, the cleaning compositions of the present disclosure comprise from about 0.1% to about 5% by weight of one or more metal care agent.

As indicated above, the cleaning compositions of the present disclosure are formulated into any suitable form and prepared by any process chosen by the formulator, non-limiting examples of which are described in U.S. Pat. Nos. 5,879,584; 5,691,297; 5,574,005; 5,569,645; 5,516,448; 5,489,392; and 5,486,303. In some embodiments in which a low pH cleaning composition is desired, the pH of such composition is adjusted via the addition of an acidic material such as HCl.

The cleaning compositions disclosed herein of find use in cleaning a situs *(e.g.,* a surface, dishware, or fabric). Typically, at least a portion of the situs is contacted with an embodiment of the present cleaning composition, in neat form or diluted in wash liquor, and then the situs is optionally washed and/or rinsed. For purposes of the present disclosure, "washing" includes but is not limited to, scrubbing and mechanical agitation. In some embodiments, the cleaning compositions are typically employed at concentrations of from about 500 ppm to about 15,000 ppm in solution. When the wash solvent is water, the water temperature typically ranges from about 5°C to about 90°C and, when the situs comprises a fabric, the water to fabric mass ratio is typically from about 1:1 to about 30:1.

Other aspects and embodiments of the present compositions and methods will be apparent from the foregoing description and following examples.

### EXAMPLES

Certain of the following examples illustrate the invention. Others provide useful context.

### EXAMPLE 1

### Identification of Bacillus and Paenibacillus mannanases

The following nucelotide and amino acid sequences for mannanases encoded by *Bacillus* and *Paenibacillus* species were extracted from the NCBI Database.

The nucleotide sequence of the *BciMan1* gene (NCBI Reference Sequence AB007123.1) isolated from *B. circulans K-1* is set forth as SEQ ID NO:1 (the sequence encoding the predicted native signal peptide is shown in bold):

ATGGGGTGGTTTTTAGTGATTTTACGCAAGTGGTTGATTGCTTTTGTCGCATTT
TTACTGATGTTCTCGTGGACTGGACAACTTACGAACAAAGCACATGCTGCAAGC
GGATTTTATGTAAGCGGTACCAAATTATTGGATGCTACAGGACAACCATTTGTGATG
CGAGGAGTCAATCATGCGCACACATGGTATAAAGATCAACTATCCACCGCAATACC
AGCCATTGCTAAAACAGGTGCCAACACGATACGTATTGTACTGGCGAATGGACACA
AATGGACGCTTGATGATGTAAACACCGTCAACAATATTCTCACCCTCTGTGAACAA
AACAAACTAATTGCCGTTTTGGAAGTACATGACGCTACAGGAAGCGATAGTCTTTC
CGATTTAGACAACGCCGTTAATTACTGGATTGGTATTAAAAGCGCGTTGATCGGCA
AGGAAGACCGTGTAATCATTAATATAGCTAACGAGTGGTACGGAACATGGGATGGA
GTCGCCTGGGCTAATGGTTATAAGCAAGCCATACCCAAACTGCGTAATGCTGGTCT
AACTCATACGCTGATTGTTGACTCCGCTGGATGGGGACAATATCCAGATTCGGTCAA
AAATTATGGGACAGAAGTACTGAATGCAGACCCGTTAAAAAACACAGTATTCTCTA
TCCATATGTATGAATATGCTGGGGGCAATGCAAGTACCGTCAAATCCAATATTGAC
GGTGTGCTGAACAAGAATCTTGCACTGATTATCGGCGAATTTGGTGGACAACATAC
AAACGGTGATGTGGATGAAGCCACCATTATGAGTTATTCCCAAGAGAAGGGAGTCG
GCTGGTTGGCTTGGTCCTGGAAGGGAAATAGCAGTGATTTGGCTTATCTCGATATGA
CAAATGATTGGGCTGGTAACTCCCTCACCTCGTTCGGTAATACCGTAGTGAATGGCA

GTAACGGCATTAAAGCAACTTCTGTGTTATCCGGCATTTTTGGAGGTGTTACGCCAA
CCTCAAGCCCTACTTCTACACCTACATCTACGCCAACCTCAACTCCTACTCCTACGC
CAAGTCCGACCCCGAGTCCAGGTAATAACGGGACGATCTTATATGATTTCGAAACA
GGAACTCAAGGCTGGTCGGGAAACAATATTTCGGGAGGCCCATGGGTCACCAATGA
ATGGAAAGCAACGGGAGCGCAAACTCTCAAAGCCGATGTCTCCTTACAATCCAATT
CCACGCATAGTCTATATATAACCTCTAATCAAAATCTGTCTGGAAAAGCAGTCTGA
AAGCAACGGTTAAGCATGCGAACTGGGGCAATATCGGCAACGGGATTTATGCAAAA
CTATACGTAAAGACCGGGTCCGGGTGGACATGGTACGATTCCGGAGAATCTGAT
TCAGTCAAACGACGGTACCATTTTGACACTATCCCTCAGCGGCATTTCGAATTTGTC
CTCAGTCAAAGAAATTGGGGTAGAATTCCGCGCCTCCTCAAACAGTAGTGGCCAAT
CAGCTATTTATGTAGATAGTGTTAGTCTGCAATGA.

The amino acid sequence of the precursor protein encoded by the *BciMan1* gene, BciMan1 (NCBI Accession No. BAA25878.1) is set forth as SEQ ID NO:2 (the predicted native signal peptide is shown in bold):

MGWFLVILRKWLIAFVAFLLMFSWTGQLTNKAHAASGFYVSGTKLLDATGQPFVM
RGVNHAHTWYKDQLSTAIPAIAKTGANTIRIVLANGHKWTLDDVNTVNNILTLCEQNK
LIAVLEVHDATGSDSLSDLDNAVNYWIGIKSALIGKEDRVIINIANEWYGTWDGVAWA
NGYKQAIPKLRNAGLTHTLIVDSAGWGQYPDSVKNYGTEVLNADPLKNTVFSIHMYEY
AGGNASTVKSNIDGVLNKNLALIIGEFGGQHTNGDVDEATIMSYSQEKGVGWLAWSW
KGNSSDLAYLDMTNDWAGNSLTSFGNTVVNGSNGIKATSVLSGIFGGVTPTSSPTSTPT
STPTSTPTPTPSPTPSPGNNGTILYDFETGTQGWSGNNISGGPWVTNEWKATGAQTLKA
DVSLQSNSTHSLYITSNQNLSGKSSLKATVKHANWGNIGNGIYAKLYVKTGSGWTWYD
SGENLIQSNDGTILTLSLSGISNLSSVKEIGVEFRASSNSSGQSAIYVDSVSLQ.

The nucleic acid sequence for the *BciMan3* gene (NCBI Reference Sequence AY907668.1, from 430 to 1413, complement) isolated from *B. circulans 196* is set forth as SEQ ID NO:3 (the sequence encoding the predicted native signal peptide is shown in bold):

ATGATGTTGATATGGATGCAGGGATGGAAGTCTATTCTAGTCGCGATCTTGGC
GTGTGTGTCAGTAGGCGGTGGGCTTCCTAGTCCAGAAGCAGCCACAGGATTTA
TGTAAACGGTACCAAGCTGTATGATTCAACGGGCAAGGCCTTTGTGATGAGGGGTG
TAAATCATCCCCACACCTGGTACAAGAATGATCTGAACGCGGCTATTCCGGCTATCG
CGCAAACGGGAGCCAATACCGTACGAGTCGTCTTGTCGAACGGGTCGCAATGGACC
AAGGATGACCTGAACTCCGTCAACAGTATCATCTCGCTGGTGTCGCAGCATCAAAT
GATAGCCGTTCTGGAGGTGCATGATGCGACAGGCAAAGATGAGTATGCTTCCCTTG
AAGCGGCCGTCGACTATTGGATCAGCATCAAAGGGGCATTGATCGGAAAAGAAGA

CCGCGTCATCGTCAATATTGCTAATGAATGGTATGGAAATTGGAACAGCAGCGGAT
GGGCCGATGGTTATAAGCAGGCCATTCCCAAATTAAGAAACGCGGGCATTAAGAAT
ACGTTGATCGTTGATGCAGCGGGATGGGGGCAATACCCGCAATCCATCGTGGATGA
GGGGGCCGCGGTATTTGCTTCCGATCAACTGAAGAATACGGTATTCTCCATCCATAT
GTATGAGTATGCCGGTAAGGATGCCGCTACGGTGAAAACGAATATGGACGATGTTT
TAAACAAAGGATTGCCTTTAATCATTGGGGAGTTCGGCGGCTATCATCAAGGTGCC
GATGTCGATGAGATTGCTATTATGAAGTACGGACAGCAGAAGGAAGTGGGCTGGCT
GGCTTGGTCCTGGTACGGAAACAGCCCGGAGCTGAACGATTTGGATCTGGCTGCAG
GGCCAAGCGGAAACCTGACCGGCTGGGGAAACACGGTGGTTCATGGAACCGACGG
GATTCAGCAAACCTCCAAGAAAGCGGGCATTTATTAA.

The amino acid sequence of the precursor protein encoded by the *BciMan3* gene, BciMan3 (NCBI Accession No. AAX87002.1) is set forth as SEQ ID NO:4 (the predicted native signal peptide is shown in bold):

MMLIWMQGWKSILVAILACVSVGGGLPSPEAATGFYVNGTKLYDSTGKAFVMRGV
NHPHTWYKNDLNAAIPAIAQTGANTVRVVLSNGSQWTKDDLNSVNSIISLVSQHQMIA
VLEVHDATGKDEYASLEAAVDYWISIKGALIGKEDRVIVNIANEWYGNWNSSGWADG
YKQAIPKLRNAGIKNTLIVDAAGWGQYPQSIVDEGAAVFASDQLKNTVFSIHMYEYAG
KDAATVKTNMDDVLNKGLPLIIGEFGGYHQGADVDEIAIMKYGQQKEVGWLAWSWY
GNSPELNDLDLAAGPSGNLTGWGNTVVHGTDGIQQTSKKAGIY.

The nucleic acid sequence for the *BciMan4* gene (NCBI Reference Sequence AY913796.1, from 785 to 1765) isolated from *Bacillus circulans CGMCC1554* is set forth as SEQ ID NO:5 (the sequence encoding the predicted native signal peptide is shown in bold):

ATGGCCAAGTTGCAAAAGGGTACAATCTTAACAGTCATTGCAGCACTGATGTT
TGTCATTTTGGGGAGCGCGGCGCCCAAAGCCGCAGCAGCTACAGGTTTTACGT
GAATGGAGGCAAATTGTACGATTCTACGGGTAAACCATTTTACATGAGGGGTATCA
ATCATGGGCACTCCTGGTTTAAAAATGATTTGAACACGGCTATCCCTGCGATCGCAA
AAACGGGTGCCAATACGGTACGAATTGTTTTATCAAACGGTACACAATACACCAAG
GATGATCTGAATTCCGTAAAAAACATCATTAATGTCGTAAATGCAAACAAGATGAT
TGCTGTGCTTGAAGTACACGATGCCACTGGGAAAGATGACTTCAACTCGTTGGATG
CAGCGGTCAACTACTGGATAAGCATCAAAGAAGCACTGATCGGGAAGGAAGATCG
GGTTATTGTAAACATTGCAAACGAGTGGTACGGAACATGGAACGGAAGCGCGTGGG
CTGACGGGTACAAAAAAGCTATTCCGAAATTAAGAGATGCGGGTATTAAAAATACC
TTGATTGTAGATGCAGCAGGCTGGGGTCAGTACCCTCAATCGATCGTCGATTACGG
ACAAAGCGTATTCGCCGCGGATTCACAGAAAAATACGGCGTTTTCCATTCACATGT

ATGAGTATGCAGGCAAGGATGCGGCCACCGTCAAATCCAATATGGAAAATGTGCTG
AATAAGGGGCTGGCCTTAATCATTGGTGAGTTCGGAGGATATCACACCAATGGAGA
TGTCGATGAATATGCAATCATGAAATATGGTCTGGAAAAAGGGGTAGGATGGCTTG
CATGGTCTTGGTACGGTAATAGCTCTGGATTAAACTATCTTGATTTGGCAACAGGAC
CTAACGGCAGTTTGACGAGCTATGGTAATACGGTTGTCAATGATACTTACGGAATTA
AAAATACGTCCCAAAAAGCGGGAATCTTTTAA.

The amino acid sequence of the precursor protein encoded by the *BciMan4* gene, BciMan4 (NCBI Accession No. AAX87003.1) is set forth as SEQ ID NO:6 (the predicted native signal peptide is shown in bold):

MAKLQKGTILTVIAALMFVILGSAAPKAAAATGFYVNGGKLYDSTGKPFYMRGINH
GHSWFKNDLNTAIPAIAKTGANTVRIVLSNGTQYTKDDLNSVKNIINVVNANKMIAVLE
VHDATGKDDFNSLDAAVNYWISIKEALIGKEDRVIVNIANEWYGTWNGSAWADGYKK
AIPKLRDAGIKNTLIVDAAGWGQYPQSIVDYGQSVFAADSQKNTAFSIHMYEYAGKDA
ATVKSNMENVLNKGLALIIGEFGGYHTNGDVDEYAIMKYGLEKGVGWLAWSWYGNS
SGLNYLDLATGPNGSLTSYGNTVVNDTYGIKNTSQKAGIF.

The nucleic acid sequence for the *PpoMan1* gene (NCBI Reference Sequence NC_014483.1, from 649134 to 650117, complement) isolated from *Paenibacillus polymyxa E681* is set forth as SEQ ID NO:7 (the sequence encoding the predicted native signal peptide is shown in bold):

ATGAAGGTATTGTTAAGAAAAGCATTATTGTCTGGACTGGTCGGCTTGCTCATC
ATGATTGGTTTAGGAGGAGTTTTCTCCAAGGTAGAAGCTGCTTCAGGATTTATG
TAAGCGGTACCAAATTGTATGACTCTACAGGCAAGCCATTTGTTATGAGAGGCGTC
AATCATGCTCACACTTGGTACAAAAACGATCTTTATACAGCTATCCCGGCAATTGCC
CAGACAGGTGCTAATACCGTCCGAATTGTCCTTTCTAACGGAAACCAGTACACCAA
GGATGACATTAATTCCGTGAAAAATATTATCTCTCTTGTCTCCAACTATAAAATGAT
TGCTGTACTTGAAGTTCATGATGCTACAGGCAAAGACGACTACGCGTCTTTGGATGC
AGCTGTGAACTACTGGATTAGCATAAAAGATGCTCTGATCGGCAAGGAAGACCGGG
TTATCGTAAACATTGCGAACGAATGGTATGGTTCTTGGAATGGAAGTGGTTGGGCT
GATGGATACAAGCAAGCGATTCCCAAGTTGAGAAACGCAGGTATCAAAAATACGCT
CATCGTCGATTGTGCCGGATGGGGACAGTATCCTCAGTCTATCAATGACTTTGGTAA
ATCTGTATTTGCAGCTGATTCTTTGAAGAATACGGTATTCTCTATTCATATGTATGAG
TTCGCTGGTAAAGATGCTCAAACCGTTCGAACCAATATTGATAACGTTCTGAATCAA
GGAATTCCTCTGATTATTGGTGAATTTGGAGGTTACCACCAGGGAGCAGACGTCGA
CGAGACAGAAATCATGAGATATGGCCAATCCAAAGGAGTAGGCTGGTTAGCCTGGT

CCTGGTATGGTAATAGTTCCAACCTTTCCTACCTTGATCTTGTAACAGGACCTAATG
GCAATCTGACGGATTGGGGAAAAACTGTAGTTAACGGAAGCAACGGGATCAAAGA
AACATCGAAAAAAGCTGGTATCTACTAA.

The amino acid sequence of the protein encoded by the *PpoMan1* gene, PpoMan1 (NCBI Accession No. YP_003868989.1) is set forth as SEQ ID NO:8 (the predicted native signal peptide is shown in bold):

MKVLLRKALLSGLVGLLIMIGLGGVFSKVEAASGFYVSGTKLYDSTGKPFVMRGVN
HAHTWYKNDLYTAIPAIAQTGANTVRIVLSNGNQYTKDDINSVKNIISLVSNYKMIAVL
EVHDATGKDDYASLDAAVNYWISIKDALIGKEDRVIVNIANEWYGSWNGSGWADGYK
QAIPKLRNAGIKNTLIVDCAGWGQYPQSINDFGKSVFAADSLKNTVFSIHMYEFAGKDA
QTVRTNIDNVLNQGIPLIIGEFGGYHQGADVDETEIMRYGQSKGVGWLAWSWYGNSSN
LSYLDLVTGPNGNLTDWGKTVVNGSNGIKETSKKAGIY.

The nucleic acid sequence for the *PpoMan2* gene (NCBI Reference Sequence NC_014622.1, from 746871 to747854, complement) isolated from *Paenibacillus polymyxa SC2* is set forth as SEQ ID NO:9 (the sequence encoding the predicted native signal peptide is shown in bold):

GTGAACGCATTGTTAAGAAAAGCATTATTGTCTGGACTCGCTGGTCTGCTTATC
ATGATTGGTTTGGGGGGATTCTTCTCCAAGGCGCAAGCTGCTTCAGGATTTAT
GTAAGCGGTACCAATCTGTATGACTCTACAGGCAAACCGTTCGTTATGAGAGGCGT
CAATCATGCTCACACTTGGTACAAAAACGATCTTTATACTGCTATCCCAGCAATTGC
TAAAACAGGTGCTAATACAGTCCGAATTGTCCTTTCTAACGGAAACCAGTACACCA
AGGATGACATTAATTCCGTGAAAAATATTATCTCTCTCGTCTCCAACCATAAAATGA
TTGCTGTACTTGAAGTTCATGACGCTACAGGTAAAGACGACTATGCGTCTTTGGATG
CAGCAGTGAATTACTGGATTAGTATAAAAGATGCTCTGATCGGCAAGGAAGATCGG
GTTATCGTGAACATTGCGAACGAATGGTATGGCTCTTGGAATGGAGGCGGTTGGGC
AGATGGGTATAAGCAAGCGATTCCCAAGCTGAGAAACGCAGGCATCAAAAATACG
CTCATCGTCGATTGTGCTGGATGGGGACAATACCCTCAGTCTATCAATGACTTTGGT
AAATCTGTGTTTGCAGCTGATTCTTTGAAAAATACCGTTTTCTCCATTCATATGTATG
AATTTGCTGGCAAAGATGTTCAAACGGTTCGAACCAATATTGATAACGTTCTGTATC
AAGGGCTCCCTTTGATTATTGGTGAATTTGGCGGTTACCATCAGGGAGCAGACGTCG
ACGAGACAGAAATCATGAGATACGGCCAATCTAAAAGCGTAGGCTGGTTAGCCTGG
TCCTGGTATGGCAATAGCTCCAACCTTAATTATCTTGATCTTGTGACAGGACCTAAC
GGCAATCTGACCGATTGGGGTCGCACCGTGGTAGAGGGAGCCAACGGGATCAAAG
AAACATCGAAAAAAGCGGGTATCTTCTAA.

The amino acid sequence of the hypothetical protein encoded by the *PpoMan2* gene, PpoMan2 (NCBI Accession No. YP_003944884.1) is set forth as SEQ ID NO:10 (the predicted native signal peptide is shown in bold):

MNALLRKALLSGLAGLLIMIGLGGFFSKAQAASGFYVSGTNLYDSTGKPFVMRGVN
HAHTWYKNDLYTAIPAIAKTGANTVRIVLSNGNQYTKDDINSVKNIISLVSNHKMIAVL
EVHDATGKDDYASLDAAVNYWISIKDALIGKEDRVIVNIANEWYGSWNGGGWADGY
KQAIPKLRNAGIKNTLIVDCAGWGQYPQSINDFGKSVFAADSLKNTVFSIHMYEFAGKD
VQTVRTNIDNVLYQGLPLIIGEFGGYHQGADVDETEIMRYGQSKSVGWLAWSWYGNSS
NLNYLDLVTGPNGNLTDWGRTVVEGANGIKETSKKAGIF.

The nucleic acid sequence for the *PspMan4* gene (NCBI Reference Sequence GQ358926.1) isolated from *Paenibacillus sp.* A1 is set forth as SEQ ID NO:11 (the sequence encoding the predicted native signal peptide is shown in bold):

ATGAAATACCTGCTGCCGACCGCTGCTGCTGGTCTGCTGCTCCTCGCTGCCCA
GCCGGCGATGGCCATGGCTACAGGTTTTATGTAAGCGGTAACAAGTTATACGATT
CCACTGGCAAGCCTTTTGTTATGAGAGGTGTTAATCACGGACATTCCTGGTTCAAAA
ATGATTTGAATACCGCTATCCCTGCCATCGCCAAAACAGGTGCCAATACGGTACGC
ATTGTTCTTTCGAATGGTAGCCTGTACACCAAAGATGATCTGAACGCTGTTAAAAAT
ATTATTAATGTGGTTAACCAGAATAAAATGATAGCTGTACTCGAAGTACATGACGC
CACAGGGAAAGATGACTATAATTCGTTGGATGCGGCGGTGAACTACTGGATTAGTA
TTAAGGAAGCTTTGATTGGAAAAGAAGATCGGGTAATTGTCAACATCGCCAATGAA
TGGTATGGAACGTGGAATGGAAGTGCGTGGGCTGATGGTTACAAAAAAGCCATTCC
GAAACTCCGAAATGCAGGAATTAAAAATACGCTAATTGTGGATGCAGCCGGATGGG
GACAGTTCCCTCAATCCATCGTGGATTATGGACAAAGTGTATTTGCAGCCGATTCAC
AGAAAAATACCGTCTTCTCCATTCATATGTATGAGTATGCTGGCAAAGATGCTGCAA
CGGTCAAAGCCAATATGGAGAATGTGCTGAACAAAGGATTGGCTCTGATCATTGGT
GAATTCGGGGGATATCACACAAACGGTGATGTGGATGAGTATGCCATCATGAGATA
TGGTCAGGAAAAAGGGGTAGGCTGGCTTGCCTGGTCTTGGTACGGAAACAGCTCCG
GTTTGAACTATCTGGACATGGCCACAGGTCCGAACGGAAGCTTAACGAGTTTTGGC
AACACTGTTGTTAATGATACCTATGGTATTAAAAACACTTCCCAAAAAGCGGGGAT
TTTCTAA.

The amino acid sequence of the protein encoded by the *PspMan4* gene, PspMan4 (NCBI Accession No. ACU30843.1) is set forth as SEQ ID NO:12 (the predicted native signal peptide is shown in bold):

MKYLLPTAAAGLLLLAAQPAMAMATGFYVSGNKLYDSTGKPFVMRGVNHGHSWFK
NDLNTAIPAIAKTGANTVRIVLSNGSLYTKDDLNAVKNIINVVNQNKMIAVLEVHDATG
KDDYNSLDAAVNYWISIKEALIGKEDRVIVNIANEWYGTWNGSAWADGYKKAIPKLR
NAGIKNTLIVDAAGWGQFPQSIVDYGQSVFAADSQKNTVFSIHMYEYAGKDAATVKA
NMENVLNKGLALIIGEFGGYHTNGDVDEYAIMRYGQEKGVGWLAWSWYGNSSGLNY
LDMATGPNGSLTSFGNTVVNDTYGIKNTSQKAGIF.

The nucleic acid sequence for the *PspMan5* gene (NCBI Reference Sequence JN603735.1, from 536 to 1519) isolated from *Paenibacillus sp.* CH-3 is set forth as SEQ ID NO:13 (the sequence encoding the predicted native signal peptide is shown in bold):

ATGAGACAACTTTTAGCAAAAGGTATTTTAGCTGCACTGGTCATGATGTTAGCG
ATGTATGGATTGGGGAATCTCTCTTCTAAAGCTTCGGCTGCAACAGGTTTTATG
TAAGCGGTACCACTCTATATGATTCTACTGGTAAACCTTTTGTAATGCGGTGTCA
ATCATTCGCATACCTGGTTCAAAAATGATCTAAATGCAGCCATCCCTGCTATTGCCA
AAACAGGTGCAAATACAGTACGTATCGTTTTATCTAATGGTGTTCAGTATACTAGAG
ATGATGTAAACTCAGTCAAAAATATTATTTCCCTGGTTAACCAAAACAAAATGATTG
CTGTTCTTGAGGTGCATGATGCTACCGGTAAAGACGATTACGCTTCTCTTGATGCCG
CTGTAAACTACTGGATCAGCATCAAAGATGCCTTGATTGGCAAGGAAGATCGAGTC
ATTGTTAATATTGCCAATGAATGGTACGGTACATGGAATGGCAGTGCTTGGGCAGA
TGGTTATAAGCAGGCTATTCCCAAACTAAGAAATGCAGGCATCAAAAACACTTTAA
TCGTTGATGCCGCCGGCTGGGGACAATGTCCTCAATCGATCGTTGATTACGGGCAA
AGTGTATTTGCAGCAGATTCGCTTAAAAATACAATTTTCTCTATTCACATGTATGAA
TATGCAGGCGGTACAGATGCGATCGTCAAAAGCAATATGGAAAATGTACTGAACAA
AGGACTTCCTTTGATCATCGGTGAATTTGGCGGGCAGCATACAAACGGCGATGTAG
ATGAACATGCAATTATGCGTTATGGTCAGCAAAAAGGTGTAGGTTGGCTGGCATGG
TCGTGGTATGGCAACAATAGTGAACTCAGTTATCTGGATTTGGCTACAGGTCCCGCC
GGTAGTCTGACAAGTATCGGCAATACGATTGTAAATGATCCATATGGTATCAAAGC
TACCTCGAAAAAAGCGGGTATCTTCTAA.

The amino acid sequence of the protein encoded by the *PspMan5* gene, PspMan5 (NCBI Accession No. AEX60762.1) is set forth as SEQ ID NO:14 (the predicted native signal peptide is shown in bold):

**MRQLLAKGILAALVMMLAMYGLGNLSSKASA**ATGFYVSGTTLYDSTGKPFVMRGV
NHSHTWFKNDLNAAIPAIAKTGANTVRIVLSNGVQYTRDDVNSVKNIISLVNQNKMIAV
LEVHDATGKDDYASLDAAVNYWISIKDALIGKEDRVIVNIANEWYGTWNGSAWADGY
KQAIPKLRNAGIKNTLIVDAAGWGQCPQSIVDYGQSVFAADSLKNTIFSIHMYEYAGGT

DAIVKSNMENVLNKGLPLIIGEFGGQHTNGDVDEHAIMRYGQQKGVGWLAWSWYGN
NSELSYLDLATGPAGSLTSIGNTIVNDPYGIKATSKKAGIF.

In addition, mannanases were identified by sequencing the genomes of *Paenibacillus amylolyticus DSM11730, DSM15211, and DSM11747, Paenibacillus pabuli DSM3036, Paenibacillus sp. FeL05* (renamed as *Paenibacillus hunanensis DSM22170),* and *Paenibacillus tundrae* (Culture Collection DuPont). The entire genomes of these organisms were sequenced by BaseClear (Leiden, The Netherlands) using the Illumina's next generation sequencing technology and subsequently assembled by BaseClear. Contigs were annotated by BioXpr (Namur, Belgium).

The nucleotide sequence of the *PamMan2* gene isolated from *Paenibacillus amylolyticus* is set forth as SEQ ID NO:15 (the identical sequence was found in *DSM11730, DSM15211, and DSM11747;* the sequence encoding the predicted native signal peptide is shown in bold):

ATGGTTAATCTGAAAAAGTGTACAATCTTCACGGTTATTGCTACACTCATGTTC
ATGGTATTAGGGAGTGCAGCACCCAAAGCATCTGCTGCTACAGGATTTTATGTA
AGCGGTAACAAGTTATACGATTCCACAGGCAAGGCTTTTGTCATGAGAGGTGTTAA
TCACGGACATTCCTGGTTCAAAAATGATTTGAATACCGCTATCCCTGCAATCGCCAA
AACAGGTGCCAATACGGTACGCATTGTTCTTTCGAATGGTAGCCTGTACACCAAAG
ATGATCTGAACGCTGTTAAAAATATTATTAATGTGGTTAACCAAAATAAAATGATA
GCTGTACTCGAGGTGCATGACGCCACAGGGAAAGATGACTATAATTCGTTGGATGC
GGCAGTGAACTACTGGATTAGCATTAAGGAAGCTTTGATTGGCAAAGAAGATCGGG
TCATCGTCAATATCGCCAATGAATGGTATGGAACGTGGAATGGAAGTGCGTGGGCT
GATGGTTACAAAAAAGCCATTCCGAAACTCCGAAATGCGGGAATTAAAAATACGCT
AATTGTGGATGCAGCCGGATGGGGACAGTTCCCTCAATCCATCGTGGATTATGGAC
AAAGTGTATTTGCAACCGATTCTCAGAAAAATACGGTCTTCTCCATTCATATGTATG
AGTATGCTGGCAAAGATGCTGCAACCGTCAAAGCCAATATGGAAAATGTGCTGAAC
AAAGGATTGGCTCTGATCATTGGTGAGTTCGGGGGATACCACACAAACGGTGATGT
GGACGAGTATGCCATCATGAGATATGGTCAGGAAAAAGGGGTGGGCTGGCTGGCCT
GGTCCTGGTATGGAAACAGTTCTGGTCTGAACTACCTGGACATGGCTACAGGTCCG
AACGGAAGTTTGACGAGCTTCGGAAACACCGTAGTGAATGATACCTATGGAATTAA
AAAAACTTCTCAAAAAGCGGGGATTTTC.

The amino acid sequence of the PamMan2 precursor protein is set forth as SEQ ID NO:16 (the predicted native signal peptide is shown in bold):

MVNLKKCTIFTVIATLMFMVLGSAAPKASAATGFYVSGNKLYDSTGKAFVMRGVNH
GHSWFKNDLNTAIPAIAKTGANTVRIVLSNGSLYTKDDLNAVKNIINVVNQNKMIAVLE
VHDATGKDDYNSLDAAVNYWISIKEALIGKEDRVIVNIANEWYGTWNGSAWADGYKK
AIPKLRNAGIKNTLIVDAAGWGQFPQSIVDYGQSVFATDSQKNTVFSIHMYEYAGKDA
ATVKANMENVLNKGLALIIGEFGGYHTNGDVDEYAIMRYGQEKGVGWLAWSWYGNS
SGLNYLDMATGPNGSLTSFGNTVVNDTYGIKKTSQKAGIF.

The sequence of the fully processed mature PamMan2 protein (297 amino acids) is set forth as SEQ ID NO:17:

ATGFYVSGNKLYDSTGKAFVMRGVNHGHSWFKNDLNTAIPAIAKTGANTVRIVLSNGS
LYTKDDLNAVKNIINVVNQNKMIAVLEVHDATGKDDYNSLDAAVNYWISIKEALIGKE
DRVIVNIANEWYGTWNGSAWADGYKKAIPKLRNAGIKNTLIVDAAGWGQFPQSIVDY
GQSVFATDSQKNTVFSIHMYEYAGKDAATVKANMENVLNKGLALIIGEFGGYHTNGD
VDEYAIMRYGQEKGVGWLAWSWYGNSSGLNYLDMATGPNGSLTSFGNTVVNDTYGI
KKTSQKAGIF.

The nucleotide sequence of the *PpaMan2* gene isolated from *Paenibacillus pabuli* DSM3036 is set forth as SEQ ID NO:18 (the sequence encoding the predicted native signal peptide is shown in bold):

ATGGTCAAGTTGCAAAAGGGTACGATCATCACCGTCATTGCTGCGCTCATTTT
GGTTATGTTGGGAAGTGCTGCACCCAAAGCTTCTGCTGCTGCTGGTTTTATGTA
AGCGGTAACAAGTTGTATGACTCTACGGGTAAAGCTTTTGTCATGCGGGGCGTCAA
CCACAGTCATACCTGGTTCAAGAACGATCTAAACACAGCGATACCCGCCATTGCAA
AAACAGGTGCGAACACGGTACGTATTGTGCTCTCCAATGGGACGCAATATACCAAA
GATGATTTGAACGCCGTTAAAAACATAATCAACCTGGTGAGTCAGAACAAAATGAT
CGCAGTGCTCGAAGTACATGATGCAACTGGTAAAGATGACTACAATTCGTTGGATG
CAGCAGTCAACTACTGGATTAGCATCAAGGAAGCTCTGATTGGCAAGGAAGACCGC
GTTATCGTCAATATTGCCAATGAATGGTACGGACCTGGAACGGCAGTGCCTGGGC
TGACGGGTACAAAAAAGCAATTCCGAAACTGAGAAATGCCGGCATTAAAAATACAT
TAATTGTAGATGCAGCTGGCTGGGGCCAATATCCGCAATCTATTGTGGACTATGGTC
AAAGTGTTTTTGCAGCAGATGCCCAGAAAAATACGGTTTTCTCCATTCACATGTATG
AATATGCAGGTAAAGATGCCGCAACGGTCAAAGCCAACATGGAAAACGTGCTGAA
CAAAGGTTTGGCCCTGATCATCGGTGAGTTTGGTGGATACCACACCAATGGGGACG
TCGATGAATATGCAATCATGAAATACGGTCAGGAAAAAGGAGTAGGCTGGCTCGCA
TGGTCCTGGTATGGGAACAACTCCGATCTCAATTATCTGGATTTGGCTACAGGTCCA

AACGGAACTTTAACAAGCTTTGGCAACACGGTGGTTTATGACACGTATGGAATTAA
AAACACTTCGGTAAAAGCAGGGATCTAT.

The amino acid sequence of the PpaMan2 precursor protein is set forth as SEQ ID NO:19 (the predicted native signal peptide is shown in italics and bold):

MVKLQKGTIITVIAALILVMLGSAAPKASAAAGFYVSGNKLYDSTGKAFVMRGVNHSHT
WFKNDLNTAIPAIAKTGANTVRIVLSNGTQYTKDDLNAVKNIINLVSQNKMIAVLEVHD
ATGKDDYNSLDAAVNYWISIKEALIGKEDRVIVNIANEWYGTWNGSAWADGYKKAIP
KLRNAGIKNTLIVDAAGWGQYPQSIVDYGQSVFAADAQKNTVFSIHMYEYAGKDAAT
VKANMENVLNKGLALIIGEFGGYHTNGDVDEYAIMKYGQEKGVGWLAWSWYGNNSD
LNYLDLATGPNGTLTSFGNTVVYDTYGIKNTSVKAGIY.

The nucleotide sequence of the *PspMan9* gene isolated from *Paenibacillus sp. FeL05* is set forth as SEQ ID NO:20 (the sequence encoding the predicted native signal peptide is shown in bold):

GTGTTTATGTTAGCGATGTATGGATGGGCTGGACTGACTGGTCAAGCTTCAGC
TGCTACAGGTTTTTATGTAAGCGGTACCAAATTATACGACTCTACAGGCAAGCCATT
TGTGATGCGTGGTGTGAATCATTCCCACACCTGGTTCAAAAATGACCTGAATGCAGC
GATCCCTGCAATTGCCAAAACAGGCGCCAACACGGTACGTATCGTATTATCGAATG
GCGTGCAGTACACCAGAGATGATGTAAACTCCGTCAAAAATATCATCTCTCTCGTCA
ACCAGAACAAAATGATCGCAGTACTGGAGGTTCATGATGCAACAGGCAAGGACGA
TTACGCTTCGCTCGATGCCGCAATCAACTACTGGATCAGCATCAAGGATGCGCTGAT
CGGTAAAGAGGATCGCGTTATCGTCAATATTGCCAACGAATGGTATGGCACATGGA
ATGGAAGCGCATGGGCAGATGGCTACAAACAGGCGATTCCAAAGCTCCGTAATGCG
GGTATAAAAAATACGCTGATTGTTGACGCAGCCGGCTGGGGTCAATATCCACAATC
GATCGTTGATTATGGACAAAGTGTATTTGCAGCGGATTCGTTAAAAAATACGGTTTT
CTCGATCCATATGTATGAGTATGCAGGTGGAACCGATGCGATGGTCAAAGCCAACA
TGGAGGGCGTACTCAATAAAGGTCTGCCACTGATCATTGGTGAATTTGGCGGACAG
CACACAAATGGAGACGTGGATGAGCTGGCGATCATGCGTTACGGACAACAAAAAG
GAGTAGGCTGGCTCGCCTGGTCCTGGTACGGCAACAATAGTGATCTGAGTTATCTCG
ATCTAGCGACAGGTCCAAATGGTAGCCTGACCACGTTTGGTAATACGGTGGTAAAT
GACACCAACGGTATCAAAGCCACCTCCAAAAAAGCAGGTATTTTCCAG.

The amino acid sequence of the PspMan9 precursor protein is set forth as SEQ ID NO:21 (the predicted native signal peptide is shown in italics and bold):

MFMLAMYGWAGLTGQASAATGFYVSGTKLYDSTGKPFVMRGVNHSHTWFKNDLNAA
IPAIAKTGANTVRIVLSNGVQYTRDDVNSVKNIISLVNQNKMIAVLEVHDATGKDDYAS

LDAAINYWISIKDALIGKEDRVIVNIANEWYGTWNGSAWADGYKQAIPKLRNAGIKNT
LIVDAAGWGQYPQSIVDYGQSVFAADSLKNTVFSIHMYEYAGGTDAMVKANMEGVLN
KGLPLIIGEFGGQHTNGDVDELAIMRYGQQKGVGWLAWSWYGNNSDLSYLDLATGPN
GSLTTFGNTVVNDTNGIKATSKKAGIFQ.

The nucleotide sequence of the *PtuMan2* gene isolated from *Paenibacillus tundrae* is set forth as SEQ ID NO:22 (the sequence encoding the predicted native signal peptide is shown in bold):

ATGGTCAAGTTGCAAAAGTGTACAGTCTTTACCGTAATTGCTGCACTTATGTTG
GTGATTCTGGCGAGTGCTGCACCCAAAGCGTCTGCTGCTACAGGATTTATGTA
AGCGGAGGCAAATTGTACGATTCTACTGGCAAGGCATTTGTTATGAGAGGTGTCAA
TCATGGACATTCATGGTTTAAGAACGACTTGAACACGGCTATTCCTGCGATAGCCAA
AACAGGTGCCAACACCGTACGGATTGTGCTCTCCAATGGCGTACAGTACACCAAAG
ACGATCTGAACTCTGTTAAAAACATCATTAATGTTGTAAGCGTAAACAAAATGATT
GCGGTGCTCGAAGTACATGATGCAACAGGTAAGGATGACTATAATTCGTTGGATGC
AGCGGTGAACTACTGGATTAGCATCAAGGAAGCACTCATTGGCAAAGAAGACAGA
GTTATCGTAAATATCGCGAACGAATGGTATGGAACATGGAACGGCAGTGCCTGGGC
TGACGGATACAAAAAAGCAATTCCGAAGCTGAGAAATGCCGGTATTAAAAATACAT
TGATCGTGGATGCAGCGGGCTGGGGGCAGTACCCGCAATCCATCGTGGATTATGGA
CAAAGTGTATTTGCAGCGGATTCACAGAAAAACACCGTATTCTCGATTCACATGTAT
GAATATGCCGGTAAAGACGCAGCAACCGTAAAAGCCAACATGGAAAGCGTATTAA
ACAAAGGTCTGGCCCTGATCATCGGTGAATTCGGTGGATATCACACGAACGGGGAT
GTCGATGAATATGCGATCATGAAATATGGTCAGGAAAAAGGGGTAGGCTGGCTCGC
ATGGTCCTGGTATGGCAATAGCTCCGATTTGAACTATTTGGACTTGGCTACGGACC
TAACGGAAGTTTGACTAGCTTTGGAAACACAGTCGTCAACGACACTTATGGAATCA
AAAATACTTCAAAAAAAGCAGGGATCTAC.

The amino acid sequence of the PtuMan2 precursor protein is set forth as SEQ ID NO:23 (the predicted native signal peptide is shown in bold):

**MVKLQKCTVFTVIAALMLVILASAAPKASA**ATGFYVSGGKLYDSTGKAFVMRGVNH
GHSWFKNDLNTAIPAIAKTGANTVRIVLSNGVQYTKDDLNSVKNIINVVSVNKMIAVLE
VHDATGKDDYNSLDAAVNYWISIKEALIGKEDRVIVNIANEWYGTWNGSAWADGYKK
AIPKLRNAGIKNTLIVDAAGWGQYPQSIVDYGQSVFAADSQKNTVFSIHMYEYAGKDA
ATVKANMESVLNKGLALIIGEFGGYHTNGDVDEYAIMKYGQEKGVGWLAWSWYGNS
SDLNYLDLATGPNGSLTSFGNTVVNDTYGIKNTSKKAGIY.

The sequence of the fully processed mature PtuMan2 (303 amino acids) is set forth as SEQ ID NO:24:

ATGFYVSGGKLYDSTGKAFVMRGVNHGHSWFKNDLNTAIPAIAKTGANTVRIVLSNGV
QYTKDDLNSVKNIINVVSVNKMIAVLEVHDATGKDDYNSLDAAVNYWISIKEALIGKE
DRVIVNIANEWYGTWNGSAWADGYKKAIPKLRNAGIKNTLIVDAAGWGQYPQSIVDY
GQSVFAADSQKNTVFSIHMYEYAGKDAATVKANMESVLNKGLALIIGEFGGYHTNGD
VDEYAIMKYGQEKGVGWLAWSWYGNSSDLNYLDLATGPNGSLTSFGNTVVNDTYGI
KNTSKKAGIY.

### EXAMPLE 2

### Heterologous Expression of mannanases

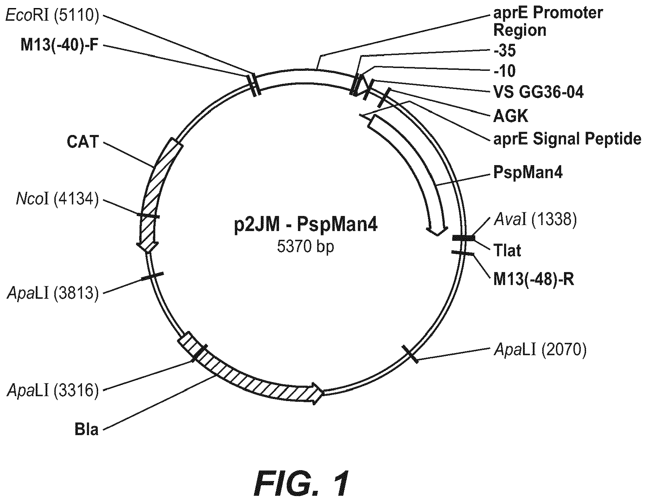

The DNA sequences of the mature forms of *BciMan1, BciMan3, BciMan4, PpaMan2, PpoMan1, PpoMan2, PspMan4, PspMan5,* and *PspMan9* genes were synthesized and inserted into the *B. subtilis* expression vector p2JM103BBI (Vogtentanz, Protein Expr Purif, 55:40-52, 2007) by Generay Biotech (Shanghai, China), resulting in expression plasmids containing an aprE promoter, an aprE signal sequence used to direct target protein secretion in *B. subtilis,* an oligonucleotide AGK-proAprE that encodes peptide Ala-Gly-Lys to facilitate the secretion of the target protein, and the synthetic nucleotide sequence encoding the mature region of the gene of interest. A representative plasmid map for PspMan4 expression plasmid (p2JM-PspMan4) is depicted in Figure 1.

A suitable *B. subtilis* host strain was transformed with each of the expression plasmids and the transformed cells were spread on Luria Agar plates supplemented with 5ppm chloramphenicol. To produce each of the mannanases listed above, *B. subtilis* transformants containing the plasmids were grown in a 250ml shake flask in a MOPS based defined medium, supplemented with additional 5mM CaCl₂.

The nucleotide sequence of the synthesized *BciMan1* gene in the expression plasmid p2JM-BciMan1 is set forth as SEQ ID NO:25 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAAGCGGCTTTTATGT
TTCAGGCACAAAACTGCTGGATGCAACAGGCCAACCGTTTGTTATGAGAGGCGTTA
ATCATGCACATACGTGGTATAAAGATCAACTGTCAACAGCAATTCCGGCAATCGCA
AAAACAGGCGCAAATACAATTAGAATTGTTCTGGCGAATGGCCATAAATGGACACT
GGATGATGTTAACACAGTCAACAATATTCTGACACTGTGCGAACAGAATAAACTGA

TTGCAGTTCTGGAAGTTCATGATGCGACAGGCTCAGATTCACTGTCAGATCTGGATA
ATGCAGTCAATTATTGGATCGGCATTAAATCAGCACTGATCGGCAAAGAAGATCGC
GTCATTATTAACATTGCGAACGAATGGTATGGCACATGGATGGCGTTGCATGGGC
AAATGGCTATAAACAAGCGATTCCGAAACTGAGAAATGCAGGCCTGACACATACAC
TGATTGTTGATTCAGCAGGCTGGGGACAATATCCGGATTCAGTTAAAAACTATGGC
ACAGAAGTTCTGAACGCAGATCCGCTGAAAAATACAGTCTTTAGCATCCACATGTA
CGAATATGCAGGCGGAAATGCATCAACAGTGAAATCAAATATTGATGGCGTCCTGA
ATAAAAACCTGGCACTGATTATTGGCGAATTTGGCGGACAACATACAAATGGCGAC
GTTGATGAAGCAACGATTATGTCATATAGCCAAGAAAAAGGCGTTGGCTGGCTTGC
ATGGTCATGGAAAGGCAATTCATCAGATCTTGCATATCTGGATATGACGAATGATT
GGGCAGGCAATAGCCTGACATCATTTGGCAATACAGTTGTCAATGGCAGCAATGGC
ATTAAAGCAACATCAGTTCTGTCAGGCATTTTTGGCGGAGTTACACCGACATCATCA
CCGACAAGCACACCGACGTCAACACCTACATCAACGCCGACACCGACACCTAGCCC
GACACCTTCACCGGGAAATAATGGCACAATTCTGTATGATTTTGAAACAGGCACAC
AAGGCTGGTCAGGCAATAACATTTCAGGCGGACCGTGGGTTACAAATGAATGGAAA
GCGACAGGCGCACAAACACTGAAAGCAGATGTTTCACTTCAAAGCAATTCAACGCA
TAGCCTGTATATCACAAGCAATCAAAATCTGAGCGGCAAATCAAGCCTGAAAGCAA
CAGTTAAACATGCGAATTGGGGCAATATTGGCAATGGAATTTATGCGAAACTGTAC
GTTAAAACAGGCAGCGGCTGGACATGGTATGATTCAGGCGAAAATCTGATTCAGTC
AAACGATGGAACAATCCTGACACTTTCACTTTCAGGCATTAGCAATCTGAGCAGCG
TTAAAGAAATTGGCGTCGAATTTAGAGCAAGCTCAAATAGCTCAGGCCAAAGCGCA
ATTTATGTTGATAGCGTTTCACTGCAG.

The amino acid sequence of the BciMan1 precursor protein expressed from the p2JM-BciMan1 plasmid is set forth as SEQ ID NO:26 (the predicted signal sequence is shown in italics, the three residue amino-terminal extension (AGK) is shown in bold):

MRSKKLWISLLFALTLIFTMAFSNMSAQAAGKASGFYVSGTKLLDATGQPFVMRGVNHA
HTWYKDQLSTAIPAIAKTGANTIRIVLANGHKWTLDDVNTVNNILTLCEQNKLIAVLEV
HDATGSDSLSDLDNAVNYWIGIKSALIGKEDRVIINIANEWYGTWDGVAWANGYKQAI
PKLRNAGLTHTLIVDSAGWGQYPDSVKNYGTEVLNADPLKNTVFSIHMYEYAGGNAST
VKSNIDGVLNKNLALIIGEFGGQHTNGDVDEATIMSYSQEKGVGWLAWSWKGNSSDL
AYLDMTNDWAGNSLTSFGNTVVNGSNGIKATSVLSGIFGGVTPTSSPTSTPTSTPTSTPT
PTPSPTPSPGNNGTILYDFETGTQGWSGNNISGGPWVTNEWKATGAQTLKADVSLQSNS
THSLYITSNQNLSGKSSLKATVKHANWGNIGNGIYAKLYVKTGSGWTWYDSGENLIQS
NDGTILTLSLSGISNLSSVKEIGVEFRASSNSSGQSAIYVDSVSLQ.

The amino acid sequence of the BciMan1 mature protein expressed from p2JM-BciMan1 plasmid is set forth as SEQ ID NO:27 (the three residue amino-terminal extension (AGK) based on the predicted cleavage site shown in bold):

AGKASGFYVSGTKLLDATGQPFVMRGVNHAHTWYKDQLSTAIPAIAKTGANTIRIVLA
NGHKWTLDDVNTVNNILTLCEQNKLIAVLEVHDATGSDSLSDLDNAVNYWIGIKSALI
GKEDRVIINIANEWYGTWDGVAWANGYKQAIPKLRNAGLTHTLIVDSAGWGQYPDSV
KNYGTEVLNADPLKNTVFSIHMYEYAGGNASTVKSNIDGVLNKNLALIIGEFGGQHTN
GDVDEATIMSYSQEKGVGWLAWSWKGNSSDLAYLDMTNDWAGNSLTSFGNTVVNGS
NGIKATSVLSGIFGGVTPTSSPTSTPTSTPTSTPTPTPSPTPSPGNNGTILYDFETGTQGWS
GNNISGGPWVTNEWKATGAQTLKADVSLQSNSTHSLYITSNQNLSGKSSLKATVKHAN
WGNIGNGIYAKLYVKTGSGWTWYDSGENLIQSNDGTILTLSLSGISNLSSVKEIGVEFRA
SSNSSGQSAIYVDSVSLQ.

The amino acid sequence of the BciMan1 mature protein, based on the predicted cleavage of the naturally occurring sequence, is set forth as SEQ ID NO:28:

ASGFYVSGTKLLDATGQPFVMRGVNHAHTWYKDQLSTAIPAIAKTGANTIRIVLANGH
KWTLDDVNTVNNILTLCEQNKLIAVLEVHDATGSDSLSDLDNAVNYWIGIKSALIGKED
RVIINIANEWYGTWDGVAWANGYKQAIPKLRNAGLTHTLIVDSAGWGQYPDSVKNYG
TEVLNADPLKNTVFSIHMYEYAGGNASTVKSNIDGVLNKNLALIIGEFGGQHTNGDVDE
ATIMSYSQEKGVGWLAWSWKGNSSDLAYLDMTNDWAGNSLTSFGNTVVNGSNGIKA
TSVLSGIFGGVTPTSSPTSTPTSTPTSTPTPTPSPTPSPGNNGTILYDFETGTQGWSGNNISG
GPWVTNEWKATGAQTLKADVSLQSNSTHSLYITSNQNLSGKSSLKATVKHANWGNIG
NGIYAKLYVKTGSGWTWYDSGENLIQSNDGTILTLSLSGISNLSSVKEIGVEFRASSNSS
GQSAIYVDSVSLQ.

The nucleotide sequence of the synthesized *BciMan3* gene in the p2JM-BciMan3 plasmid is set forth as SEQ ID NO:29 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAACAGGCTTTTATGT
CAATGGCACGAAACTGTATGATAGCACAGGCAAAGCATTTGTTATGAGAGGCGTTA
ATCATCCGCATACGTGGTATAAAAACGATCTGAATGCAGCAATTCCGGCTATTGCA
CAAACAGGCGCAAATACAGTTAGAGTTGTTCTGTCAAATGGCAGCCAATGGACAAA
AGATGATCTGAATAGCGTCAACAGCATTATTTCACTGGTTAGCCAACATCAAATGAT
TGCAGTTCTGGAAGTTCATGATGCAACGGGCAAAGATGAATATGCATCACTGGAAG
CAGCAGTCGATTATTGGATTTCAATTAAAGGCGCACTGATCGGCAAAGAAGATAGA
GTCATTGTCAATATTGCGAACGAATGGTATGGCAATTGGAATTCATCAGGCTGGGC
AGATGGCTATAAACAAGCGATTCCGAAACTGAGAAATGCAGGCATTAAAAACACA
CTGATTGTTGATGCAGCAGGCTGGGGACAATATCCGCAATCAATTGTCGATGAAGG
CGCAGCAGTTTTTGCATCAGATCAACTGAAAAACACGGTCTTTAGCATCCACATGTA
TGAATACGCTGGAAAAGATGCAGCAACAGTCAAAACAAATATGGATGACGTTCTGA
ATAAAGGCCTGCCGCTGATTATTGGCGAATTTGGCGGATATCATCAAGGCGCAGAT
GTTGATGAAATTGCGATTATGAAATACGGCCAGCAAAAAGAGGTTGGCTGGCTTGC
ATGGTCATGGTATGGAAACTCACCGGAACTGAATGATCTGGATCTGGCAGCAGGAC
CGTCAGGCAATCTGACAGGATGGGGCAATACAGTTGTTCATGGCACAGATGGCATT
CAACAGACATCAAAAAAAGCAGGCATCTAT.

The amino acid sequence of the BciMan3 precursor protein expressed from the p2JM-BciMan3 plasmid is set forth as SEQ ID NO:30 (the predicted signal sequence is shown in italics, the three residue amino-terminal extension (AGK) is shown in bold):

MRSKKLWISLLFALTLIFTMAFSNMSAQAAGKATGFYVNGTKLYDSTGKAFVMRGVNHP
HTWYKNDLNAAIPAIAQTGANTVRVVLSNGSQWTKDDLNSVNSIISLVSQHQMIAVLE
VHDATGKDEYASLEAAVDYWISIKGALIGKEDRVIVNIANEWYGNWNSSGWADGYKQ
AIPKLRNAGIKNTLIVDAAGWGQYPQSIVDEGAAVFASDQLKNTVFSIHMYEYAGKDA
ATVKTNMDDVLNKGLPLIIGEFGGYHQGADVDEIAIMKYGQQKEVGWLAWSWYGNSP
ELNDLDLAAGPSGNLTGWGNTVVHGTDGIQQTSKKAGIY.

The amino acid sequence of the BciMan3 mature protein expressed from p2JM-BciMan3 is set forth as SEQ ID NO:31 (the three residue amino-terminal extension based on the predicted cleavage site shown in bold):

AGKATGFYVNGTKLYDSTGKAFVMRGVNHPHTWYKNDLNAAIPAIAQTGANTVRVV
LSNGSQWTKDDLNSVNSIISLVSQHQMIAVLEVHDATGKDEYASLEAAVDYWISIKGAL
IGKEDRVIVNIANEWYGNWNSSGWADGYKQAIPKLRNAGIKNTLIVDAAGWGQYPQSI

VDEGAAVFASDQLKNTVFSIHMYEYAGKDAATVKTNMDDVLNKGLPLIIGEFGGYHQ
GADVDEIAIMKYGQQKEVGWLAWSWYGNSPELNDLDLAAGPSGNLTGWGNTVVHGT
DGIQQTSKKAGIY.

The amino acid sequence of the BciMan3 mature protein, based on the predicted cleavage of the naturally occurring sequence, is set forth as SEQ ID NO:32:

ATGFYVNGTKLYDSTGKAFVMRGVNHPHTWYKNDLNAAIPAIAQTGANTVRVVLSNG
SQWTKDDLNSVNSIISLVSQHQMIAVLEVHDATGKDEYASLEAAVDYWISIKGALIGKE
DRVIVNIANEWYGNWNSSGWADGYKQAIPKLRNAGIKNTLIVDAAGWGQYPQSIVDE
GAAVFASDQLKNTVFSIHMYEYAGKDAATVKTNMDDVLNKGLPLIIGEFGGYHQGAD
VDEIAIMKYGQQKEVGWLAWSWYGNSPELNDLDLAAGPSGNLTGWGNTVVHGTDGI
QQTSKKAGIY.

The nucleotide sequence of the synthesized *BciMan4* gene in the expression plasmid p2JM-BciMan4 is set forth as SEQ ID NO:33 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAACAGGCTTTTATGT
TAATGGCGGAAAACTGTATGATAGCACAGGCAAACCGTTTTATATGCGTGGCATTA
ATCATGGCCATAGCTGGTTTAAAAACGATCTGAATACAGCGATTCCGGCTATTGCA
AAAACAGGCGCAAATACAGTTAGAATTGTTCTGTCAAATGGCACGCAGTATACGAA
AGATGATCTGAACTCAGTCAAAAACATCATCAATGTCGTCAACGCGAACAAAATGA
TTGCAGTTCTGGAAGTTCATGATGCAACGGGCAAAGATGATTTCAATTCACTGGATG
CAGCAGTCAACTATTGGATCTCAATTAAAGAAGCGCTGATCGGCAAAGAAGATCGC
GTTATTGTTAATATTGCGAACGAATGGTATGGCACATGGAATGGCTCAGCATGGGC
AGATGGCTACAAAAAAGCAATTCCGAAACTGAGAGATGCAGGCATTAAAAACACA
CTGATTGTTGATGCGGCAGGCTGGGGACAATATCCGCAATCAATTGTTGATTATGGC
CAAAGCGTTTTTGCAGCAGATAGCCAGAAAAATACAGCGTTTAGCATCCACATGTA
TGAATATGCGGGAAAAGATGCAGCAACAGTCAAAAGCAATATGGAAAACGTCCTG
AATAAAGGCCTGGCACTGATTATTGGCGAATTTGGCGGATATCATACAAATGGCGA
CGTTGACGAATATGCGATTATGAAATATGGCCTGGAAAAAGGCGTTGGCTGGCTTG
CATGGTCATGGTATGGAAATTCATCAGGCCTTAATTATCTGGATCTGGCAACAGGAC
CGAATGGCAGCCTGACATCATATGGCAATACAGTTGTCAATGATACGTATGGCATC
AAAAATACGTCACAGAAAGCAGGCATCTTT.

The amino acid sequence of the BciMan4 precursor protein expressed from plasmid p2JM-BciMan4 is set forth as SEQ ID NO:34 (the predicted signal sequence is shown in italics, the three residue amino-terminal extension (AGK) is shown in bold):

MRSKKLWISLLFALTLIFTMAFSNMSAQAAGKATGFYVNGGKLYDSTGKPFYMRGINHGH
SWFKNDLNTAIPAIAKTGANTVRIVLSNGTQYTKDDLNSVKNIINVVNANKMIAVLEVH
DATGKDDFNSLDAAVNYWISIKEALIGKEDRVIVNIANEWYGTWNGSAWADGYKKAIP
KLRDAGIKNTLIVDAAGWGQYPQSIVDYGQSVFAADSQKNTAFSIHMYEYAGKDAAT
VKSNMENVLNKGLALIIGEFGGYHTNGDVDEYAIMKYGLEKGVGWLAWSWYGNSSG
LNYLDLATGPNGSLTSYGNTVVNDTYGIKNTSQKAGIF.

The amino acid sequence of the BciMan4 mature protein expressed from p2JM-BciMan4 is set forth as SEQ ID NO:35 (the three residue amino-terminal extension based on the predicted cleavage site shown in bold):

AGKATGFYVNGGKLYDSTGKPFYMRGINHGHSWFKNDLNTAIPAIAKTGANTVRIVLS
NGTQYTKDDLNSVKNIINVVNANKMIAVLEVHDATGKDDFNSLDAAVNYWISIKEALI
GKEDRVIVNIANEWYGTWNGSAWADGYKKAIPKLRDAGIKNTLIVDAAGWGQYPQSI
VDYGQSVFAADSQKNTAFSIHMYEYAGKDAATVKSNMENVLNKGLALIIGEFGGYHT
NGDVDEYAIMKYGLEKGVGWLAWSWYGNSSGLNYLDLATGPNGSLTSYGNTVVNDT
YGIKNTSQKAGIF.

The amino acid sequence of the BciMan4 mature protein, based on the predicted cleavage of the naturally occurring sequence, is set forth as SEQ ID NO:36:

ATGFYVNGGKLYDSTGKPFYMRGINHGHSWFKNDLNTAIPAIAKTGANTVRIVLSNGT
QYTKDDLNSVKNIINVVNANKMIAVLEVHDATGKDDFNSLDAAVNYWISIKEALIGKE
DRVIVNIANEWYGTWNGSAWADGYKKAIPKLRDAGIKNTLIVDAAGWGQYPQSIVDY
GQSVFAADSQKNTAFSIHMYEYAGKDAATVKSNMENVLNKGLALIIGEFGGYHTNGD
VDEYAIMKYGLEKGVGWLAWSWYGNSSGLNYLDLATGPNGSLTSYGNTVVNDTYGI
KNTSQKAGIF.

The nucleotide sequence of the synthesized *PpaMan2* gene in plasmid p2JM-PpaMan2 is set forth as SEQ ID NO:37 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAGCAGGCTTTTATGT
TTCAGGCAACAAGCTGTATGATTCAACAGGAAAAGCATTTGTTATGAGAGGCGTTA
ATCATTCACATACATGGTTTAAGAACGATCTTAATACAGCCATTCCGGCAATCGCGA
AGACAGGAGCAAATACAGTGAGAATTGTTCTTTCAAACGGAACGCAATATACAAAA
GATGACCTGAACGCCGTTAAGAATATCATTAATCTGGTTTCACAAAATAAGATGATT

GCAGTTCTGGAGGTTCATGATGCAACAGGCAAGGATGACTACAATAGCCTGGATGC
AGCGGTCAATTACTGGATTTCAATTAAAGAAGCACTTATTGGCAAAGAGGATAGAG
TTATTGTTAATATCGCAAATGAATGGTATGGAACGTGGAACGGCTCAGCATGGGCA
GATGGCTACAAAAAAGCAATTCCGAAACTGAGAAATGCAGGAATCAAAAATACAC
TGATTGTTGACGCCGCAGGCTGGGGACAATATCCGCAAAGCATCGTTGATTATGGC
CAAAGCGTTTTTGCCGCAGACGCACAGAAAAACACGGTTTTCTCAATTCATATGTAC
GAGTATGCTGGAAAGGATGCTGCAACGGTTAAAGCTAACATGGAAAATGTTCTGAA
TAAAGGCCTGGCACTGATCATTGGCGAATTTGGAGGCTATCACACAAATGGCGATG
TTGATGAATACGCAATTATGAAATATGGACAAGAAAAAGGCGTTGGATGGCTTGCA
TGGTCATGGTACGGAAACAACTCAGACCTTAATTACCTGGACCTGGCTACGGGACC
GAATGGCACACTGACATCATTCGGCAATACGGTCGTTTATGACACGTATGGCATCA
AGAACACGAGCGTGAAAGCCGGCATTTAT.

The amino acid sequence of the PpaMan2 precursor protein expressed from plasmid p2JM-PpaMan2 is set forth as SEQ ID NO:38 (the predicted signal sequence is shown in italics, the three residue amino-terminal extension(AGK) is shown in bold):

MRSKKLWISLLFALTLIFTMAFSNMSAQAAGKAAGFYVSGNKLYDSTGKAFVMRGVNHS
HTWFKNDLNTAIPAIAKTGANTVRIVLSNGTQYTKDDLNAVKNIINLVSQNKMIAVLEV
HDATGKDDYNSLDAAVNYWISIKEALIGKEDRVIVNIANEWYGTWNGSAWADGYKKA
IPKLRNAGIKNTLIVDAAGWGQYPQSIVDYGQSVFAADAQKNTVFSIHMYEYAGKDAA
TVKANMENVLNKGLALIIGEFGGYHTNGDVDEYAIMKYGQEKGVGWLAWSWYGNNS
DLNYLDLATGPNGTLTSFGNTVVYDTYGIKNTSVKAGIY.

The amino acid sequence of the PpaMan2 mature protein expressed from p2JM-PpaMan2 is set forth as SEQ ID NO:39 (the three residue amino-terminal extension (AGK) based on the predicted cleavage site shown in bold):

AGKAAGFYVSGNKLYDSTGKAFVMRGVNHSHTWFKNDLNTAIPAIAKTGANTVRIVL
SNGTQYTKDDLNAVKNIINLVSQNKMIAVLEVHDATGKDDYNSLDAAVNYWISIKEAL
IGKEDRVIVNIANEWYGTWNGSAWADGYKKAIPKLRNAGIKNTLIVDAAGWGQYPQSI
VDYGQSVFAADAQKNTVFSIHMYEYAGKDAATVKANMENVLNKGLALIIGEFGGYHT
NGDVDEYAIMKYGQEKGVGWLAWSWYGNNSDLNYLDLATGPNGTLTSFGNTVVYDT
YGIKNTSVKAGIY.

The amino acid sequence of the PpaMan2 mature protein, based on the predicted cleavage of the naturally occurring sequence, is set forth as SEQ ID NO:40:

AAGFYVSGNKLYDSTGKAFVMRGVNHSHTWFKNDLNTAIPAIAKTGANTVRIVLSNGT
QYTKDDLNAVKNIINLVSQNKMIAVLEVHDATGKDDYNSLDAAVNYWISIKEALIGKE

DRVIVNIANEWYGTWNGSAWADGYKKAIPKLRNAGIKNTLIVDAAGWGQYPQSIVDY
GQSVFAADAQKNTVFSIHMYEYAGKDAATVKANMENVLNKGLALIIGEFGGYHTNGD
VDEYAIMKYGQEKGVGWLAWSWYGNNSDLNYLDLATGPNGTLTSFGNTVVYDTYGI
KNTSVKAGIY.

The nucleotide sequence of the synthesized *PpoMan1* gene in plasmid p2JM-PpoMan1 is set forth as SEQ ID NO:41(the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAAGCGGCTTTTATGT
TTCAGGCACAAAACTGTATGATAGCACAGGCAAACCGTTTGTTATGAGAGGCGTTA
ATCATGCACATACGTGGTATAAAAACGATCTGTATACGGCAATTCCGGCTATTGCAC
AAACAGGCGCAAATACAGTTAGAATTGTTCTGAGCAATGGCAACCAGTATACGAAA
GATGATATCAACAGCGTCAAAAACATTATCAGCCTGGTCAGCAACTATAAAATGAT
TGCAGTTCTGGAAGTCCATGATGCAACGGGCAAAGATGATTATGCATCACTGGATG
CAGCAGTCAATTATTGGATTAGCATTAAAGATGCGCTGATCGGCAAAGAAGATCGC
GTTATTGTTAATATTGCGAACGAATGGTATGGCTCATGGAATGGCTCAGGCTGGGC
AGATGGCTATAAACAAGCAATTCCGAAACTGAGAAATGCAGGCATTAAAAACACA
CTGATTGTTGATTGCGCAGGCTGGGGACAATATCCGCAATCAATTAATGATTTTGGC
AAAAGCGTTTTTGCAGCGGATAGCCTGAAAAATACAGTCTTTAGCATCCATATGTAT
GAATTTGCGGGAAAAGATGCACAGACAGTCCGCACAAATATTGATAATGTCCTGAA
TCAAGGCATCCCGCTGATTATTGGCGAATTTGGCGGATATCATCAAGGCGCAGATG
TTGATGAAACAGAAATTATGAGATACGGCCAATCAAAAGGCGTTGGCTGGCTTGCA
TGGTCATGGTATGGAAATTCAAGCAATCTGTCATATCTGGATCTGGTTACAGGACCG
AATGGCAATCTTACAGATTGGGGCAAAACAGTTGTTAATGGCTCAAATGGCATCAA
AGAAACGTCAAAAAAAGCAGGCATCTAT.

The amino acid sequence of the PpoMan1 precursor protein expressed from plasmid p2JM-PpoMan1 is set forth as SEQ ID NO:42 (the predicted signal sequence is shown in italics, the three residue amino-terminal extension(AGK) is shown in bold):

MRSKKLWISLLFALTLIFTMAFSNMSAQAAGKASGFYVSGTKLYDSTGKPFVMRGVNHAH
TWYKNDLYTAIPAIAQTGANTVRIVLSNGNQYTKDDINSVKNIISLVSNYKMIAVLEVH
DATGKDDYASLDAAVNYWISIKDALIGKEDRVIVNIANEWYGSWNGSGWADGYKQAI
PKLRNAGIKNTLIVDCAGWGQYPQSINDFGKSVFAADSLKNTVFSIHMYEFAGKDAQT
VRTNIDNVLNQGIPLIIGEFGGYHQGADVDETEIMRYGQSKGVGWLAWSWYGNSSNLS
YLDLVTGPNGNLTDWGKTVVNGSNGIKETSKKAGIY.

The amino acid sequence of the PpoMan1 mature protein expressed from p2JM-PpoMan1 is set forth as SEQ ID NO:43 (the three residue amino-terminal extension based on the predicted cleavage site shown in bold):

AGKASGFYVSGTKLYDSTGKPFVMRGVNHAHTWYKNDLYTAIPAIAQTGANTVRIVLS
NGNQYTKDDINSVKNIISLVSNYKMIAVLEVHDATGKDDYASLDAAVNYWISIKDALIG
KEDRVIVNIANEWYGSWNGSGWADGYKQAIPKLRNAGIKNTLIVDCAGWGQYPQSIN
DFGKSVFAADSLKNTVFSIHMYEFAGKDAQTVRTNIDNVLNQGIPLIIGEFGGYHQGAD
VDETEIMRYGQSKGVGWLAWSWYGNSSNLSYLDLVTGPNGNLTDWGKTVVNGSNGI
KETSKKAGIY.

The amino acid sequence of the PpoMan1 mature protein, based on the predicted cleavage of the naturally occurring sequence, is set forth as SEQ ID NO:44:

ASGFYVSGTKLYDSTGKPFVMRGVNHAHTWYKNDLYTAIPAIAQTGANTVRIVLSNGN
QYTKDDINSVKNIISLVSNYKMIAVLEVHDATGKDDYASLDAAVNYWISIKDALIGKED
RVIVNIANEWYGSWNGSGWADGYKQAIPKLRNAGIKNTLIVDCAGWGQYPQSINDFG
KSVFAADSLKNTVFSIHMYEFAGKDAQTVRTNIDNVLNQGIPLIIGEFGGYHQGADVDE
TEIMRYGQSKGVGWLAWSWYGNSSNLSYLDLVTGPNGNLTDWGKTVVNGSNGIKETS
KKAGIY.

The nucleotide sequence of the synthesized *PpoMan2* gene in plasmid p2JM-PpoMan2 is set forth as SEQ ID NO:45 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAAGCGGCTTTTATGT
TTCAGGCACAAATCTGTATGATAGCACAGGCAAACCGTTTGTTATGAGAGGCGTTA
ATCATGCACATACGTGGTATAAAAACGATCTGTATACGGCAATTCCGGCAATCGCA
AAAACAGGCGCAAATACAGTTAGAATTGTTCTGAGCAATGGCAACCAGTATACGAA
AGATGATATCAACAGCGTCAAAAACATTATCAGCCTGGTCAGCAACCATAAAATGA
TTGCAGTTCTGGAAGTTCATGATGCAACGGGCAAAGATGATTATGCATCACTGGAT
GCAGCAGTCAATTATTGGATTAGCATTAAAGATGCGCTGATCGGCAAAGAAGATCG

CGTTATTGTTAATATTGCGAACGAATGGTATGGCTCATGGAATGGCGGAGGCTGGG
CAGATGGCTATAAACAAGCAATTCCGAAACTGAGAAATGCAGGCATTAAAAACAC
ACTGATTGTTGATTGCGCAGGCTGGGGACAATATCCGCAATCAATTAATGATTTTGG
CAAAAGCGTTTTTGCAGCGGATAGCCTGAAAAATACAGTCTTTAGCATCCATATGTA
TGAATTTGCAGGCAAAGACGTCCAAACAGTCCGCACAAATATTGATAATGTCCTGT
ATCAAGGCCTGCCGCTGATTATTGGCGAATTTGGCGGATATCATCAAGGCGCAGAT
GTTGATGAAACAGAAATTATGAGATACGGCCAGTCAAAATCAGTTGGCTGGCTTGC
ATGGTCATGGTATGGAAATTCAAGCAATCTGAACTATCTGGATCTGGTTACAGGAC
CGAATGGCAATCTTACAGATTGGGGCAGAACAGTTGTTGAAGGCGCTAATGGAATT
AAAGAAACGTCAAAAAAAGCAGGCATTTTT.

The amino acid sequence of the PpoMan2 precursor protein expressed from plasmid p2JM-PpoMan2 is set forth as SEQ ID NO:46 (the predicted signal sequence is shown in italics, the three residue amino-terminal extension (AGK) is shown in bold):

MRSKKLWISLLFALTLIFTMAFSNMSAQAAGKASGFYVSGTNLYDSTGKPFVMRGVNHAH
TWYKNDLYTAIPAIAKTGANTVRIVLSNGNQYTKDDINSVKNIISLVSNHKMIAVLEVH
DATGKDDYASLDAAVNYWISIKDALIGKEDRVIVNIANEWYGSWNGGGWADGYKQAI
PKLRNAGIKNTLIVDCAGWGQYPQSINDFGKSVFAADSLKNTVFSIHMYEFAGKDVQT
VRTNIDNVLYQGLPLIIGEFGGYHQGADVDETEIMRYGQSKSVGWLAWSWYGNSSNLN
YLDLVTGPNGNLTDWGRTVVEGANGIKETSKKAGIF.

The amino acid sequence of the PpoMan2 mature protein expressed from p2JM-PpoMan2 is set forth as SEQ ID NO:47 (the three residue amino-terminal extension (AGK) based on the predicted cleavage site shown in bold):

AGKASGFYVSGTNLYDSTGKPFVMRGVNHAHTWYKNDLYTAIPAIAKTGANTVRIVLS
NGNQYTKDDINSVKNIISLVSNHKMIAVLEVHDATGKDDYASLDAAVNYWISIKDALIG
KEDRVIVNIANEWYGSWNGGGWADGYKQAIPKLRNAGIKNTLIVDCAGWGQYPQSIN
DFGKSVFAADSLKNTVFSIHMYEFAGKDVQTVRTNIDNVLYQGLPLIIGEFGGYHQGAD
VDETEIMRYGQSKSVGWLAWSWYGNSSNLNYLDLVTGPNGNLTDWGRTVVEGANGI
KETSKKAGIF.

The amino acid sequence of the PpoMan2 mature protein, based on the predicted cleavage of the naturally occurring sequence, is set forth as SEQ ID NO:48:

ASGFYVSGTNLYDSTGKPFVMRGVNHAHTWYKNDLYTAIPAIAKTGANTVRIVLSNGN
QYTKDDINSVKNIISLVSNHKMIAVLEVHDATGKDDYASLDAAVNYWISIKDALIGKED
RVIVNIANEWYGSWNGGGWADGYKQAIPKLRNAGIKNTLIVDCAGWGQYPQSINDFG
KSVFAADSLKNTVFSIHMYEFAGKDVQTVRTNIDNVLYQGLPLIIGEFGGYHQGADVDE

TEIMRYGQSKSVGWLAWSWYGNSSNLNYLDLVTGPNGNLTDWGRTVVEGANGIKETS
KKAGIF.

The nucleotide sequence of the synthesized *PspMan4* gene in plasmid p2JM-PspMan4 is set forth as SEQ ID NO:49 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAATGGCGACAGGCTTTT
ATGTTTCAGGCAACAAACTGTATGATAGCACAGGCAAACCGTTTGTTATGAGAGGC
GTTAATCATGGCCATAGCTGGTTTAAAAACGATCTGAATACAGCGATTCCGGCTATT
GCAAAAACAGGCGCAAATACAGTTAGAATTGTTCTGTCAAATGGCAGCCTGTATAC
GAAAGATGATCTGAATGCAGTCAAAAACATCATCAATGTCGTCAACCAGAACAAAA
TGATTGCAGTTCTGGAAGTTCATGATGCAACGGGCAAAGATGATTACAATTCACTG
GATGCAGCAGTCAACTATTGGATCTCAATTAAAGAAGCGCTGATCGGCAAAGAAGA
TCGCGTTATTGTTAATATTGCGAACGAATGGTATGGCACATGGAATGGCTCAGCATG
GGCAGATGGCTACAAAAAAGCAATTCCGAAACTGAGAAATGCAGGCATCAAAAAC
ACACTGATTGTTGATGCGGCAGGCTGGGGACAATTTCCGCAATCAATTGTTGATTAT
GGCCAAAGCGTTTTTGCAGCAGATAGCCAGAAAAATACAGTCTTTAGCATCCATAT
GTACGAATACGCTGGAAAAGATGCAGCAACAGTTAAAGCGAATATGGAAAACGTC
CTGAATAAAGGCCTGGCACTGATTATTGGCGAATTTGGCGGATATCATACAAATGG
CGACGTTGATGAATATGCGATTATGAGATATGGCCAAGAAAAAGGCGTTGGCTGGC
TTGCATGGTCATGGTATGGAAATTCATCAGGCCTTAACTATCTGGATATGGCAACAG
GACCGAATGGATCACTGACATCATTTGGCAATACAGTCGTCAATGATACGTATGGA
ATCAAAAATACGAGCCAGAAAGCTGGCATCTTT.

The amino acid sequence of the *PspMan4* precursor protein expressed from plasmid p2JM-PspMan4 is set forth as SEQ ID NO:50 (the predicted signal sequence is shown in italics, the three residue amino-terminal extension (AGK) is shown in bold):

MRSKKLWISLLFALTLIFTMAFSNMSAQAAGKMATGFYVSGNKLYDSTGKPFVMRGVNH
GHSWFKNDLNTAIPAIAKTGANTVRIVLSNGSLYTKDDLNAVKNIINVVNQNKMIAVLE
VHDATGKDDYNSLDAAVNYWISIKEALIGKEDRVIVNIANEWYGTWNGSAWADGYKK
AIPKLRNAGIKNTLIVDAAGWGQFPQSIVDYGQSVFAADSQKNTVFSIHMYEYAGKDA
ATVKANMENVLNKGLALIIGEFGGYHTNGDVDEYAIMRYGQEKGVGWLAWSWYGNS
SGLNYLDMATGPNGSLTSFGNTVVNDTYGIKNTSQKAGIF.

The amino acid sequence of the confirmed PspMan4 mature protein expressed from p2JM-PspMan4 is set forth as SEQ ID NO:51 (the three residue amino-terminal extension (AGK) based on the predicted cleavage site shown in bold):

AGKMATGFYVSGNKLYDSTGKPFVMRGVNHGHSWFKNDLNTAIPAIAKTGANTVRIV
LSNGSLYTKDDLNAVKNIINVVNQNKMIAVLEVHDATGKDDYNSLDAAVNYWISIKEA
LIGKEDRVIVNIANEWYGTWNGSAWADGYKKAIPKLRNAGIKNTLIVDAAGWGQFPQS
IVDYGQSVFAADSQKNTVFSIHMYEYAGKDAATVKANMENVLNKGLALIIGEFGGYHT
NGDVDEYAIMRYGQEKGVGWLAWSWYGNSSGLNYLDMATGPNGSLTSFGNTVVNDT
YGIKNTSQKAGIF.

The amino acid sequence of the confirmed PspMan4 mature protein, based on the predicted cleavage of the naturally occurring sequence, is set forth as SEQ ID NO:52:

MATGFYVSGNKLYDSTGKPFVMRGVNHGHSWFKNDLNTAIPAIAKTGANTVRIVLSNG
SLYTKDDLNAVKNIINVVNQNKMIAVLEVHDATGKDDYNSLDAAVNYWISIKEALIGK
EDRVIVNIANEWYGTWNGSAWADGYKKAIPKLRNAGIKNTLIVDAAGWGQFPQSIVD
YGQSVFAADSQKNTVFSIHMYEYAGKDAATVKANMENVLNKGLALIIGEFGGYHTNG
DVDEYAIMRYGQEKGVGWLAWSWYGNSSGLNYLDMATGPNGSLTSFGNTVVNDTYG
IKNTSQKAGIF.

The nucleotide sequence of the synthesized *PspMan5* gene in plasmid p2JM-PspMan5 is set forth as SEQ ID NO:53 (the gene has an alternative start codon (GTG), the oligonucleotide encoding the three residue amino-terminal extension (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAACAGGCTTTTATGT
TTCAGGCACAACACTGTATGATTCAACAGGCAAACCGTTTGTTATGAGAGGCGTTA
ATCATAGCCATACGTGGTTTAAAAACGATCTGAATGCAGCAATTCCGGCAATCGCA
AAAACAGGCGCAAATACAGTTAGAATTGTTCTGTCAAATGGCGTCCAGTATACAAG
AGATGATGTCAATAGCGTCAAAAACATTATCAGCCTGGTCAACCAGAACAAAATGA
TTGCAGTTCTGGAAGTTCATGATGCGACAGGCAAAGATGATTATGCATCACTGGAT
GCAGCAGTCAATTATTGGATTAGCATTAAAGATGCGCTGATCGGCAAAGAAGATCG
CGTTATTGTTAATATTGCGAACGAATGGTATGGCACATGGAATGGCTCAGCATGGG
CAGATGGCTATAAACAAGCGATTCCGAAACTGAGAAATGCAGGCATTAAAAACAC
ACTGATTGTTGATGCGGCAGGCTGGGGACAATGTCCGCAATCAATTGTTGATTATGG
CCAATCAGTTTTTGCAGCGGATAGCCTGAAAAACACAATCTTTAGCATCCATATGTA
TGAATATGCAGGCGGAACGGATGCAATTGTCAAAAGCAATATGGAAAACGTCCTGA
ATAAAGGCCTGCCGCTGATTATTGGCGAATTTGGCGGACAACATACAAATGGCGAC
GTTGATGAACATGCAATTATGAGATATGGCCAACAAAAAGGCGTTGGCTGGCTTGC
ATGGTCATGGTATGGAAATAATTCAGAACTGAGCTATCTGGATCTGGCAACAGGAC

CGGCAGGCTCACTGACATCAATTGGAAATACAATTGTGAACGATCCGTATGGCATT
AAAGCGACATCAAAAAAAGCAGGCATTTTT.

The amino acid sequence of the PspMan5 precursor protein expressed from plasmid p2JM-PspMan5 is set forth as SEQ ID NO:54 (the predicted signal sequence is shown in italics, the three residue amino-terminal extension (AGK) is shown in bold):

MRSKKLWISLLFALTLIFTMAFSNMSAQAAGKATGFYVSGTTLYDSTGKPFVMRGVNHSH
TWFKNDLNAAIPAIAKTGANTVRIVLSNGVQYTRDDVNSVKNIISLVNQNKMIAVLEVH
DATGKDDYASLDAAVNYWISIKDALIGKEDRVIVNIANEWYGTWNGSAWADGYKQAI
PKLRNAGIKNTLIVDAAGWGQCPQSIVDYGQSVFAADSLKNTIFSIHMYEYAGGTDAIV
KSNMENVLNKGLPLIIGEFGGQHTNGDVDEHAIMRYGQQKGVGWLAWSWYGNNSEL
SYLDLATGPAGSLTSIGNTIVNDPYGIKATSKKAGIF.

The amino acid sequence of the PspMan5 mature protein expressed from p2JM-PspMan5 is set forth as SEQ ID NO:55 (the three residue amino-terminal extension (AGK) based on the predicted cleavage site shown in bold):

AGKATGFYVSGTTLYDSTGKPFVMRGVNHSHTWFKNDLNAAIPAIAKTGANTVRIVLS
NGVQYTRDDVNSVKNIISLVNQNKMIAVLEVHDATGKDDYASLDAAVNYWISIKDALI
GKEDRVIVNIANEWYGTWNGSAWADGYKQAIPKLRNAGIKNTLIVDAAGWGQCPQSI
VDYGQSVFAADSLKNTIFSIHMYEYAGGTDAIVKSNMENVLNKGLPLIIGEFGGQHTNG
DVDEHAIMRYGQQKGVGWLAWSWYGNNSELSYLDLATGPAGSLTSIGNTIVNDPYGI
KATSKKAGIF.

The amino acid sequence of the PspMan5 mature protein, based on the predicted cleavage of the naturally occurring sequence, is set forth as SEQ ID NO:56:

ATGFYVSGTTLYDSTGKPFVMRGVNHSHTWFKNDLNAAIPAIAKTGANTVRIVLSNGV
QYTRDDVNSVKNIISLVNQNKMIAVLEVHDATGKDDYASLDAAVNYWISIKDALIGKE
DRVIVNIANEWYGTWNGSAWADGYKQAIPKLRNAGIKNTLIVDAAGWGQCPQSIVDY
GQSVFAADSLKNTIFSIHMYEYAGGTDAIVKSNMENVLNKGLPLIIGEFGGQHTNGDVD
EHAIMRYGQQKGVGWLAWSWYGNNSELSYLDLATGPAGSLTSIGNTIVNDPYGIKATS
KKAGIF.

The nucleotide sequence of the synthesized *PspMan9* gene in plasmid p2JM-PspMan9 is set forth as SEQ ID NO: 57 (the gene has an alternative start codon (GTG), the oligonucleotide encoding the three residue addition (AGK) is shown in bold):

GTGAGAAGCAAAAAATTGTGGATCAGCTTGTTGTTTGCGTTAACGTTAATCTTTACG
ATGGCGTTCAGCAACATGAGCGCGCAGGCTGCTGGAAAAGCAACAGGCTTTTATGT
TTCAGGAACAAAACTTTATGATAGCACGGGAAAACCGTTTGTGATGAGAGGCGTTA

ATCACTCACATACATGGTTTAAGAATGATCTGAATGCAGCTATCCCTGCGATTGCGA
AGACAGGCGCAAACACGGTTAGAATTGTTCTGTCAAACGGCGTTCAATATACGAGA
GATGATGTTAATTCAGTCAAGAATATCATTTCACTGGTGAATCAAAATAAGATGATT
GCAGTTCTGGAAGTTCATGATGCTACAGGAAAAGACGATTATGCATCACTGGATGC
AGCAATTAACTATTGGATTTCAATTAAAGATGCACTGATTGGCAAAGAAGATAGAG
TTATTGTGAACATTGCAAATGAATGGTATGGCACATGGAATGGCTCAGCATGGGCA
GATGGATATAAACAAGCTATTCCTAAACTGAGAAATGCGGGCATCAAAAATACGCT
GATCGTGGATGCGGCTGGCTGGGGCCAATATCCGCAATCAATTGTTGATTACGGCC
AGTCAGTTTTTGCAGCAGATTCACTGAAGAACACAGTGTTTAGCATCCATATGTATG
AATATGCAGGCGGCACAGATGCAATGGTTAAAGCTAATATGGAAGGAGTTCTGAAT
AAAGGCCTGCCGCTGATTATTGGAGAATTTGGCGGACAACATACAAATGGCGATGT
TGACGAACTGGCAATTATGAGATATGGCCAACAAAAAGGCGTGGATGGCTGGCAT
GGTCATGGTACGGCAACAACAGCGATCTGTCATATCTTGATCTGGCAACGGACCG
AATGGATCACTGACAACGTTTGGAAATACAGTGGTGAACGATACGAACGGAATTAA
GGCAACGAGCAAGAAGGCGGGAATTTTTCAA.

The amino acid sequence of the *PspMan9* precursor protein expressed from plasmid p2JM-PspMan9 is set forth as SEQ ID NO:58 (the predicted signal sequence is shown in italics, the three residue amino-terminal extension (AGK) is shown in bold):

MRSKKLWISLLFALTLIFTMAFSNMSAQAAGKATGFYVSGTKLYDSTGKPFVMRGVNHSH
TWFKNDLNAAIPAIAKTGANTVRIVLSNGVQYTRDDVNSVKNIISLVNQNKMIAVLEVH
DATGKDDYASLDAAINYWISIKDALIGKEDRVIVNIANEWYGTWNGSAWADGYKQAIP
KLRNAGIKNTLIVDAAGWGQYPQSIVDYGQSVFAADSLKNTVFSIHMYEYAGGTDAM
VKANMEGVLNKGLPLIIGEFGGQHTNGDVDELAIMRYGQQKGVGWLAWSWYGNNSD
LSYLDLATGPNGSLTTFGNTVVNDTNGIKATSKKAGIFQ

The amino acid sequence of the PspMan9 mature protein expressed from p2JM-PspMan9 is set forth as SEQ ID NO:59 (the three residue amino-terminal extension (AGK) based on the predicted cleavage site shown in bold):

AGKATGFYVSGTKLYDSTGKPFVMRGVNHSHTWFKNDLNAAIPAIAKTGANTVRIVLS
NGVQYTRDDVNSVKNIISLVNQNKMIAVLEVHDATGKDDYASLDAAINYWISIKDALIG
KEDRVIVNIANEWYGTWNGSAWADGYKQAIPKLRNAGIKNTLIVDAAGWGQYPQSIV
DYGQSVFAADSLKNTVFSIHMYEYAGGTDAMVKANMEGVLNKGLPLIIGEFGGQHTN
GDVDELAIMRYGQQKGVGWLAWSWYGNNSDLSYLDLATGPNGSLTTFGNTVVNDTN
GIKATSKKAGIFQ.

The amino acid sequence of the PspMan9 mature protein, based on the predicted cleavage of the naturally occurring sequence, is set forth as SEQ ID NO:60:

ATGFYVSGTKLYDSTGKPFVMRGVNHSHTWFKNDLNAAIPAIAKTGANTVRIVLSNGV
QYTRDDVNSVKNIISLVNQNKMIAVLEVHDATGKDDYASLDAAINYWISIKDALIGKED
RVIVNIANEWYGTWNGSAWADGYKQAIPKLRNAGIKNTLIVDAAGWGQYPQSIVDYG
QSVFAADSLKNTVFSIHMYEYAGGTDAMVKANMEGVLNKGLPLIIGEFGGQHTNGDV
DELAIMRYGQQKGVGWLAWSWYGNNSDLSYLDLATGPNGSLTTFGNTVVNDTNGIK
ATSKKAGIFQ.

### EXAMPLE 3

### Purification of mannanases

BciMan1, BciMan4, and PspMan4 proteins were purified via two chromatography steps: anion-exchange and hydrophobic interaction chromatography. The concentrated and desalted crude protein samples were loaded onto a 70 ml Q-Sepharose High Performance column pre-equilibrated with buffer A (Tris-HCl, pH7.5, 20mM). After column washing, the proteins were eluted with a gradient of 0-50% buffer A with 1 M NaCl in 5 column volumes. The target protein was in the flowthrough. Ammonium sulfate was then added to the flowthrough to a final concentration of 0.8-1 M. The solution was loaded onto a Phenyl-Sepharose Fast Flow column pre-equilibrated with 20 mM Tris pH 7.5 with 0.8-1 M ammonium sulfate (buffer B). Gradient elution (0-100% buffer A) in 4 column volumes followed with 3 column volumes step elution (100% buffer A) was performed and the protein of interest was eventually eluted. The purity of the fractions was detected with SDS-PAGE and the results showed that the target protein had been completely purified. The active fractions were pooled and concentrated using 10 kDa Amicon Ultra-15 devices. The sample was above 90% pure and stored in 40% glycerol at -20°C to -80°C until usage.

BciMan3, PpoMan1, PpoMan2 proteins were purified using a three step anion-exchange, hydrophobic interaction chromatography and gel filtration purification strategy. The 700 mL crude broth from the shake flask was concentrated by VIVAFLOW 200 (cutoff 10 kDa) and buffer exchanged into 20 mM Tris-HCl (pH 7.5). The liquid was then loaded onto a 50 mL Q-Sepharose High Performance column which was pre-equilibrated with 20 mM Tris-HCl, pH 7.5 (buffer A). The column was eluted with a linear gradient from 0 to 50% buffer B (buffer A containing 1 M NaCl) in 3 column volumes, followed with 3 column volumes of 100% buffer B. The protein of interest was detected in the gradient elution part and the pure fractions were pooled. Subsequently, 3 M ammonium sulfate solution was added to the active fractions to an ultimate concentration of 1 M, and then the pretreated fraction was loaded onto a 50 mL Phenyl-Sepharose Fast Flow column equilibrated with 20 mM Tris-HCl (pH 7.5) containing 1 M ammonium sulfate. Four column volumes gradient elution (0-100% buffer A) followed with 3 column volumes step elution (100% buffer A) was performed and the relative pure fractions were pooled. The collected fraction was concentrated into 10 mL and loaded onto the HiLoad™ 26/60, Superdex-75 column (1 column volume = 320 mL) pre-equilibrated with 20 mM sodium phosphate buffer containing 0.15 M NaCl (pH 7.0). The pure fractions were pooled and concentrated using 10 kDa Amicon Ultra-15 devices. The purified sample was stored in 20 mM sodium phosphate buffer (pH 7.0) with 40% glycerol at -20°C until usage.

To purify PspMan5 and PspMan9 proteins, ammonium sulfate was added to the crude samples to a final concentration of 1 M. The solution was applied to a HiPrep™ 16/10 Phenyl FF column pre-equilibrated with 20 mM Tris (pH 8.5), 1M ammonium sulfate (buffer A). The target protein was eluted from the column with a linear salt gradient from 1 to 0 M ammonium sulfate. The active fractions were pooled, concentrated and buffer exchanged into 20 mM Tris (pH8.5) using a VivaFlow 200 ultra filtration device (Sartorius Stedim). The resulting solution was applied to a HiPrep™ Q XL 16/10 column pre-equilibrated with 20 mM Tris (pH8.5). The target protein was eluted from the column with a linear salt gradient from 0 to 0.6 M NaCl in buffer A. The resulting active protein fractions were then pooled and concentrated via 10 kDa Amicon Ultra devices, and stored in 40% glycerol at -20°C until usage.

PpaMan2 was purified using hydrophobic interaction chromatography and cation exchange chromatography. 800 mL crude broth was concentrated by VIVAFLOW 200 (cutoff 10 kDa) and ammonium sulfate was added to a final concentration of 0.8 M. The sample was then loaded onto a 50 mL Phenyl-Sepharose High Performance column which was pre-equilibrated with buffer A (20 mM sodium acetate containing 0.8 M ammonium sulfate, pH 5.5). The column was treated with a gradient elution of 0-100% buffer B (20 mM sodium acetate at pH 5.5) in 5 column volumes, followed with 3 column volumes of 100% buffer B. The relative pure active fractions were pooled and buffer exchanged into buffer B. The solution turned to be cloudy and was dispensed to 50 mL tubes, centrifuged at 3800 rpm for 20 min. The supernatant and the precipitant were collected. According to the SDS-PAGE gel analysis results, the target protein was identified in the supernatant which was then subjected onto an SP-Sepharose Fast Flow column, a linear gradient elution with 0-50% buffer C (20 mM sodium acetate containing 1M sodium chloride) in 4 column volumes followed with 3 column volumes' step elution (100% buffer C) was performed. The purity of the each fraction was evaluated with SDS-PAGE. Pure fractions were pooled and concentrated using 10 kDa Amicon Ultra-15 devices. The purified sample was stored in 20 mM sodium acetate buffer (pH 5.5) with 40% glycerol at -20°C.

### EXAMPLE 4

### Activity of mannanases

The beta 1-4 mannanase activity of the mannanases was measured using 0.5% locust bean gum galactomannan (Sigma G0753) and konjac glucomannan (Megazyme P-GLCML) as substrates. The assays were performed at 50°C for 10 minutes using two different buffer systems: 50 mM sodium acetatepH 5, and 50 mM HEPES pH 8.2. In both sets of assays, the released reducing sugar was quantified using a PAHBAH (p-Hydroxy benzoic acid hydrazide) assay (Lever, Anal Biochem, 47:248, 1972). A standard curve using mannose was created for each buffer, and was used to calculate enzyme activity units. In this assay, one mannanase unit is defined as the amount of enzyme required to generate 1 micromole of mannose reducing sugar equivalent per minute. The specific activities of the mannanases are summarized in Table 1.

| **Table1. Specific activities (U/mg) of mannanases at pH 5.0 and pH 8.2 using different substrates** | | | | |
|---|---|---|---|---|
| | pH5.0 | | pH8.2 | |
| Mannanase | Locust bean gum | Konjac glucomannan | Locust bean gum | Konjac glucomannan |
| BciMan1 | 25 | 70 | 328 | 363 |
| BciMan3 | 17 | 35 | 377 | 414 |
| BciMan4 | 160 | 221 | 590 | 681 |
| PpaMan2 | 94 | 162 | 419 | 454 |
| PpoMan1 | 148 | 205 | 616 | 601 |
| PpoMan2 | 62 | 108 | 618 | 615 |
| PspMan4 | 112 | 159 | 520 | 624 |
| PspMan5 | 105 | 136 | 116 | 152 |
| PspMan9 | 145 | 251 | 518 | 628 |

### EXAMPLE 5

### pH Profile of Mannanases

The pH profile of mannanases was determined by assaying for mannanase activity at various pH values ranging from 2 to 9 at 50°C for 10 min with locust bean gum as the substrate. The proteins were diluted in 0.005% Tween-80 to an appropriate concentration based on the dose response curve. The substrate solutions, buffered using sodium citrate/sodium phosphate buffers of different pH units, were pre-incubated in the thermomixer at 50°C for 5 min. The reaction was initiated by the addition of mannanases. The mixture was incubated at 50°C for 10 min, and then the reaction was stopped by transferring 10 microliters of reaction mixture to a 96-well PCR plate containing 100 microliters of the PAHBAH solution. The PCR plate was heated at 95°C for 5 minutes in a Bio-Rad DNA Engine. Then 100 microliters were transferred from each well to a new 96-well plate. The release of reducing sugars from the substrate was quantified by measuring the optical density at 410 nm in a spectrophotometer. Enzyme activity at each pH is reported as relative activity where the activity at the pH optimum was set to 100%. The pH optimum and range of ≥ 70% activity for the mannanases under these assay conditions is shown in Table 2.

| **Table 2. Optimal pH and pH range of activity for mannanases** | | |
|---|---|---|
| **Mannanase** | **pH Optimum** | **pH range of ≥ 70% activity** |
| BciMan1 | 7.0 | 6.0-8.5 |
| BciMan3 | 7.0 | 6.5-8.5 |
| BciMan4 | 7.0 | 5.5-8.5 |
| PpaMan2 | 8.0 | 5.5-9.0* |
| PpoMan1 | 7.0 | 5.5-8.5 |
| PpoMan2 | 7.0 | 6.0-8.5 |
| PspMan4 | 7.5 | 5.5-9.0 |
| PspMan5 | 6.0 | 4.5-7.5 |
| PspMan9 | 6.0-8.0 | 5.5-9.0* |

| | | |
|---|---|---|
| *PpaMan2 and PspMan9 showed mannanase activity above pH9 | | |

### EXAMPLE 6

### Temperature Profile of mannanases

The temperature profile of mannanases was determined by assaying for mannanase activity with locust bean gum as the substrate at various temperatures for 10 min in 50 mM sodium citrate buffer at pH 6.0. The activity is reported as relative activity where the activity at the temperature optimum was set to 100%. The temperature optimum and temperature range of ≥ 70% activity for the mannanases under these assay conditions is shown in Table 3.

| **Table 3. Optimal temperature and temperature range of activity for mannanases.** | | |
|---|---|---|
| **Mannanase** | **Temperature Optimum (°C)** | **Temperature range of ≥ 70% activity (°C)** |
| BciMan1 | 60-65 | 45-70 |
| BciMan3 | 55 | 40-65 |
| BciMan4 | 55 | 50-60 |
| PpaMan2 | 60 | 54-63 |
| PpoMan1 | 55-58 | 45-65 |
| PpoMan2 | 50-55 | <35-60 |
| PspMan4 | 55 | 47-60 |
| PspMan5 | 50 | 40-55 |
| PspMan9 | 58 | 48-62 |

### EXAMPLE 7

### Thermostability of Paenibacillus and Bacillus mannanases

The temperature stability of *Paenibacillus* and *Bacillus* mannanases was determined in 50 mM sodium citrate buffer at pH 6.0. The enzyme was incubated at temperatures ranging from 40°C to 90°C for 2 hours in a thermocycler. The remaining enzyme activity was measured using locust bean gum as the substrate. The activity of the sample kept on ice was defined as 100% activity. The temperatures at which the enzymes retain 50% activity (T₅₀) after a 2-hour incubation period under these assay conditions are shown in Table 4.

| **Table 4: Thermal Stability of Mannanases.** | |
|---|---|
| **Mannanase** | **T₅₀ (°C)** |
| PspMan4 | 57 |
| BciMan1 | 53 |
| BciMan3 | 47 |
| BciMan4 | 53 |
| PpoMan1 | 54 |
| PpoMan2 | 52 |
| PspMan5 | 53 |
| PspMan9 | 54 |
| PpaMan2 | 58 |

### EXAMPLE 8

### Cleaning performance of mannanases

Cleaning performance was measured using a high throughput assay developed to measure galactomannan removal from technical soils. The assay measures the release of locust bean gum from the technical soils containing locust bean gum. The BCA reagent measures the reducing ends of oligosaccharides released in the presence of mannanase enzyme, as compared to a blank (no enzyme) control. This measurement correlates with the cleaning performance for the enzymes. As the mannanases hydrolyze galactomannans, oligosaccharides of varying lengths with new reducing ends are released from the cotton swatch. The bicinchoninic acid in the BCA reagent then allows for the highly sensitive colorimetric detection as Cu¹⁺ is formed by the reduction of Cu²⁺.

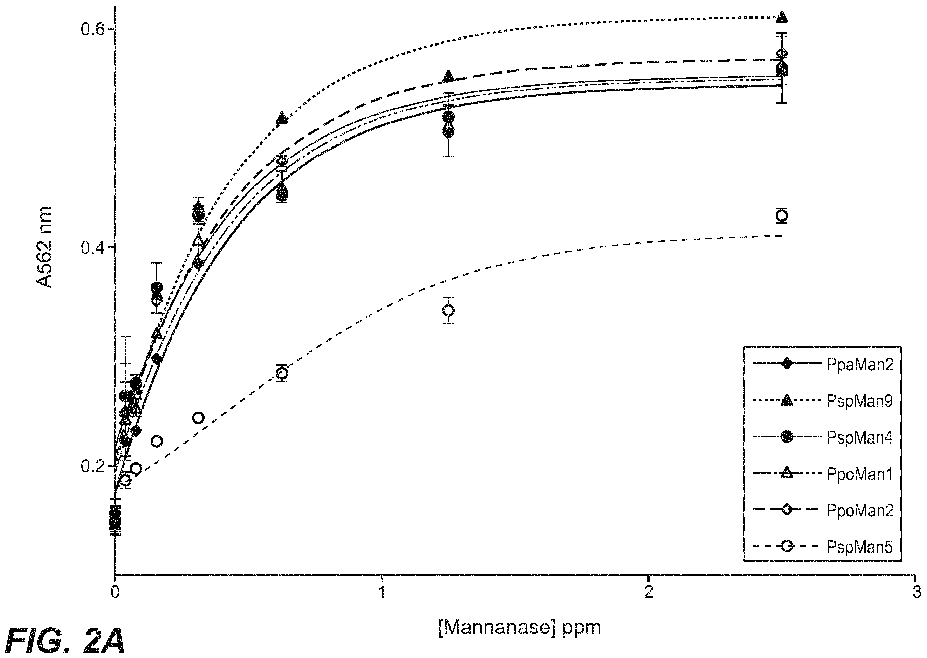

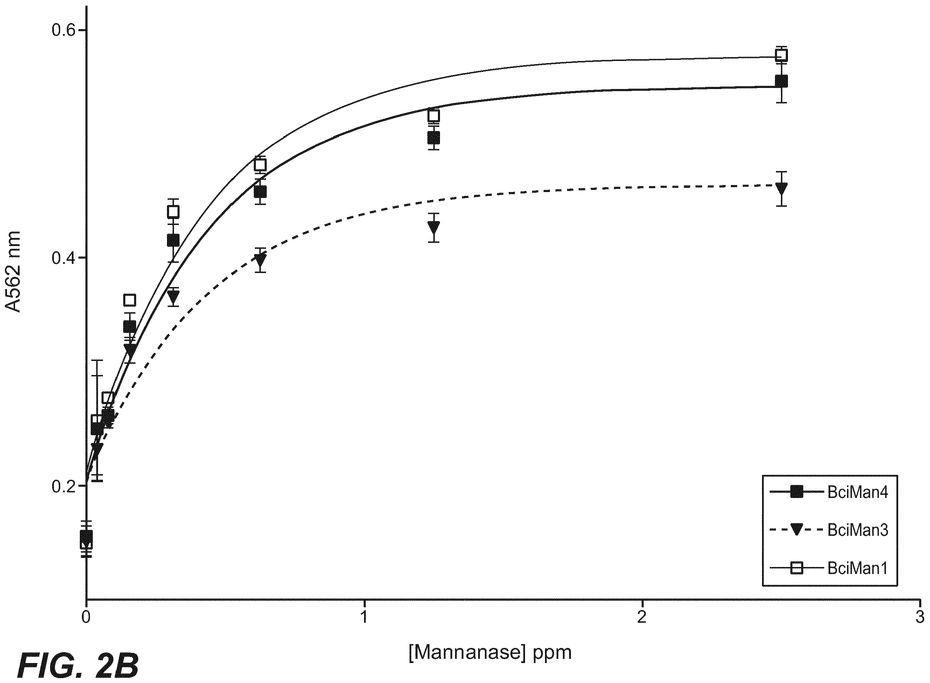

Two 5.5 cm diameter locust bean gum CS-73 microswatches (CFT, Vlaardingen, Holland) were placed into each well of a flat-bottom, non-binding 96-well assay plate. Enzymes were diluted into 50 mM MOPS, pH 7.2, 0.005% Tween-80. Diluted enzyme and microswatch assay buffer (25 mM HEPES, pH 8, 2 mM CaCl₂, 0.005% Tween-80) was added into each well for a combined volume of 100 microliters. Plates were sealed and incubated in an iEMS machine at 25°C with agitation at 1150 rpm for 20 minutes. To measure the new reducing ends produced, 100 microliters of the BCA assay reagent (Thermo Scientific Pierce, Rockford, IL) was pipetted into each well of a fresh PCR plate. 15 microliters of wash liquor was removed from each well of the microswatch assay plates after the incubation period was completed, and transferred to the plate containing the BCA reagent. Plates were sealed and incubated in a PCR machine at 95°C for 2-3 minutes. After the plate cooled to 25°C, 100 microliters of the supernatant was transferred to a fresh microtiter flat-bottom assay plate and absorbance was measured at 562 nm in a spectrophotometer. Figures 2A and 2B show the response of the mannanases in this assay. All mannanases tested exhibited galactomannan removal activity.

### EXAMPLE 9

### Identification of Homologous mannanases

Related proteins were identified by a BLAST search (Altschul et al., Nucleic Acids Res, 25:3389-402, 1997) against the NCBI non-redundant protein database using the mature protein amino acid sequence of PpaMan2 (SEQ ID NO:40), PspMan4 (SEQ ID NO:52), and PspMan9 (SEQ ID NO:60) and a subset of the results are shown on Tables 5A, 6A, and 7A, respectively. A similar search was run against the Genome Quest Patent database with search parameters set to default values using the mature protein amino acid sequence of PpaMan2 (SEQ ID NO:40), PspMan4 (SEQ ID NO:52), and PspMan9 (SEQ ID NO:60) as the query sequences, and a subset of the results are shown in Tables 5B, 6B, and 7B, respectively. Percent identity (PID) for both search sets is defined as the number of identical residues divided by the number of aligned residues in the pairwise alignment. The column labeled "Sequence Length" refers to the length (in amino acids) of the protein sequences associated with the listed Accession Nos., while the column labeled "Aligned Length" refers to the length (in amino acids) of the aligned protein sequence used for the PID calculation.

| **Table 5A: List of sequences with percent identity to PpaMan2 protein identified from the NCBI non-redundant protein database** | | | | |
|---|---|---|---|---|
| **Accession #** | **PID** | **Organism** | **Sequence Length** | **Alignment Length** |
| WP_024633848.1 | 95 | *Paenibacillus sp. MAEPY2]* | 326 | 296 |
| ETT37549.1 | 94 | *Paenibacillus sp. FSL R5-192* | 326 | 296 |
| WP_017688745.1 | 93 | *Paenibacillus sp. PAMC 26794* | 326 | 296 |
| ACU30843.1 | 93 | *Paenibacillus sp. A1* | 319 | 296 |
| AAX87003.1 | 91 | *B. circulans* | 326 | 296 |
| WP_017813111.1 | 88 | *Paenibacillus sp. A9* | 327 | 296 |
| AEX60762.1 | 86 | *Paenibacillus sp. CH-3* | 327 | 296 |
| YP_003868989.1/WP_013308634.1 | 81 | *Paenibacillus polymyxa E681* | 327 | 296 |
| WP_016819573.1 | 81 | *Paenibacillus polymyxa* | 327 | 296 |
| WP_017427981.1 | 81 | *Paenibacillus sp. ICGEB2008* | 327 | 296 |
| YP_003944884.1/WP_013369280.1 | 80 | *Paenibacillus polymyxa* SC2 | 327 | 296 |
| WP_009593769.1 | 80 | *Paenibacillus sp. HGF5* | 326 | 296 |
| AAX87002.1 | 81 | *B. circulans* | 327 | 296 |
| BAA25878.1 | 71 | *B. circulans* | 516 | 297 |
| WP_019912481.1 | 66 | *Paenibacillus sp. FEW567* | 547 | 294 |
| YP_006190599.1/WP_ 014651264.1 | 66 | *Paenibacillus mucilaginosus K02* | 475 | 296 |

| **Table 5B: List of sequences with percent identity to PpaMan2 protein identified from the Genome Quest database** | | | | |
|---|---|---|---|---|
| **Patent ID #** | **PID** | **Organism** | **Sequence Length** | **Alignment Length** |
| EP2260105-0418 | 91.6 | *B. circulans* | 326 | 296 |
| EP2260105-0427 | 81.1 | *B. circulans* | 327 | 296 |
| CN100410380-0004, | 81.1 | *B. circulansB48* | 296 | 296 |
| CN1904052-0003 | 80.4 | *B. circulansB48* | 327 | 296 |
| US20090325240-0477 | 71.7 | *B. circulans* | 516 | 297 |
| US20140199705-0388 | 68.4 | *empty* | 490 | 291 |
| WO2014100018-0002 | 66 | *Bacillus lentus* | 299 | 297 |
| WO2015022428-0015 | 63.1 | *Bacillus sp.* | 309 | 290 |
| US20030203466-0004 | 62.8 | *Bacillus sp.* | 490 | 290 |
| EP2260105-0445 | 62.1 | *B. circulans* | 493 | 290 |
| EP2260105-0429 | 61.8 | *Bacillus sp. JAMB-602* | 490 | 296 |
| US20030215812-0002 | 60.6 | *Bacillus sp.* | 493 | 297 |
| US20030203466-0008 | 60.6 | *Bacillus agaradhaerens* | 468 | 297 |
| US20030215812-0002 | 60.6 | *Bacillus sp* | 493 | 297 |

| **Table 6A: List of sequences with percent identity to PspMan4 protein identified from the NCBI non-redundant protein database** | | | | |
|---|---|---|---|---|
| **Accession #** | **PID** | **Organism** | **Sequence Length** | **Alignment Length** |
| ACU30843.1 | 100 | *Paenibacillus sp. A1* | 319 | 297 |
| ETT37549.1 | 99 | *Paenibacillus sp. FSL R5-192* | 326 | 296 |
| WP_017688745.1 | 99 | *Paenibacillus sp. PAMC 26794* | 326 | 296 |
| AAX87003.1 | 94 | *B. circulans* | 326 | 296 |
| WP_024633848.1 | 94 | *Paenibacillus sp. MAEPY2* | 326 | 296 |
| WP_017813111.1 | 89 | *Paenibacillus sp. A9* | 327 | 296 |
| AEX60762.1 | 87 | *Paenibacillus sp. CH-3* | 327 | 296 |
| YP_003868989.1/WP_013308634.1 | 81 | *Paenibacillus polymyxa E681* | 327 | 296 |
| YP_003944884.1/WP_013369280.1 | 80 | *Paenibacillus polymyxa SC2* | 327 | 296 |
| WP_016819573.1 | 80 | *Paenibacillus polymyxa* | 327 | 296 |
| WP_017427981.1 | 80 | *Paenibacillus sp. ICGEB2008* | 327 | 296 |
| AAX87002.1 | 79 | *B. circulans* | 327 | 296 |
| WP_009593769.1 | 78 | *Paenibacillus sp. HGF5* | 326 | 296 |
| BAA25878.1 | 72 | *B. circulans* | 516 | 297 |
| YP_006190599.1/WP_014651264.1 | 67 | *Paenibacillus mucilaginosus K02* | 475 | 296 |
| WP_019912481.1 | 65 | *Paenibacillus sp. FEW567* | 547 | 294 |
| BAD99527.1 | 62 | *Bacillus sp. JAMB-602* | 490 | 296 |
| AGU71466.1 | 64 | *Bacillus nealsonii* | 353 | 297 |
| WP_017426982.1 | 63 | *Paenibacillus sp. ICGEB2008* | 796 | 296 |
| AAS48170.1 | 61 | *Bacillus circulans* | 493 | 296 |
| AAT06599.1 | 60 | *Bacillus sp. N16-5* | 493 | 297 |
| WP_018887458.1 | 63 | *Paenibacillus massiliensis* | 592 | 294 |
| YP_006844719.1 | 60 | *Amphibacillus xylanus NBRC 15112* | 497 | 297 |

| **Table 6B: List of sequences with percent identity to PspMan4 protein identified from the Genome Quest database** | | | | |
|---|---|---|---|---|
| **Patent ID #** | **PID** | **Organism** | **Sequence Length** | **Alignment Length** |
| EP2260105-0418 | 94.3 | *B. circulans* | 326 | 296 |
| CN100410380-0004 | 79.1 | *B. circulansB48* | 296 | 296 |
| EP2260105-0427 | 79.1 | *B. circulans* | 327 | 296 |
| CN1904052-0003 | 78.4 | *B. circulansB48* | 327 | 296 |
| US20090325240-0477 | 72.1 | *B. circulans* | 516 | 297 |
| EP2409981-0388 | 67.7 | *empty* | 490 | 297 |
| WO2014100018-0002 | 66.3 | *Bacillus lentus* | 299 | 297 |
| WO2015022428-001 5 | 62.5 | *Bacillus sp.* | 309 | 296 |
| JP2006087401-0006 | 62.5 | *Bacillus sp.* | 458 | 296 |
| US20090325240-0429 | 62.5 | *Bacillus sp. JAMB-602* | 490 | 296 |
| EP2284272-0004 | 62.2 | *Bacillus sp.* | 476 | 296 |
| EP2287318-0002 | 62.2 | *Bacillus sp. I633* | 490 | 296 |
| WO2014124927-0018 | 62.2 | *Bacillus sp. I633* | 490 | 296 |
| US20090325240-0445 | 61.5 | *B. circulans* | 493 | 296 |
| US20030203466-0008 | 60.9 | *Bacillus agaradhaerens* | 468 | 297 |
| US6964943-0002 | 60.9 | *Bacillus sp.* | 493 | 297 |

| **Table 7A: List of sequences with percent identity to PspMan9 protein identified from the NCBI non-redundant protein database** | | | | |
|---|---|---|---|---|
| **Accession #** | **PID** | **Organism** | **Sequence Length** | **Alignment Length** |
| AEX60762.1 | 94 | *Paenibacillus sp. CH-3* | 327 | 296 |
| WP_017813111.1 | 89 | *Paenibacillus sp. A9* | 327 | 296 |
| ACU30843.1 | 88 | *Paenibacillus sp. A1* | 319 | 297 |
| WP_024633848.1 | 88 | *Paenibacillus sp. MAEPY2]* | 326 | 296 |
| ETT37549.1 | 88 | *Paenibacillus sp. FSL R5-192* | 326 | 296 |
| WP_017688745.1 | 87 | *Paenibacillus sp. PAMC 26794* | 326 | 296 |
| AAX87003.1 | 86 | *B. circulans* | 326 | 296 |
| YP_003868989.1/WP_013308634.1 | 83 | *Paenibacillus polymyxa E681* | 327 | 296 |
| WP_016819573.1 | 83 | *Paenibacillus polymyxa* | 327 | 296 |
| WP_017427981.1 | 82 | *Paenibacillus sp. ICGEB2008* | 327 | 296 |
| YP_003944884.1/WP_013369280.1 | 82 | *Paenibacillus polymyxa SC2* | 327 | 296 |
| AAX87002.1 | 80 | *B. circulans* | 327 | 296 |
| WP_009593769.1 | 79 | *Paenibacillus sp. HGF5* | 326 | 296 |
| BAA25878.1 | 73 | *B. circulans* | 516 | 297 |
| YP_006190599.1/ WP_ 014651264.1 | 68 | *Paenibacillus mucilaginosus K02* | 475 | 296 |
| WP_019912481.1 | 66 | *Paenibacillus sp. HW567* | 547 | 294 |
| AGU71466.1 | 68 | *B. nealsonii* | 353 | 297 |
| WP_018887458.1 | 65 | *Paenibacillus massiliensis* | 592 | 294 |
| WP_019687326.1 | 64 | *Paenibacillus polymyxa* | 796 | 296 |
| WP_006037399.1 | 64 | *Paenibacillus curdlanolyticus* | 707 | 297 |

| **Table 7B: List of sequences with percent identity to PspMan9 protein identified from the Genome Quest database** | | | | |
|---|---|---|---|---|
| **Patent ID #** | **PID** | **Organism** | **Sequence Length** | **Alignment Length** |
| EP2260105-0418 | 86.2 | *B. circulans* | 326 | 296 |
| CN100410380-0004 | 80.4 | *B. circulansB48* | 296 | 296 |
| EP2260105-0427 | 80.4 | *B. circulans* | 327 | 296 |
| CN1904052-0003 | 79.7 | *B. circulansB48* | 327 | 296 |
| EP2260105-0477 | 73.4 | *B. circulans* | 516 | 297 |
| US20140199705-0388 | 68.4 | *empty* | 490 | 297 |
| WO2014100018-0002 | 68 | *Bacillus lentus* | 299 | 297 |
| JP2006087401-0001 | 62.8 | *Bacillus sp.* | 458 | 296 |
| WO2015022428-0015 | 62.5 | *Bacillus sp.* | 309 | 296 |
| US20030203466-0004 | 62.2 | *Bacillus sp.* | 490 | 296 |
| JP2006087401-0005 | 62.8 | *Bacillus sp.* | 490 | 296 |
| US20090325240-0429 | 62.8 | *Bacillus sp. JAMB-602* | 490 | 296 |
| EP2287318-0004 | 62.2 | *Bacillus sp.* | 476 | 296 |
| EP2260105-0445 | 61.5 | *B. circulans* | 493 | 296 |

### EXAMPLE 10

### Analysis of Homologous Sequences

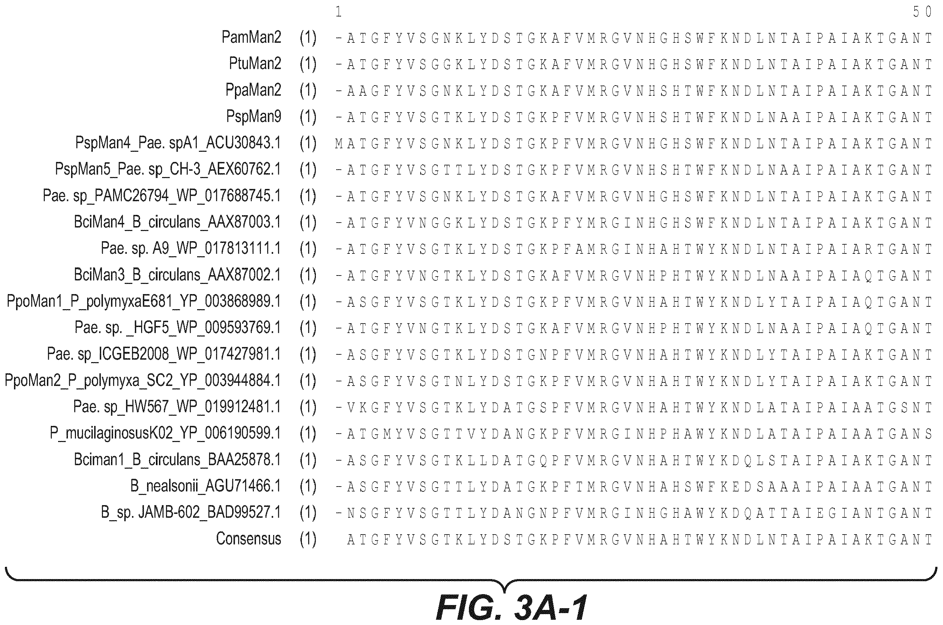

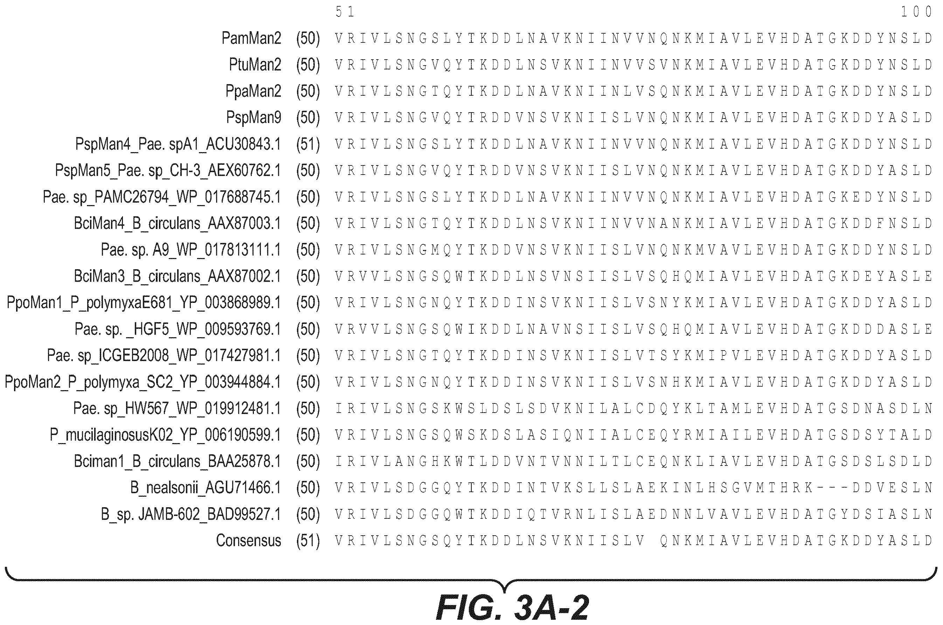

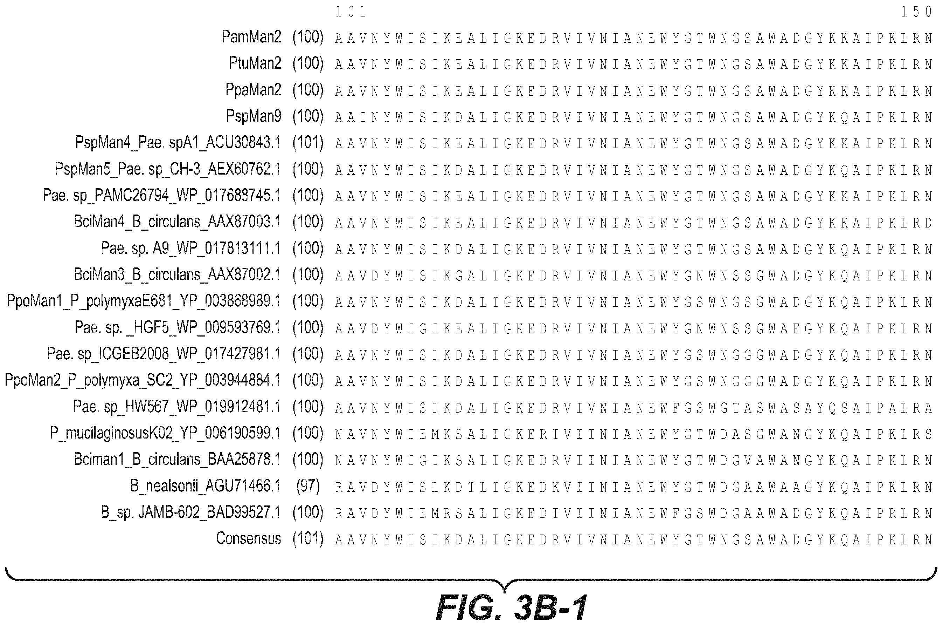

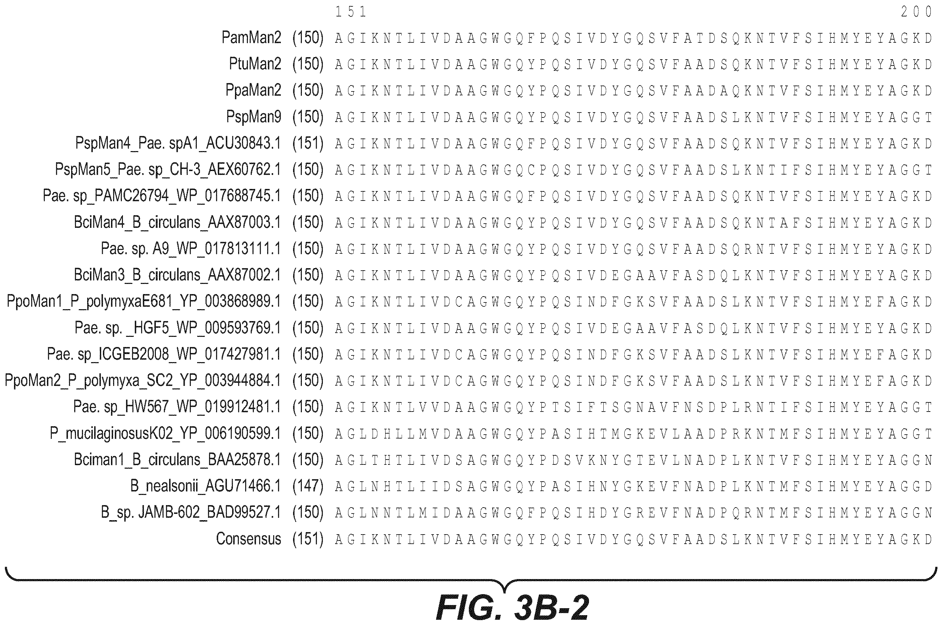

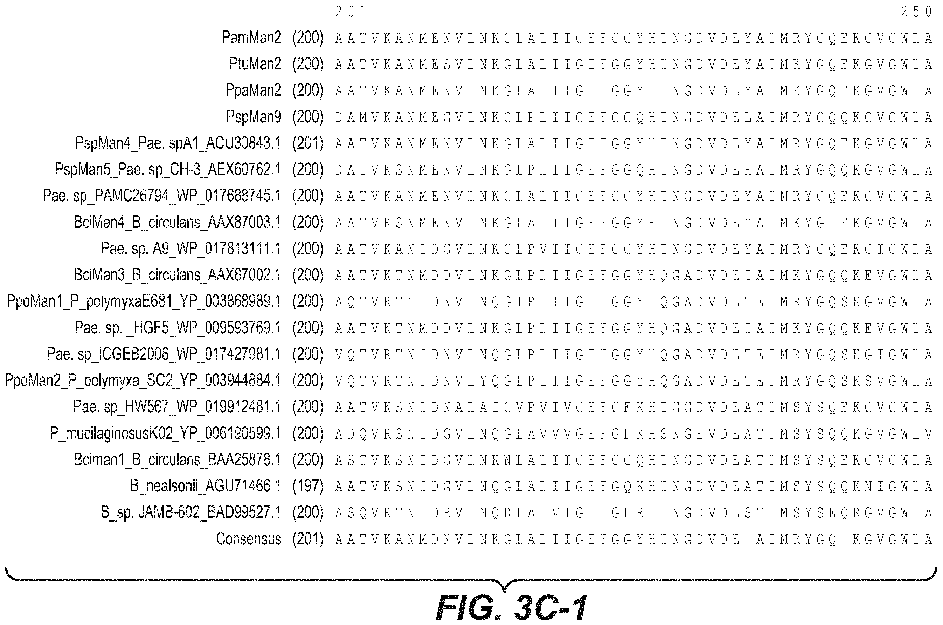

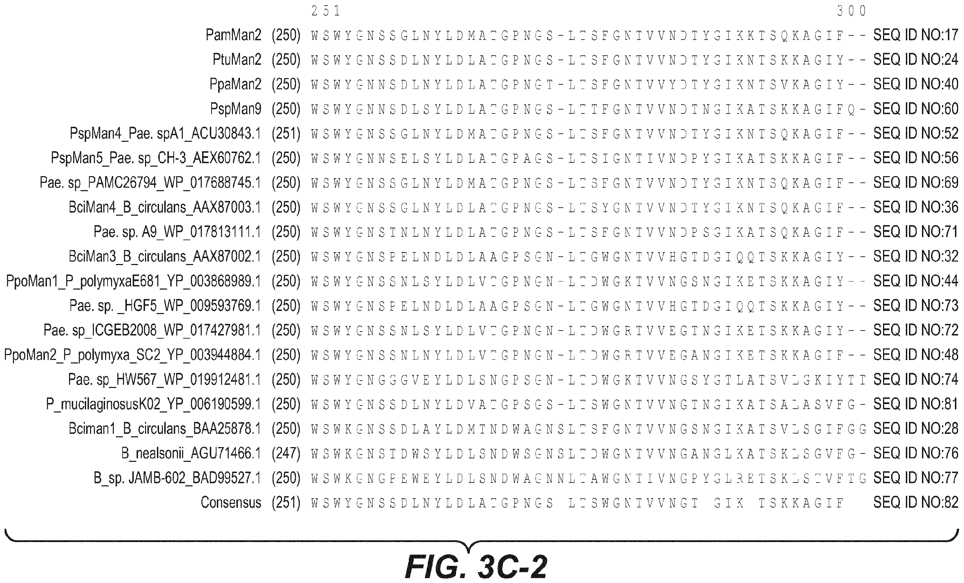

An alignment of the amino acid sequences of the mature BciMan1 (SEQ ID NO:28), BciMan3 (SEQ ID NO:32), BciMan4 (SEQ ID NO:36), PamMan2 (SEQ ID NO:17), PpaMan2 (SEQ ID NO:40), PpoMan1 (SEQ ID NO:44), PpoMan2 (SEQ ID NO:48), PspMan4 (SEQ ID NO:52), PspMan5 (SEQ ID NO:56), PspMan9 (SEQ ID NO:60), and PtuMan2 (SEQ ID NO:24) mannanases with some of the sequences of the mature forms of mannanases from Tables 5A, 6A, and 7A (identified from NCBI searches) is shown in Figure 3. The full-length, untrimmed sequences were aligned using CLUSTALW software (Thompson et al., Nucleic Acids Research, 22:4673-4680, 1994) with the default parameters, wherein Figure 3 displays the alignment of amino acids 1-300 and not the alignment of the entire full-length, untrimmed sequences.

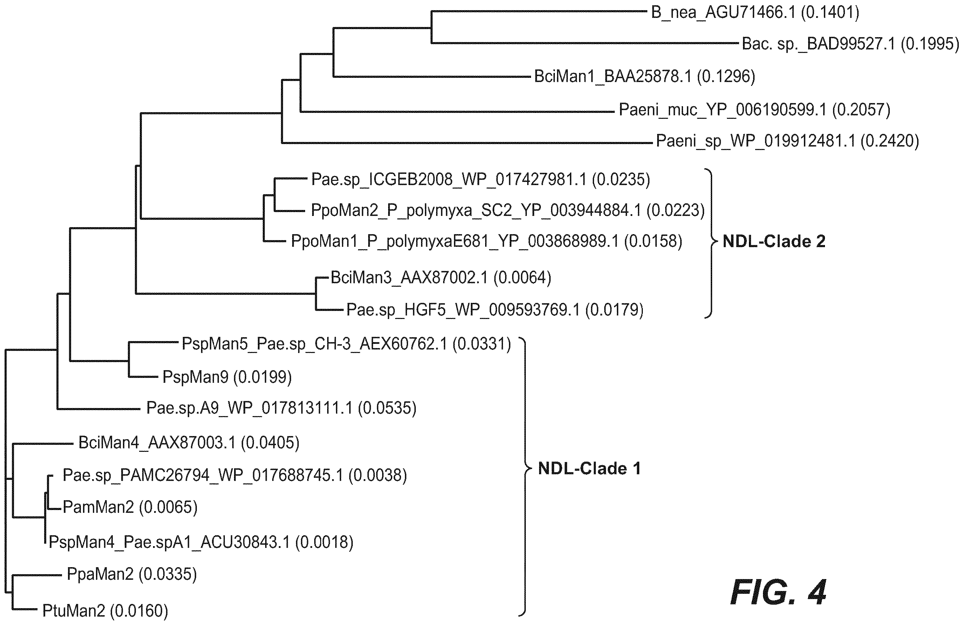

A phylogenetic tree for amino acid sequences of the mannanases aligned in Figure 3 was built, and is shown on Figure 4. The full-length, untrimmed sequences were entered in the Vector NTI Advance suite and a Guide Tree was created using the Neighbor Joining (NJ) method (Saitou and Nei, Mol Biol Evol, 4:406-425, 1987). The tree construction was calculated using the following parameters: Kimura's correction for sequence distance and ignoring positions with gaps. AlignX displays the calculated distance values in parenthesis following the molecule name displayed on the tree shown in Figure 4.

### EXAMPLE 11

### Unique features of the NDL-Clade mannanases

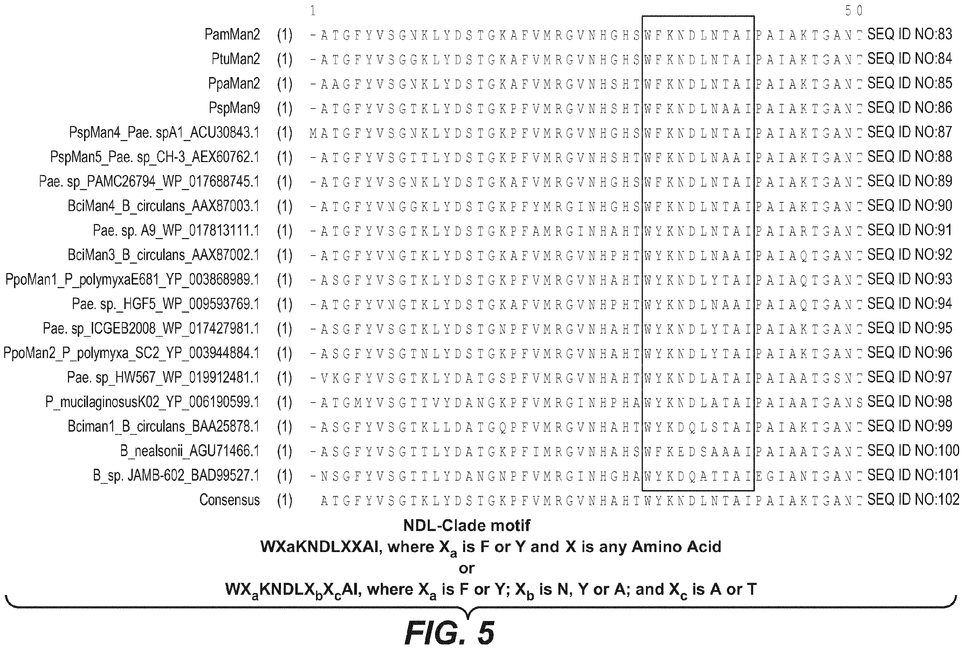

When the mannanases described in Example 10 were aligned common features were shared among BciMan3, BciMan4, PamMan2, PpaMan2, PpoMan1, PpoMan2, PspMan4, PspMan5, PspMan9, and PtuMan2 mannanases. In one case, there is a common pattern of conserved amino acids between residues Trp30 and Ile39, wherein the amino acid positions of the polypeptide are numbered by correspondence with the amino sequence set forth in SEQ ID NO:32. The NDL mannanases share features to create a clade, subsequently termed NDL-Clade, where the term NDL derives from the complete conserved residues NDL near the N-terminus (Asn-Asp-Leu 33-35). The numbering of residues for the mannanases shown is the consecutive linear sequence and are numbered by correspondence with the amino acid sequence set forth in SEQ ID NO:32. The pattern of conserved amino acids related to the NDL-Clade is highlighted in Figure 5, and can be described as WXₐKNDLXXAI, where where Xₐ is F or Y and X is any amino acid; WXₐKNDLXbX_{c}AI, where Xₐ is F or Y, X_{b} is N, Y or A, and X_{c} is A or T; or WF/YKNDLX₁T/AAI, where X₁ is N, Y or A.

The phylogenetic tree described in Example 10 shows a differentiation between the NDL-Clade mannanases and other mannanases. The clade further differentiates into NDL-Clade 1 and NDL-Clade 2 where NDL-Clade 1 includes PtuMan2, PamMan2, PspMan4, BciMan4, PpaMan2, PspMan9 and PspMan5 while NDL-Clade 2 includes BciMan3, PpoMan2 and PpoMan1.

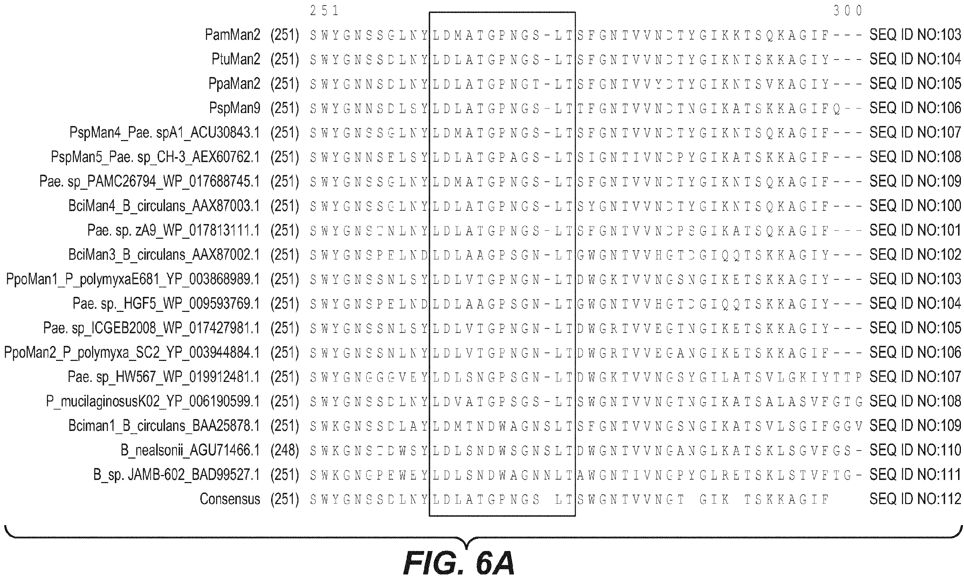

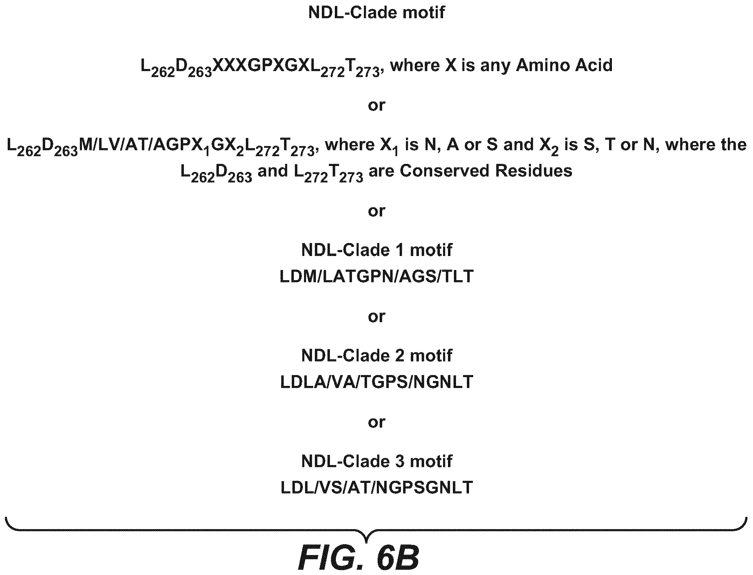

All members of the NDL-Clade have a conserved motif with the key feature of a deletion which is not present in the *Bacillus sp. JAMB-602* and other reference mannanase sequences (hereinafter the "deletion motif"). The deletion motif starts at position 262 in the conserved linear sequence of the amino acid sequences set forth in Figure 6 and includes the sequence LDXXXGPXGXLT, where X is any amino acid or LDM/LV/AT/AGPX₁GX₂LT, where X₁ is N, A or S and X₂ is S, T or N. The sequence further differentiates into LDM/LATGPN/AGS/TLT for NDL-Clade 1 mannanases; LDLA/VA/TGPS/NGNLT for NDL-Clade 2 mannanases; and LDL/VS/AT/NGPSGNLT for NDL-Clade 3 mannanases. All members of the NDL-Clade have a conserved deletion motif not seen in the *Bacillus sp. JAMB-602_*BAD99527.1, *B_nealsonii_*AGU71466.1, and Bciman1_*B*_*circulans*_BAA25878.1 mannanase sequences. The NDL-Clade deletion motif (i.e., LDM/LV/AT/AGPX₁GX₂LT, where X₁ is N, A or S and X₂ is S, T or N) set forth in Figure 6 occurs between the conserved residues Leu262-Asp263 (LD) and Leu272-Thr273 (LT).

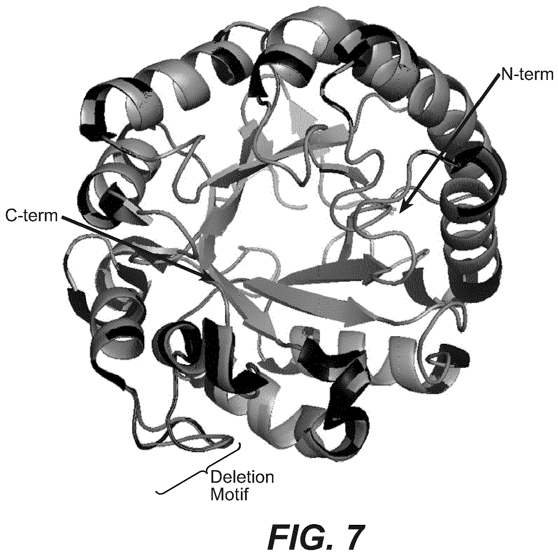

The closest related structure to the NDL-Clade mannanases is that from *Bacillus sp. JAMB-602* (1WKY.pdb) and thus this will be used as a reference to understand the probable consequences of the differentiating characteristics of the NDL-Clade mannanases. Figure 7 shows the structure of *Bacillus sp. JAMB-602* (black) and models of the NDL-Clade mannanases PspMan4, PspMan9 and PpaMan2 (gray). The structures of PspMan4, PspMan9 and PpaMan2 were modelled using the "align" option in the Molecular Operating Environment (MOE) software (Chemical Computing Group, Montreal, Quebec, Canada) to look for structural similarities. The alignment applies conserved structural motifs as an additional guide to conventional sequence alignment. This alignment was performed using standard program defaults present in the 2012.10 distribution of MOE. The deletion motif segment is designated with an arrow. This deletion motif is located in a loop in the structure in the C-terminus. The C-terminal region of the *Bacillus sp. JAMB-602* mannanase is thought to be important to understanding how these mannanases interact in alkaline environments (Akita et al., Acta Cryst, 60:1490-1492, 2004). It is postulated that the deletion impacts the structure, length and flexibility of this loop which then impacts the activity and performance of the NDL-Clade mannanases.

## Claims

1. A cleaning composition comprising a surfactant and an endo-β-mannanase polypeptide comprising an amino acid sequence having at least 90% identity to an amino acid sequence selected from SEQ ID NO: 8, 10, 12, 42, 43, 44, 46, 47, 48, 50, 51 and 52, or an active fragment thereof, wherein said polypeptide or active fragment thereof has mannanase activity in the presence of a surfactant.

2. The cleaning composition of claim 1, wherein the endo-β-mannanase polypeptide or active fragment thereof contains Asn33-Asp-34-Leu35, wherein the amino acid positions of the polypeptide are numbered by correspondence with the amino sequence set forth in SEQ ID NO:32 and are based on the conserved linear sequence numbering.

3. The cleaning composition of claim 1 or claim 2, wherein the endo-β-mannanase polypeptide or active fragment thereof further comprises a WXaKNDLXXAI motif at positions 30-38, wherein Xₐ is F or Y and X is any amino acid, wherein the amino acid positions of the polypeptide are numbered by correspondence with the amino sequence set forth in SEQ ID NO:32 and are based on the conserved linear sequence numbering.

4. The cleaning composition of any preceding claim, wherein the endo-β-mannanase polypeptide or active fragment thereof further comprises a WXₐKNDLX_{b}X_{c}AI motif at positions 30-38, wherein Xₐ is F or Y, X_{b} is N, Y or A, and X_{c} is A or T, wherein the amino acid positions of the polypeptide are numbered by correspondence with the amino sequence set forth in SEQ ID NO:32 and are based on the conserved linear sequence numbering.

5. The cleaning composition of any preceding claim, wherein the endo-β-mannanase polypeptide or active fragment thereof further comprises a L₂₆₂D₂₆₃XXXGPXGXL₂₇₂T₂₇₃, motif at positions 262-273, where X is any amino acid and wherein the amino acid positions of the polypeptide are numbered by correspondence with the amino sequence set forth in SEQ ID NO:32 and are based on the conserved linear sequence numbering.

6. The cleaning composition of any preceding claim, wherein the endo-β-mannanase polypeptide or active fragment thereof further comprises a L₂₆₂D₂₆₃M/LV/AT/AGPX₁GX₂L₂₇₂T₂₇₃ motif at positions 262-273, where X₁ is N, A or S and X₂ is S, T or N, and wherein the amino acid positions of the polypeptide are numbered by correspondence with the amino sequence set forth in SEQ ID NO:32 and are based on the conserved linear sequence numbering.

7. The cleaning composition of any preceding claim, wherein the endo-β-mannanase polypeptide is an NDL-Clade-1 polypeptide further comprising a LDM/LATGPA/NGS/TLT motif at positions 262-273, wherein the amino acid positions of the polypeptide are numbered by correspondence with the amino sequence set forth in SEQ ID NO:32 and are based on the conserved linear sequence numbering.

8. The cleaning composition of any of claims 1 to 6, wherein the endo-β-mannanase polypeptide is an NDL-Clade 2 polypeptide further comprising a LDLA/VA/TGPS/NGNLT motif at positions 262-273, wherein the amino acid positions of the polypeptide are numbered by correspondence with the amino sequence set forth in SEQ ID NO:32 and are based on the conserved linear sequence numbering.

9. The cleaning composition of any preceding claim, wherein the endo-β-mannanase polypeptide or active fragment thereof has mannanase activity on locust bean gum galactomannan or konjac glucomannan.

10. The cleaning composition of any preceding claim, wherein the endo-β-mannanase polypeptide or active fragment thereof retains at least 70% of its maximal mannanase activity at a pH range of 4.5-9.0; retains at least 70% of its maximal mannanase activity at a pH range of 5.5-8.5; retains at least 70% of its maximal mannanase activity at a pH range of 6.0-7.5; retains at least 70% of its maximal mannanase activity at a temperature range of 40°C to 70°C; retains at least 70% of its maximal mannanase activity at a temperature range of 45°C to 65°C; and/or retains at least 70% of its maximal mannanase activity at a temperature range of 50°C to 60°C.

11. The cleaning composition of any preceding claim, wherein the endo-β-mannanase polypeptide or active fragment thereof is capable of hydrolyzing a substrate selected from the group consisting of guar gum, locust bean gum, and combinations thereof.

12. The cleaning composition of any preceding claim, wherein the endo-β-mannanase polypeptide or active fragment thereof does not further comprise a carbohydrate-binding module.

13. The cleaning composition of any preceding claim, further comprising; at least one adjunct ingredient; and/or an enzyme selected from the group consisting of acyl transferases, amylases, alpha-amylases, beta-amylases, alpha-galactosidases, arabinases, arabinosidases, aryl esterases, beta-galactosidases, beta-glucanases, carrageenases, catalases, cellobiohydrolases, cellulases, chondroitinases, cutinases, endo-beta-1, 4-glucanases, endo-beta-mannanases, exo-beta-mannanases, esterases, exo-mannanases, galactanases, glucoamylases, hemicellulases, hyaluronidases, keratinases, laccases, lactases, ligninases, lipases, lipolytic enzymes, lipoxygenases, mannanases, oxidases, pectate lyases, pectin acetyl esterases, pectinases, pentosanases, perhydrolases, peroxidases, phenoloxidases, phosphatases, phospholipases, phytases, polygalacturonases, proteases, pullulanases, reductases, rhamnogalacturonases, beta-glucanases, tannases, transglutaminases, xylan acetyl-esterases, xylanases, xyloglucanases, xylosidases, metalloproteases, and combinations thereof.

14. The cleaning composition of any preceding claim, wherein the cleaning composition:
(i) is a detergent composition selected from the group consisting of a laundry detergent, a fabric softening detergent, a dishwashing detergent, and a hard-surface cleaning detergent;
(ii) is in a form selected from the group consisting of a liquid, a powder, a granulated solid, a tablet, a sheet, and a unit dose;
(iii) is phosphate-free; or
(iv) is boron-free.

15. A method of textile cleaning comprising contacting a soiled textile with the cleaning composition of any one of the preceding claims to produce a clean textile.

## Patentansprüche

1. Reinigungszusammensetzung, umfassend ein Tensid und ein Endo-β-Mannanase-Polypeptid, umfassend eine Aminosäuresequenz, die eine Identität von mindestens 90% zu einer Aminosäuresequenz aufweist, die ausgewählt ist aus SEQ ID No: 8, 10, 12, 42, 43, 44, 46, 47, 48, 50, 51 und 52 oder einem aktiven Fragment davon, wobei das Polypeptid oder aktive Fragment davon in der Gegenwart eines Tensids MannanaseAktivität aufweist.

2. Reinigungszusammensetzung nach Anspruch 1, wobei das Endo-β-Mannanase-Polypeptid oder aktive Fragment davon Asn33-Asp-34-Leu35 enthält, wobei die Aminosäurepositionen des Polypeptids durch Korrespondenz mit der in der SEQ ID NO: 32 festgelegten Aminosäuresequenz nummeriert sind und auf der konservierten linearen Sequenznummerierung basieren.

3. Reinigungszusammensetzung nach Anspruch 1 oder Anspruch 2, wobei das Endo-β-Mannanase-Polypeptid oder aktive Fragment davon ferner ein WXaKNDLXXAI-Motiv an den Positionen 30 bis 38 umfasst, wobei Xₐ F oder Y ist und X eine beliebige Aminosäure ist, wobei die Aminosäurepositionen des Polypeptids in Korrespondenz mit der in der SEQ ID NO: 32 festgelegten Aminosäuresequenz nummeriert sind und auf der konservierten linearen Sequenznummerierung basieren.

4. Reinigungszusammensetzung nach einem der vorhergehenden Ansprüche, wobei das Endo-β-Mannanase-Polypeptid oder aktive Fragment davon ferner ein WXₐKNDLXbX_{c}AI-Motiv an den Positionen 30 bis 38 umfasst, wobei Xₐ F oder Y ist, X_{b} N, Y oder A ist und X_{c} A oder T ist, wobei die Aminosäurepositionen des Polypeptids in Korrespondenz mit der in der SEQ ID NO: 32 festgelegten Aminosäuresequenz nummeriert sind und auf der konservierten linearen Sequenznummerierung basieren.

5. Reinigungszusammensetzung nach einem der vorhergehenden Ansprüche, wobei das Endo-β-Mannanase-Polypeptid oder aktive Fragment davon ferner ein L₂₆₂D₂₆₃XXXGPXGXL₂₇₂T₂₇₃-Motiv an den Positionen 262-273 umfasst, wobei X eine beliebige Aminosäure ist und wobei die Aminosäurepositionen des Polypeptids in Korrespondenz mit der in der SEQ ID NO: 32 festgelegten Aminosäuresequenz nummeriert sind und auf der konservierten linearen Sequenznummerierung basieren.

6. Reinigungszusammensetzung nach einem der vorhergehenden Ansprüche, wobei das Endo-β-Mannanase-Polypeptid oder aktive Fragment davon ferner ein L₂₆₂D₂₆₃M/LV/AT/AGPX₁GX₂L₂₇₂T₂₇₃-Motiv an den Positionen 262-273 umfasst, wobei X₁ N, A oder S und X₂ S, T oder N ist und wobei die Aminosäurepositionen des Polypeptids in Korrespondenz mit der in der SEQ ID NO: 32 festgelegten Aminosäuresequenz nummeriert sind und auf der konservierten linearen Sequenznummerierung basieren.

7. Reinigungszusammensetzung nach einem der vorhergehenden Ansprüche, wobei das Endo-β-Mannanase-Polypeptid ein NDL-Clade-1-Polypeptid ist, das ferner ein LDM/LATGPA/NGS/TLT-Motiv an den Positionen 262-273 umfasst, wobei die Aminosäurepositionen des Polypeptids in Korrespondenz mit der in der SEQ ID NO: 32 festgelegten Aminosäuresequenz nummeriert sind und auf der konservierten linearen Sequenznummerierung basieren.

8. Reinigungszusammensetzung nach einem der Ansprüche 1 bis 6, wobei das Endo-β-Mannanase-Polypeptid ein NDL-Clade-2-Polypeptid ist, das ferner ein LDLA/VA/TGPS/NGNLT-Motiv an den Positionen 262-273 umfasst, wobei die Aminosäurepositionen des Polypeptids in Korrespondenz mit der in der SEQ ID NO: 32 festgelegten Aminosäuresequenz nummeriert sind und auf der konservierten linearen Sequenznummerierung basieren.

9. Reinigungszusammensetzung nach einem der vorhergehenden Ansprüche, wobei das Endo-β-Mannanase-Polypeptid oder aktive Fragment davon Mannanaseaktivität auf Johannisbrotkernmehl-Galactomannan oder Konjac-Glucomannan aufweist.

10. Reinigungszusammensetzung nach einem der vorhergehenden Ansprüche, wobei das Endo-β-Mannanase-Polypeptid oder aktive Fragment davon mindestens 70% seiner maximalen Mannanaseaktivität bei einem pH-Bereich von 4,5 bis 9,0 beibehält; mindestens 70% seiner maximalen Mannanaseaktivität bei einem pH-Bereich von 5,5 bis 8,5 beibehält; mindestens 70% seiner maximalen Mannanaseaktivität bei einem pH-Bereich von 6,0 bis 7,5 beibehält; mindestens 70% seiner maximalen Mannanaseaktivität bei einem Temperatur-Bereich von 40 °C bis 70 °C beibehält; mindestens 70% seiner maximalen Mannanaseaktivität bei einem Temperatur-Bereich von 45 °C bis 65 °C beibehält; und/oder mindestens 70% seiner maximalen Mannanaseaktivität bei einem Temperatur-Bereich von 50 °C bis 60 °C beibehält.

11. Reinigungszusammensetzung nach einem der vorhergehenden Ansprüche, wobei das Endo-β-Mannanase-Polypeptid oder aktive Fragment davon zum Hydrolysieren eines Substrats in der Lage ist, das ausgewählt ist aus der Gruppe bestehend aus Guarmehl, Johannisbrotkernmehl und Kombinationen davon.

12. Reinigungszusammensetzung nach einem der vorhergehenden Ansprüche, wobei das Endo-β-Mannanase-Polypeptid oder aktive Fragment davon weiterhin kein Kohlenhydrat-Bindungsmodul umfasst.

13. Reinigungszusammensetzung nach einem der vorhergehenden Ansprüche, ferner umfassend einen zusätzlichen Bestandteil und/oder ein Enzym, ausgewählt aus der Gruppe bestehend aus Acyltransferasen, Amylasen, Alpha-Amylasen, Beta-Amylasen, Alpha-Galactosidasen, Arabinasen, Arabinosidasen, Arylesterasen, Beta-Galactosidasen. Beta-Glucanasen, Carrageenasen, Katalasen, Cellobiohydrolasen, Cellulasen, Chondroitinasen, Cutinasen, Endo-Beta-1,4-Glucanasen, Endo-Beta-Mannanasen, Exo-Beta-Mannanasen, Esterasen, Exo-Mannanasen, Galactanasen, Glucoamylasen, Hemicellulasen, Hyaluronidasen, Keratinasen, Laccasen, Lactasen, Ligninasen, Lipasen, lipolytische Enzyme, Lipoxygenasen, Mannanasen, Oxidasen, Pektatlyasen, Pektinacetylesterasen, Pektinasen, Pentosanasen, Perhydrolasen, Peroxidasen, Phenoloxidasen, Phosphatasen, Phospholipasen, Phytasen, Polygalacturonasen, Proteasen, Pullulanasen, Reduktasen, Rhamnogalacturonasen, Beta-Glucanasen, Tannasen, Transglutaminasen, Xylanacetylesterasen, Xylanasen, Xyloglucanasen, Xylosidasen, Metalloproteasen und Kombinationen davon.

14. Reinigungszusammensetzung nach einem der vorhergehenden Ansprüche, wobei die Reinigungszusammensetzung:
(i) eine Detergens-Zusammensetzung ist, ausgewählt aus der Gruppe bestehend aus einem Waschmittel-Detergens, einem Textilweichmacher-Detergens, einem Geschirrspülmittel-Detergens und einem Detergens zum Reinigen harter Oberflächen;
(ii) in einer Form vorliegt, ausgewählt aus der Gruppe bestehend aus einer Flüssigkeit, einem Pulver, einem granulierten Feststoff, einer Tablette, einer Folie und einer Einheitsdosis;
(iii) phosphatfrei ist; oder
(iv) borfrei ist.

15. Verfahren zum Textilreinigen, umfassend Inkontaktbringen eines verschmutzten Textils mit der Reinigungszusammensetzung nach einem der vorhergehenden Ansprüche, um ein sauberes Textil herzustellen.

## Revendications

1. Composition de nettoyage comprenant un tensioactif et un polypeptide endo-β-mannanase comprenant une séquence d'acides aminés ayant au moins 90 % d'identité avec une séquence d'acides aminés sélectionnée parmi les SEQ ID NO; 8, 10, 12, 42, 43, 44, 46, 47, 48, 50, 51 et 52, ou un fragment actif de celui-ci, où ledit polypeptide ou fragment actif de celui-ci a une activité de mannanase en présence d'un tensioactif.

2. Composition de nettoyage selon la revendication 1, dans laquelle le polypeptide endo-β-mannanase ou fragment actif de celui-ci contient Asn33-Asp-34-Leu35, où les positions des acides aminés du polypeptide sont numérotées par correspondance à la séquence d'acides aminés présentée dans la SEQ ID NO; 32 et sont basées sur la numérotation de la séquence linéaire conservée.

3. Composition de nettoyage selon la revendication 1 ou la revendication 2, dans laquelle le polypeptide endo-β-mannanase ou fragment actif de celui-ci comprend en outre un motif WXaKNDLXXAI aux positions 30-38, où Xₐ est F ou Y et X est un acide aminé quelconque, où les positions des acides aminés du polypeptide sont numérotées par correspondance à la séquence d'acides aminés présentée dans la SEQ ID NO; 32 et sont basées sur la numérotation de la séquence linéaire conservée.

4. Composition de nettoyage selon l'une quelconque des revendications précédentes, dans laquelle le polypeptide endo-β-mannanase ou fragment actif de celui-ci comprend en outre un motif WXₐKNDLX_{b}X_{c}AI aux positions 30-38, où Xₐ est F ou Y, X_{b} est N, Y ou A et X_{c} est A ou T, où les positions des acides aminés du polypeptide sont numérotées par correspondance à la séquence d'acides aminés présentée dans la SEQ ID NO; 32 et sont basées sur la numérotation de la séquence linéaire conservée.

5. Composition de nettoyage selon l'une quelconque des revendications précédentes, dans laquelle le polypeptide endo-β-mannanase ou fragment actif de celui-ci comprend en outre un motif L₂₆₂D₂₆₃XXXGPXGXL₂₇₂T₂₇₃ aux positions 262-273, où X est un acide aminé quelconque et où les positions des acides aminés du polypeptide sont numérotées par correspondance à la séquence d'acides aminés présentée dans la SEQ ID NO; 32 et sont basées sur la numérotation de la séquence linéaire conservée.

6. Composition de nettoyage selon l'une quelconque des revendications précédentes, dans laquelle le polypeptide endo-β-mannanase ou fragment actif de celui-ci comprend en outre un motif L₂₆₂D₂₆₃M/LV/AT/AGPX₁GX₂L₂₇₂T₂₇₃ aux positions 262-273, où X₁ est N, A ou S et X₂ est S, T ou N, et où les positions des acides aminés du polypeptide sont numérotées par correspondance à la séquence d'acides aminés présentée dans la SEQ ID NO; 32 et sont basées sur la numérotation de la séquence linéaire conservée.

7. Composition de nettoyage selon l'une quelconque des revendications précédentes, dans laquelle le polypeptide endo-β-mannanase est un polypeptide NDL-Clade-1 comprenant en outre un motif LDM/LATGPA/NGS/TLT aux positions 262-273, où les positions des acides aminés du polypeptide sont numérotées par correspondance à la séquence d'acides aminés présentée dans la SEQ ID NO; 32 et sont basées sur la numérotation de la séquence linéaire conservée.

8. Composition de nettoyage selon l'une quelconque des revendications 1 à 6, dans laquelle le polypeptide endo-β-mannanase est un polypeptide NDL-Clade-2 comprenant en outre un motif LDLA/VA/TGPS/NGNLT aux positions 262-273, où les positions des acides aminés du polypeptide sont numérotées par correspondance à la séquence d'acides aminés présentée dans la SEQ ID NO; 32 et sont basées sur la numérotation de la séquence linéaire conservée.

9. Composition de nettoyage selon l'une quelconque des revendications précédentes, dans laquelle le polypeptide endo-β-mannanase ou fragment actif de celui-ci a une activité de mannanase sur le galactomannane de gomme de caroube ou galactomannane de konjac.

10. Composition de nettoyage selon l'une quelconque des revendications précédentes, dans laquelle le polypeptide endo-β-mannanase ou fragment actif de celui-ci conserve au moins 70 % de son activité maximale de mannanase à une plage de pH de 4,5-9,0; conserve au moins 70 % de son activité maximale de mannanase à une plage de pH de 5,5-8,5; conserve au moins 70 % de son activité maximale de mannanase à une plage de pH de 6,0-7,5; conserve au moins 70 % de son activité maximale de mannanase à une plage de température de 40 °C à 70 °C; conserve au moins 70 % de son activité maximale de mannanase à une plage de température de 45 °C à 65 °C; et/ou conserve au moins 70 % de son activité maximale de mannanase à une plage de température de 50 °C à 60 °C.

11. Composition de nettoyage selon l'une quelconque des revendications précédentes, dans laquelle le polypeptide endo-β-mannanase ou fragment actif de celui-ci est capable d'hydrolyser un substrat sélectionné parmi le groupe consistant en gomme de guar, gomme de caroube et des combinaisons de celles-ci.

12. Composition de nettoyage selon l'une quelconque des revendications précédentes, dans laquelle le polypeptide endo-β-mannanase ou fragment actif de celui-ci ne comprend pas également une molécule de liaison à l'hydrate de carbone.

13. Composition de nettoyage selon l'une quelconque des revendications précédentes, comprenant en outre; au moins un ingrédient annexe; et/ou une enzyme sélectionnée parmi le groupe consistant en acyle transférases, amylases, alpha-amylases, bêta-amylases, alpha-galactosidases, arabinases, arabinosidases, aryle estérases, bêta-galactosidases, bêta-glucanases, carraghénases, catalases, cellobiohydrolases, cellulases, chondroïtinases, cutinases, endo-bêta-1,4-glucanases, endo-bêta-mannanases, exo-bêta-mannanases, estérases, exo-mannanases, galactanases, gluco-amylases, hémicellulases, hyaluronidases, kératinases, laccases, lactases, ligninases, lipases, enzymes lipolytiques, lipoxygénases, mannanases, oxydases, pectate lyases, pectine acétyle estérases, pectinases, pentosanases, perhydrolases, peroxydases, phénoloxydases, phosphatases, phospholipases, phytases, polygalacturonases, protéases, pullulanases, réductases, rhamnogalacturonases, bêta-glucanases, tannases, transglutaminases, xylane acétyle estérases, xylanases, xyloglucanases, xylosidases, métalloprotéases et des combinaisons de celles-ci.

14. Composition de nettoyage selon l'une quelconque des revendications précédentes, où la composition de nettoyage;
(i) est une composition détergente sélectionnée parmi le groupe consistant en un détergent de lessive, un détergent adoucisseur de textile, un détergent de vaisselle ou un détergent de nettoyage de surface dure;
(ii) est sous une forme sélectionnée parmi le groupe consistant en un liquide, une poudre, un solide granulé, une pastille, une feuille et une dose unitaire;
(iii) ne contient pas de phosphate; ou
(iv) ne contient pas de bore.

15. Procédé de nettoyage de textile comprenant mettre un textile souillé en contact avec la composition de nettoyage selon l'une quelconque des revendications précédentes pour produire un textile propre.
